# EUROPEAN PATENT APPLICATION

(11) **EP 4 592 285 A1**
(43) Date of publication of application: **30.07.2025**
(21) Application number: 23868264.5
(22) Date of filing: 21.09.2023
(51) Int. Cl.: C07D 405/06, A61K 31/4184, A61K 31/4355, A61K 31/4375, A61K 31/4725, A61K 31/4741, A61K 31/498, A61K 31/4985, A61K 31/519, A61P 3/04, A61P 3/10, A61P 43/00, C07D 405/14, C07D 471/04, C07D 491/056, C07D 519/00

(54) **FUSED RING COMPOUND HAVING GLP-1 RECEPTOR AGONIST EFFECT**

(30) Priority: 22.09.2022 JP 2022150845
(71) Applicant: Shionogi & Co., Ltd., Osaka-shi, Osaka 541-0045 (JP)
(72) Inventor: KITAMURA FUJIWARA, Misato, Osaka-shi, Osaka 541-0045 (JP); NISHIURA, Yuji, Osaka-shi, Osaka 541-0045 (JP); MAENO, Shomitsu, Osaka-shi, Osaka 541-0045 (JP); KUSANO, Hiroki, Osaka-shi, Osaka 541-0045 (JP); WADA, Toshihiro, Osaka-shi, Osaka 541-0045 (JP)
(74) Representative: Vossius & Partner Patentanwälte Rechtsanwälte mbB
(86) International application number: PCT/JP2023/034298
(87) International publication number: WO 2024/063143

(57) **Abstract**

The present invention provides a compound represented by the following formula (I) or (II), or a pharmaceutically acceptable salt thereof.

A compound represented by formula (I): 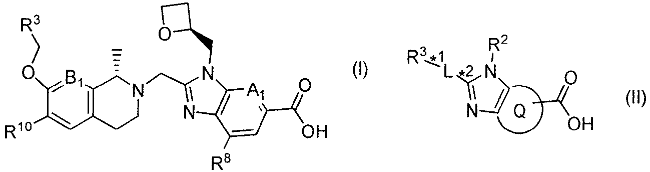 wherein
A₁ is C(R¹) or the like, R¹ is a hydrogen atom or the like,
R⁸ is a hydrogen atom or the like, B₁ is CH or the like, R¹⁰ is cyano or the like, R³ is phenyl optionally substituted with substituent group F or the like,
the substituent group F: halogen, cyano, alkyl, haloalkyl, alkyloxy, and haloalkyloxy,
Q is a substituted or unsubstituted benzene ring or the like, R² is substituted or unsubstituted alkyl or the like, and -L- is a group or the like shown below:

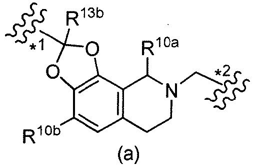

## Description

### [TECHNICAL FIELD]

The present invention relates to a compound that has GLP-1 receptor agonist activity and is useful as a therapeutic or prophylactic agent for diseases associated with the GLP-1 receptor, or its pharmaceutically acceptable salt, and a pharmaceutical composition containing thereof, particularly a prophylactic and/or therapeutic agent for non-insulin-dependent diabetes mellitus (type 2 diabetes mellitus) or obesity.

### [BACKGROUND ART]

Glucagon-like peptide-1 (GLP-1) is an incretin hormone that is secreted by L cells in the intestine in response to ingestion of food. GLP-1 is known to exhibit effects via the GLP-1 receptor such as promotion of glucose-dependent insulin secretion, reduction of glucagon secretion, delayed gastric emptying, and reduction of appetite. So far, it has been studied that an agonist of the GLP-1 receptor is used for treating diabetes and obesity (Non Patent Documents 1 and 2). Liraglutide, an analog formulation of human GLP-1, is known as a representative agonist, but it has been found that it exhibits a potent HbA1c lowering action and body weight reduction. Due to such attractive effects, a plurality of GLP-1 analog formulations have been put into practical use as therapeutic agents for diabetes and obesity. However, most of these GLP-1 analog formulations are sold as injectable formulations because of their poor oral absorbability. Therefore, development of an orally administrable GLP-1 receptor agonist is expected. Specifically, a method of causing semaglutide, which is an analog of GLP-1, to be orally absorbed by using an absorption promoting agent (Patent Document 1) has been put into practical use, but improvement of pharmaceutical properties such as bioavailability is required. In addition, attempts have been made to create a plurality of small molecule pharmaceuticals as non-peptidic GLP-1 receptor agonists (Patent Documents 2 to 58). The compounds substantially disclosed in Patent Documents 2 to 42, 44 to 55, and 57 have different chemical structures from those of the compounds of the present invention. On the other hand, the compounds disclosed in Patent Documents 43, 56, and 58 are structurally close to the compounds of the present invention.

### [PRIOR ART REFERENCES]

### [Patent Documents]

[Patent Document 1] International Publication WO 2012/080471A
[Patent Document 2] International Publication WO 2009/111700A
[Patent Document 3] International Publication WO 2010/114824A
[Patent Document 4] International Publication WO 2018/056453A
[Patent Document 5] International Publication WO 2018/109607A
[Patent Document 6] International Publication WO 2019/239319A
[Patent Document 7] International Publication WO 2019/239371A
[Patent Document 8] International Publication WO 2020/103815A
[Patent Document 9] International Publication WO 2020/207474A
[Patent Document 10] International Publication WO 2020/263695A
[Patent Document 11] International Publication WO 2021/018023A
[Patent Document 12] International Publication WO 2021/081207A
[Patent Document 13] International Publication WO 2021/096284A
[Patent Document 14] International Publication WO 2021/096304A
[Patent Document 15] International Publication WO 2021/112538A
[Patent Document 16] International Publication WO 2021/155841A
[Patent Document 17] International Publication WO 2021/160127A
[Patent Document 18] International Publication WO 2021/187886A
[Patent Document 19] Chinese Patent Application Publication CN 113493447A
[Patent Document 20] International Publication WO 2021/197464A
[Patent Document 21] International Publication WO 2021/219019A
[Patent Document 22] Chinese Patent Application Publication CN 113480534A
[Patent Document 23] International Publication WO 2021/244645A
[Patent Document 24] International Publication WO 2021/249492A
[Patent Document 25] International Publication WO 2021/242817A
[Patent Document 26] Chinese Patent Application Publication CN 113773310A
[Patent Document 27] Chinese Patent Application Publication CN 113816948A
[Patent Document 28] Chinese Patent Application Publication CN 113801136A
[Patent Document 29] International Publication WO 2021/254470A
[Patent Document 30] International Publication WO 2021/259309A
[Patent Document 31] International Publication WO 2022/028572A
[Patent Document 32] International Publication WO 2022/031994A
[Patent Document 33] International Publication WO 2022/040600A
[Patent Document 34] International Publication WO 2022/042691A
[Patent Document 35] International Publication WO 2022/078380A
[Patent Document 36] International Publication WO 2022/078152A
[Patent Document 37] International Publication WO 2022/078407A
[Patent Document 38] International Publication WO 2022/109182A
[Patent Document 39] International Publication WO 2022/111624A
[Patent Document 40] International Publication WO 2022/116693A
[Patent Document 41] Chinese Patent Application Publication CN 114478497A
[Patent Document 42] Chinese Patent Application Publication CN 114716423A
[Patent Document 43] International Patent Application No. JP2022/13362
[Patent Document 44] International Publication WO 2022/068772A
[Patent Document 45] International Publication WO 2022/184849A
[Patent Document 46] International Publication WO 2022/192428A
[Patent Document 47] International Publication WO 2022/192430A
[Patent Document 48] International Publication WO 2022/199458A
[Patent Document 49] International Publication WO 2022/199661A
[Patent Document 50] International Publication WO 2022/219495A
[Patent Document 51] International Publication WO 2022/225914A
[Patent Document 52] International Publication WO 2022/225941A
[Patent Document 53] International Publication WO 2022/228490A
[Patent Document 54] International Publication WO 2022/246019A
[Patent Document 55] International Publication WO 2022/268152A
[Patent Document 56] Chinese Patent Application Publication CN 115594669A
[Patent Document 57] U.S. Patent Application Publication No. 20220396569
[Patent Document 58] International Publication WO 2023/138684A

### [Non-patent Documents]

[Non-patent Document 1] Lancet 374, 1606-1616 (2009)
[Non-patent Document 2] Clin. Invest. 2, 59-72 (2012)

### [SUMMARY OF THE INVENTION]

### [PROBLEMS TO BE SOLVED BY THE INVENTION]

An object of the present invention is to provide a compound that has GLP-1 receptor agonist activity and is useful as a therapeutic or prophylactic agent for diseases relating to the GLP-1 receptor, or its pharmaceutically acceptable salt, and a pharmaceutical composition containing thereof, particularly a prophylactic and/or therapeutic agent for non-insulin-dependent diabetes mellitus (type 2 diabetes mellitus) or obesity.

### [MEANS FOR SOLVING THE PROBLEM]

The present invention relates to the following.
[1] A compound represented by formula (I): 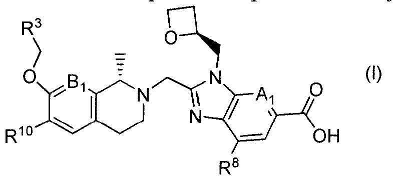 wherein
   A₁ is C(R¹) or N,
   R¹ is a hydrogen atom or halogen,
   R⁸ is a hydrogen atom, halogen, alkyloxy, or 5- or 6-membered aromatic heterocyclyl optionally substituted with halogen or alkyl,
   B₁ is CH or N,
   R¹⁰ is a hydrogen atom, cyano, a fluorine atom, a chlorine atom, methyl, difluoromethyl, or trifluoromethyl,
   R³ is phenyl optionally substituted with substituent group F, 5- or 6-membered aromatic heterocyclyl optionally substituted with substituent group F, bicyclic 9- or 10-membered aromatic heterocyclyl optionally substituted with substituent group F, or 5- to 12-membered non-aromatic heterocyclyl optionally substituted with substituent group F,
   the substituent group F: halogen, cyano, alkyl, haloalkyl, alkyloxy, and haloalkyloxy,
   wherein
      (i) when R¹⁰ is a chlorine atom, B₁ is N,
      (ii) when R¹⁰ is trifluoromethyl, R³ is 5-membered aromatic heterocyclyl optionally substituted with substituent group F, bicyclic 9- or 10-membered aromatic heterocyclyl optionally substituted with substituent group F, or 5- to 12-membered non-aromatic heterocyclyl optionally substituted with substituent group F, or R⁸ is 5- or 6-membered aromatic heterocyclyl optionally substituted with halogen or alkyl, and
      (iii) the compound is other than those below: 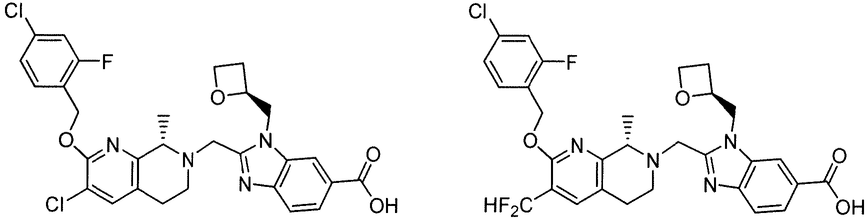 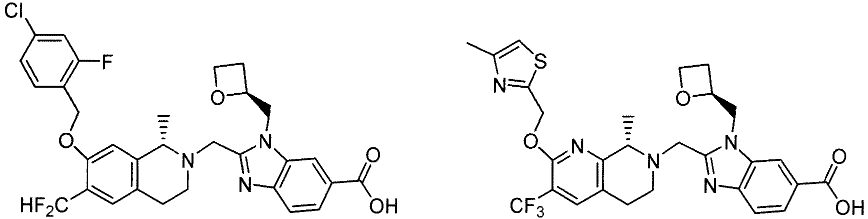 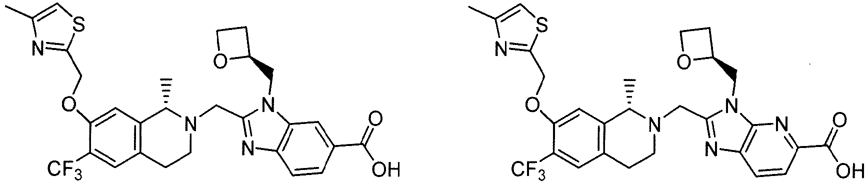 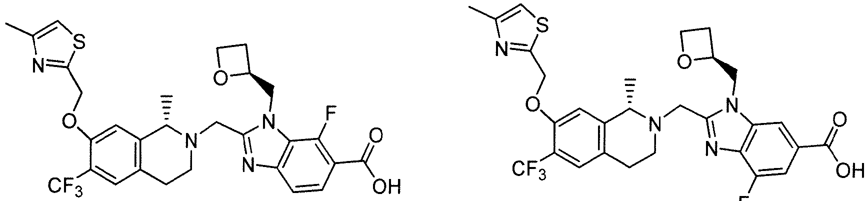 and 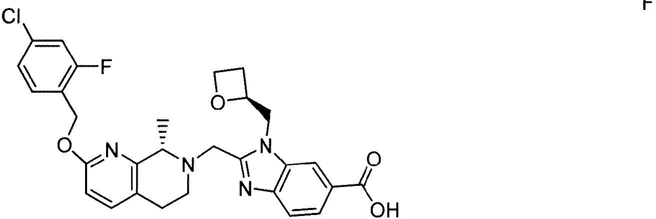
   or a pharmaceutically acceptable salt thereof.
[2] The compound according to [1] or a pharmaceutically acceptable salt thereof, wherein R¹⁰ is a hydrogen atom, cyano, a fluorine atom, a chlorine atom, methyl, or difluoromethyl.
[3] The compound according to [1] or [2] or a pharmaceutically acceptable salt thereof, wherein R³ is a group below: 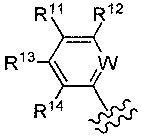 wherein
   W is N, or CR¹⁵,
   R¹¹ is a hydrogen atom, halogen, cyano, alkyl, haloalkyl, alkyloxy, or haloalkyloxy,
   R¹² and R¹³ are each independently a hydrogen atom or halogen,
   R¹⁴ and R¹⁵ are each independently a hydrogen atom, halogen, cyano, alkyl, haloalkyl, alkyloxy, or haloalkyloxy,
   R¹¹ and R¹² may be taken together to form 5-membered aromatic heterocycle optionally substituted with substituent group F, or 5- to 7-membered non-aromatic heterocycle optionally substituted with substituent group F,
   R¹¹ and R¹³ may be taken together to form 5-membered aromatic heterocycle optionally substituted with substituent group F, or 5- to 7-membered non-aromatic heterocycle optionally substituted with substituent group F, and
   R¹³ and R¹⁴ may be taken together to form 5-membered aromatic heterocycle optionally substituted with substituent group F, or 5- to 7-membered non-aromatic heterocycle optionally substituted with substituent group F.
[4] The compound according to any one of [1] to [3] or a pharmaceutically acceptable salt thereof, wherein R³ is any one of groups below: 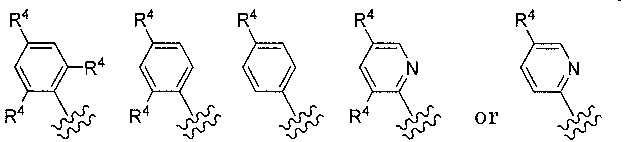 wherein R⁴s are each independently halogen, cyano, alkyl, haloalkyl, alkyloxy, or haloalkyloxy.
[5] The compound according to [4] or a pharmaceutically acceptable salt thereof, wherein R⁴s are each independently a fluorine atom, a chlorine atom, cyano, methyl, methyloxy, or difluoromethyloxy.
[6] The compound according to any one of [1] to [5] or a pharmaceutically acceptable salt thereof, wherein R¹⁰ is a chlorine atom or difluoromethyl.
[7] The compound according to any one of [1] to [6] or a pharmaceutically acceptable salt thereof, wherein R¹⁰ is difluoromethyl.
[8] The compound according to any one of [1] to [6] or a pharmaceutically acceptable salt thereof, wherein R¹⁰ is a chlorine atom, and B₁ is N.
[9] The compound according to any one of [1] and [3] to [5] or a pharmaceutically acceptable salt thereof, wherein
   R¹⁰ is trifluoromethyl, and
   R³ is 5-membered aromatic heterocyclyl optionally substituted with substituent group F, bicyclic 9- or 10-membered aromatic heterocyclyl optionally substituted with substituent group F, or bicyclic 8- to 11-membered non-aromatic heterocyclyl optionally substituted with substituent group F.
[10] The compound according to any one of [1] and [3] to [5] or a pharmaceutically acceptable salt thereof, wherein
   R¹⁰ is trifluoromethyl, and
   R⁸ is 5- or 6-membered aromatic heterocyclyl optionally substituted with halogen or alkyl.
[11] A compound represented by formula (II): 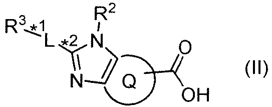 wherein
   Q is a substituted or unsubstituted benzene ring or substituted or unsubstituted 5- or 6-membered aromatic heterocycle,
   R² is substituted or unsubstituted alkyl, or substituted or unsubstituted non-aromatic heterocyclyl,
   -L- is any one of groups below: 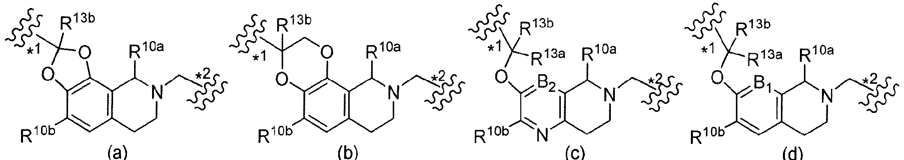 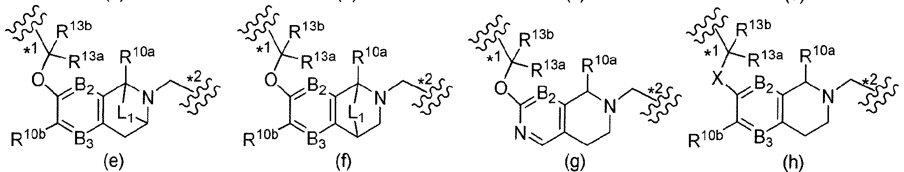 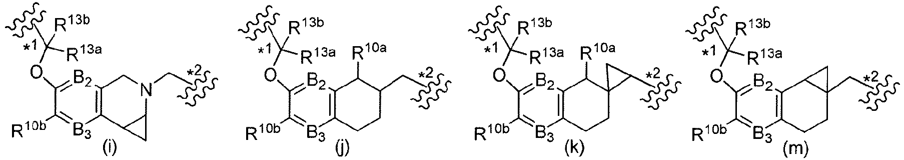 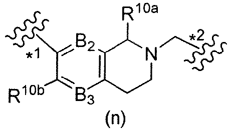
   wherein
   B₁ is CH or N,
   B₂ is C(R⁹) or N,
   R⁹ is a hydrogen atom or R⁹ and R^{13a} are taken together to form -O- or -CH₂-O-, B₃ is CH or N,
   R^{10a} is a hydrogen atom, or substituted or unsubstituted alkyl,
   R^{10b} is a hydrogen atom, cyano, halogen, or substituted or unsubstituted alkyl,
   -L₁- is alkylene,
   X is S, NH or CH₂,
   R^{13a} is a hydrogen atom or R^{13a} and R⁹ are taken together to form -O- or -CH₂-O-, and
   R^{13b} is a hydrogen atom, or substituted or unsubstituted alkyl, and
   R³ is phenyl optionally substituted with substituent group F, 5- or 6-membered aromatic heterocyclyl optionally substituted with substituent group F, bicyclic 9- or 10-membered aromatic heterocyclyl optionally substituted with substituent group F, or 5- to 12-membered non-aromatic heterocyclyl optionally substituted with substituent group F,
   the substituent group F: halogen, cyano, alkyl, haloalkyl, alkyloxy, and haloalkyloxy,
   wherein
      (i) when -L- is a group represented by (d), Q is a benzene ring substituted with substituted or unsubstituted aromatic heterocyclyl, 6-membered aromatic heterocycle substituted with substituted or unsubstituted aromatic heterocyclyl, or substituted or unsubstituted 5-membered aromatic heterocycle, and
      (ii) the compound is other than those below: 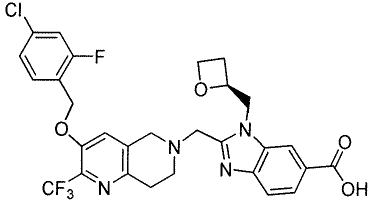
   or a pharmaceutically acceptable salt thereof.
[12] The compound according to [11] or a pharmaceutically acceptable salt thereof, wherein R³ is a group below: 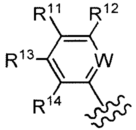 wherein
   W is N, or CR¹⁵,
   R¹¹ is a hydrogen atom, halogen, cyano, alkyl, haloalkyl, alkyloxy, or haloalkyloxy,
   R¹² and R¹³ are each independently a hydrogen atom or halogen,
   R¹⁴ and R¹⁵ are each independently a hydrogen atom, halogen, cyano, alkyl, haloalkyl, alkyloxy, or haloalkyloxy,
   R¹¹ and R¹² may be taken together to form 5-membered aromatic heterocycle optionally substituted with substituent group F, or 5- to 7-membered non-aromatic heterocycle optionally substituted with substituent group F,
   R¹¹ and R¹³ may be taken together to form 5-membered aromatic heterocycle optionally substituted with substituent group F, or 5- to 7-membered non-aromatic heterocycle optionally substituted with substituent group F, and
   R¹³ and R¹⁴ may be taken together to form 5-membered aromatic heterocycle optionally substituted with substituent group F, or 5- to 7-membered non-aromatic heterocycle optionally substituted with substituent group F.
[13] The compound according to [11] or [12] or a pharmaceutically acceptable salt thereof, wherein R³ is any one of groups below: 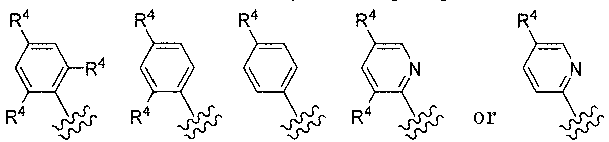 wherein R⁴s are each independently halogen, cyano, alkyl, haloalkyl, alkyloxy, or haloalkyloxy.
[14] The compound according to [13] or a pharmaceutically acceptable salt thereof, wherein R⁴s are each independently a fluorine atom, a chlorine atom, cyano, methyl, methyloxy, or difluoromethyloxy.
[15] The compound according to any one of [11] to [14] or a pharmaceutically acceptable salt thereof, wherein Q is any one of groups below: 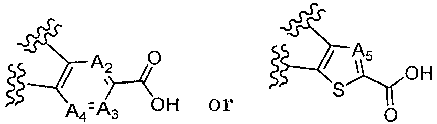 wherein
   A₂ is C(R⁵) or N,
   A₃ is C(R⁶) or N,
   A₄ is C(R⁷) or N,
   R⁵, R⁶, and R⁷ are each independently a hydrogen atom, halogen, cyano, substituted or unsubstituted alkyl, substituted or unsubstituted alkyloxy, substituted or unsubstituted aromatic heterocyclyl, or substituted or unsubstituted non-aromatic carbocyclyl,
   A₅ is C(R⁹) or N, and
   R⁹ is each independently a hydrogen atom, halogen, cyano, substituted or unsubstituted alkyl, or substituted or unsubstituted alkyloxy.
[16] The compound according to [15] or a pharmaceutically acceptable salt thereof, wherein
   (i) A₂ is C(R⁵), A₃ is C(R⁶), and A₄ is C(R⁷),
   (ii) A₂ is N, A₃ is C(R⁶), and A₄ is C(R⁷),
   (iii) A₂ is C(R⁵), A₃ is C(R⁶), and A₄ is N, or
   (iv) A₂ is N, A₃ is C(R⁶), and A₄ is N.
[17] The compound according to [16] or a pharmaceutically acceptable salt thereof, wherein
   (i) A₂ is C(R⁵), A₃ is C(R⁶), and A₄ is C(R⁷), or
   (ii) A₂ is N, A₃ is C(R⁶), and A₄ is C(R⁷).
[18] The compound according to any one of [15] to [17] or a pharmaceutically acceptable salt thereof, wherein R⁵, R⁶, and R⁷ are each independently a hydrogen atom, halogen, alkyl, or alkyloxy.
[19] The compound according to any one of [15] to [17] or a pharmaceutically acceptable salt thereof, wherein R⁵ is a hydrogen atom or halogen, R⁶ is a hydrogen atom, and R⁷ is a hydrogen atom, halogen, alkyloxy, or methylpyrazolyl.
[20] The compound according to any one of [11] to [19] or a pharmaceutically acceptable salt thereof, wherein -L- is any one of groups below: 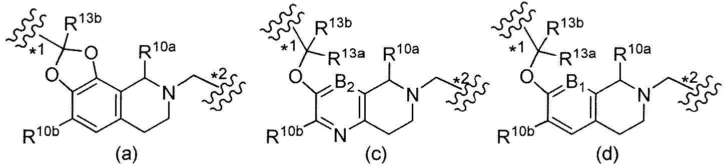 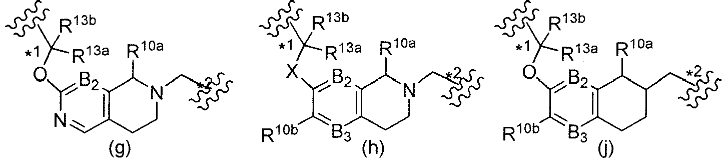
[21] The compound according to [20] or a pharmaceutically acceptable salt thereof, wherein -L- is any one of groups below: 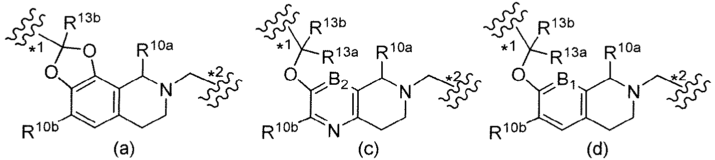
[21-1] The compound according to [20] or a pharmaceutically acceptable salt thereof, wherein -L- is any one of groups below: 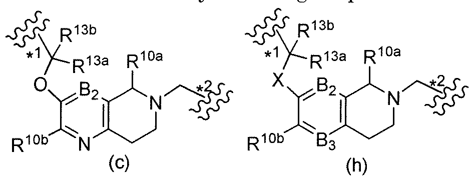
[22] The compound according to [21] or a pharmaceutically acceptable salt thereof, wherein -L- is a group below: 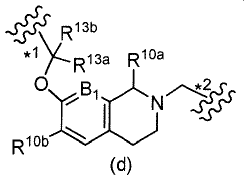
[23] The compound according to any one of [11] to [21], [21-1], and [22] or a pharmaceutically acceptable salt thereof, wherein R² is oxetanylmethyl.
[24] A pharmaceutical composition comprising a compound according to any one of [1] to [21], [21-1], [22], and [23] or a pharmaceutically acceptable salt thereof.
[25] The pharmaceutical composition according to [24], which is a GLP-1 receptor agonist.
[26] A method for treating and/or preventing a disease associated with GLP-1 receptor, comprising administering the compound according to any one of the above [1] to [21], [21-1], [22], and [23] or a pharmaceutically acceptable salt thereof.
[27] Use of the compound according to any one of the above [1] to [21], [21-1], [22], and [23] or a pharmaceutically acceptable salt thereof for manufacture of a medicament for treating and/or preventing a disease associated with GLP-1 receptor.
[28] The compound according to any one of the above [1] to [21], [21-1], [22], and [23] or a pharmaceutically acceptable salt thereof for use in treating and/or preventing a disease associated with GLP-1 receptor.

### [EFFECT OF THE INVENTION]

The compound of the present invention has GLP-1 receptor agonist effect, and is useful as a prophylactic and/or therapeutic agent for diseases associated with GLP-1 receptor, particularly non-insulin-dependent diabetes mellitus, such as type 2 diabetes mellitus or obesity.

### [MODE FOR CARRYING OUT THE INVENTION]

Terms used in this description are explained below. Each term, unless otherwise indicated, has the same meaning when it is used alone or together with other terms.

The term "consisting of" means having only components.

The term "comprising" means not restricting to components and not excluding undescribed factors.

Hereinafter, the present invention will be described while showing exemplary embodiments. Throughout the present specification, it should be understood that, unless particularly stated otherwise, an expression of a singular form also includes the concept of a plural form thereof. Therefore, it should be understood that, unless particularly stated otherwise, an article for a singular form (for example, in the case of English, "a", "an", "the", or the like) also includes the concept of a plural form thereof. Furthermore, it should be understood that, unless particularly stated otherwise, the terms used in the present specification are used in the meanings normally used in the above-described art. Accordingly, unless otherwise defined, all terminologies and scientific and technical terms used in the present specification have the same meanings as commonly understood by those having ordinary skill in the art to which the present invention belongs. In a case of contradiction, priority is given to the present specification including definitions.

"Halogen" includes a fluorine atom, a chlorine atom, a bromine atom and an iodine atom. Particularly, a fluorine atom and a chlorine atom are preferred.

"Alkyl" includes a C1 to C15, preferably C1 to C10, more preferably C1 to C6 and further preferably C1 to C4 linear or branched hydrocarbon group. Examples include methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl and n-decyl.

Preferred embodiments of "alkyl" include methyl, ethyl, n-propyl, isopropyl, n-butyl, isobutyl, sec-butyl, tert-butyl and n-pentyl. More preferred embodiments include methyl, ethyl, n-propyl, isopropyl and tert-butyl.

"Alkenyl" includes a C2 to C15, preferably a C2 to C10, more preferably a C2 to C6 and further preferably a C2 to C4 linear or branched hydrocarbon group having one or more double bond(s) at any position(s). Examples include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl and pentadecenyl.

Preferred embodiments of "alkenyl" include vinyl, allyl, propenyl, isopropenyl and butenyl.

"Alkynyl" includes a C2 to C10, preferably a C2 to C8, more preferably a C2 to C6 and further preferably a C2 to C4 linear or branched hydrocarbon group having one or more triple bond(s) at any position(s). Alkynyl may further have double bond(s) at any position(s). Examples include ethynyl, propynyl, butynyl, pentynyl, hexynyl, heptynyl, octynyl, nonynyl and decynyl.

Preferred embodiments of "alkynyl" include ethynyl, propynyl, butynyl and pentynyl.

"Aromatic carbocyclyl" means a cyclic aromatic hydrocarbon group having a single ring or two or more rings. Examples include phenyl, naphthyl, anthryl and phenanthryl.

Preferred embodiments of the "aromatic carbocyclyl" include phenyl.

"Aromatic carbocycle" means a ring derived from the above "aromatic carbocyclyl".

Preferred embodiment of "aromatic carbocycle" include a benzene ring.

"Non-aromatic carbocyclyl" means a cyclic saturated hydrocarbon group or a cyclic non-aromatic unsaturated hydrocarbon group, both having a single ring or two or more rings. A "non-aromatic carbocyclyl" having two or more rings also includes a fused ring group wherein a non-aromatic carbocyclyl having a single ring or two or more rings is fused with a ring of the above "aromatic carbocyclyl", and the linking bond may be carried by any of the rings.

Examples include rings as follows.

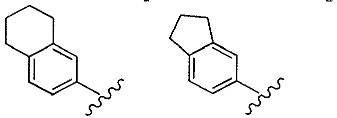

Furthermore, the "non-aromatic carbocyclyl" also includes a group having a bridged group or a group forming a spiro ring, such as follows.

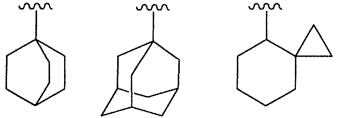

A non-aromatic carbocyclyl having a single ring is preferably C3 to C16, more preferably C3 to C12 and further preferably C4 to C8 non-aromatic carbocyclyl. Examples include cyclopropyl, cyclobutyl, cyclopentyl, cyclohexyl, cycloheptyl, cyclooctyl, cyclononyl, cyclodecyl, cyclopropenyl, cyclobutenyl, cyclopentenyl, cyclohexenyl, cycloheptenyl and cyclohexadienyl.

The non-aromatic carbocyclyl which is polycyclic having two or more rings is preferably C8 to C20, more preferably C8 to C16 non-aromatic carbocyclyl. Examples include indanyl, indenyl, acenaphthyl, tetrahydronaphthyl and fluorenyl.

"Non-aromatic carbocycle" means a ring derived from the above "non-aromatic carbocyclyl".

"Aromatic heterocyclyl" means an aromatic cyclyl having a single ring or two or more rings, which has one or more identical or different heteroatoms optionally selected from O, S, and N in the ring(s).

An aromatic heterocyclyl having two or more rings also includes a fused ring group wherein an aromatic heterocyclyl having a single ring or two or more rings is fused with a ring of the above "aromatic carbocyclyl", and the linking bond may be carried by any of the rings.

The aromatic heterocyclyl having a single ring is preferably a 5- to 8-membered ring, and more preferably a 5-membered or 6-membered ring. Examples of 5-membered aromatic heterocyclyl include pyrrolyl, imidazolyl, pyrazolyl, triazolyl, tetrazolyl, furyl, thienyl, isoxazolyl, oxazolyl, oxadiazolyl, isothiazolyl, thiazolyl and thiadiazolyl. Examples of 6-membered aromatic heterocyclyl include pyridyl, pyridazinyl, pyrimidinyl, pyrazinyl and triazinyl. The aromatic heterocyclyl having two rings is preferably an 8- to 10-membered ring, and more preferably a 9-membered or 10-membered ring. Examples thereof include indolyl, isoindolyl, indazolyl, indolizinyl, quinolinyl, isoquinolinyl, cinnolinyl, phthalazinyl, quinazolinyl, naphthyridinyl, quinoxalinyl, purinyl, pteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, oxazolopyridyl and thiazolopyridyl.

The aromatic heterocyclyl having three or more rings is preferably a 13- to 15-membered group. Examples include carbazolyl, acridinyl, xanthenyl, phenothiazinyl, phenoxathiinyl, phenoxazinyl and dibenzofuryl.

"Aromatic heterocycle" means a ring derived from the above "aromatic heterocyclyl".

The aromatic heterocycle having a single ring is preferably a 5- to 8-membered ring and more preferably a 5-membered or 6- membered ring. Examples of the 5-membered aromatic heterocycle include a pyrroline ring, an imidazoline ring, a pyrazoline ring, a triazole ring, a tetrazole ring, a furan ring, a thiophen ring, an isoxazole ring, an oxazole ring, an oxadiazole ring, an isothiazole ring, a thiazole ring and a thiadiazole ring. Examples of 6-membered aromatic heterocycle include a pyridine ring, a pyridazine ring, a pyrimidine ring, a pyrazine ring and a triazine ring.

The aromatic heterocyclyl having two rings is preferably an 8- to 10-membered ring, and more preferably a 9-membered or 10-membered ring. Examples include an indole ring, an isoindole ring, an indazole ring, an indolizine ring, a quinoline ring, an isoquinoline ring, a cinnoline ring, a phthalazine ring, a quinazoline ring, a naphthyridine ring, a quinoxaline ring, a purine ring, a pteridine ring, a benzimidazole ring, a benzisoxazole ring, a benzoxazole ring, a benzoxadiazole ring, a benzisothiazole ring, a benzothiazole ring, a benzothiadiazole ring, a benzofuran ring, an isobenzofuran ring, a benzothiophene ring, a benzotriazole ring, an imidazopyridine ring, a triazolopyridine ring, an imidazothiazole ring, a pyrazinopyridazine ring, an oxazolopyridine ring and a thiazolopyridine ring.

An aromatic heterocycle having three or more rings is preferably a 13- to 15-membered group. Examples include a carbazole ring, an acridine ring, a xanthene ring, a phenothiazine ring, a phenoxathiin ring, a phenoxazine ring and a dibenzofuran ring.

"Non-aromatic heterocyclyl" means a non-aromatic cyclyl having a single ring or two or more rings, which has one or more identical or different heteroatoms optionally selected from O, S, and N in the ring(s). A non-aromatic heterocyclyl having two or more rings also includes a fused ring group wherein a non-aromatic heterocyclyl having a single ring or two or more rings is fused with a ring in each of the above "aromatic carbocyclyl", "non-aromatic carbocyclyl" and/or "aromatic heterocyclyl", as well as a non-aromatic carbocyclyl having a single ring or two or more rings is fused with a ring in the above "aromatic heterocyclyl", and the linking bond may be carried by any of the rings.

Furthermore, the "non-aromatic heterocyclyl" also includes a group having a bridged group or a group forming a spiro ring, such as follows.

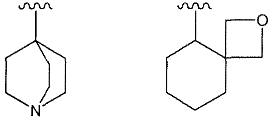

The non-aromatic heterocyclyl having a single ring is preferably a 3- to 8-membered and more preferably a 4- to 6- membered ring.

Examples of the 3-membered non-aromatic heterocyclyl include thiiranyl, oxiranyl and aziridinyl. Examples of 4-membered non-aromatic heterocyclyl include oxetanyl and azetidinyl. Examples of 5-membered non-aromatic heterocyclyl include oxathiolanyl, thiazolidinyl, pyrrolidinyl, pyrrolinyl, imidazolidinyl, imidazolinyl, pyrazolidinyl, pyrazolinyl, tetrahydrofuryl, dihydrothiazolyl, tetrahydroisothiazolyl, dioxolanyl, dioxolyl and thiolanyl. Examples of 6-membered non-aromatic heterocyclyl include dioxanyl, thianyl, piperidyl, piperazinyl, morpholinyl, morpholino, thiomorpholinyl, thiomorpholino, dihydropyridyl, tetrahydropyridyl, tetrahydropyranyl, dihydroxazinyl, tetrahydropyridazinyl, hexahydropyrimidinyl, dioxazinyl, thiinyl and thiazinyl. Examples of the 7-membered non-aromatic heterocyclyl include hexahydroazepinyl, tetrahydrodiazepinyl and oxepanyl.

The non-aromatic heterocyclyl having two or more rings is preferably an 8- to 20-membered group, and more preferably an 8- to 10-membered group. Examples include indolinyl, isoindolinyl, chromanyl and isochromanyl.

"Non-aromatic heterocycle" means a ring derived from the above "non-aromatic heterocyclyl".

The alkyl moiety of "alkyloxy", "haloalkyloxy", "alkylcarbonyloxy", "alkylcarbonyl", "alkyloxycarbonyl", "alkylsulfanyl", "alkylsulfinyl", "alkylsulfonyl", "alkyloxyalkyloxy", and "alkyloxyalkyl" have the same meanings as the aforementioned "alkyl".

The alkenyl moiety of "alkenyloxy", "alkenylcarbonyloxy", "alkenylcarbonyl", "alkenyloxycarbonyl", "alkenylsulfanyl", "alkenylsulfinyl", and "alkenylsulfonyl" have the same meanings as the aforementioned"alkenyl".

The alkynyl moiety of "alkynyloxy", "alkynylcarbonyloxy", "alkynylcarbonyl", "alkynyloxycarbonyl", "alkynylsulfanyl", "alkynylsulfinyl", and "alkynylsulfonyl" is synonymous with the aforementioned"alkynyl".

In the present specification, the phrase "optionally substituted with substituent group A" means that "optionally substituted with one or more group(s) selected from substituent group A". The same applies to substituent groups B, C, D, E, F, α, β, γ, γ', and the like.

Substituents for "substituted alkyl", "substituted alkenyl", "substituted alkynyl", "substituted alkyloxy", "substituted alkenyloxy", "substituted alkynyloxy", "substituted alkylcarbonyloxy", "substituted alkenylcarbonyloxy", "substituted alkynylcarbonyloxy", "substituted alkylcarbonyl", "substituted alkenylcarbonyl", "substituted alkynylcarbonyl", "substituted alkyloxycarbonyl", "substituted alkenyloxycarbonyl", "substituted alkynyloxycarbonyl", "substituted alkylsulfanyl", "substituted alkenylsulfanyl", "substituted alkynylsulfanyl", "substituted alkylsulfinyl", "substituted alkenylsulfinyl", "substituted alkynylsulfinyl", "substituted alkylsulfonyl", "substituted alkenylsulfonyl", "substituted alkynylsulfonyl" and the like include the following substituent group A. A carbon atom at any position may be bonded to one or more group(s) selected from the following substituent group A.

Substituent group A: halogen, hydroxy, carboxy, formyl, formyloxy, sulfanyl, sulfino, sulfo, thioformyl, thiocarboxy, dithiocarboxy, thiocarbamoyl, cyano, nitro, nitroso, azide, hydrazino, ureido, amidino, guanidino, pentafluorothio, trialkylsilyl,
alkyloxy optionally substituted with substituent group α, alkenyloxy optionally substituted with substituent group α, alkynyloxy optionally substituted with substituent group α, alkylcarbonyloxy optionally substituted with substituent group α, alkenylcarbonyloxy optionally substituted with substituent group α, alkynylcarbonyloxy optionally substituted with substituent group α, alkylcarbonyl optionally substituted with substituent group α, alkenylcarbonyl optionally substituted with substituent group α, alkynylcarbonyl optionally substituted with substituent group α, alkyloxycarbonyl optionally substituted with substituent group α, alkenyloxycarbonyl optionally substituted with substituent group α, alkynyloxycarbonyl optionally substituted with substituent group α, alkylsulfanyl optionally substituted with substituent group α, alkenylsulfanyl optionally substituted with substituent group α, alkynylsulfanyl optionally substituted with substituent group α, alkylsulfinyl optionally substituted with substituent group α, alkenylsulfinyl optionally substituted with substituent group α, alkynylsulfinyl optionally substituted with substituent group α, alkylsulfonyl optionally substituted with substituent group α, alkenylsulfonyl optionally substituted with substituent group α, alkynylsulfonyl optionally substituted with substituent group α,
amino optionally substituted with substituent group β, imino optionally substituted with substituent group β, carbamoyl optionally substituted with substituent group β, sulfamoyl optionally substituted with substituent group β,
aromatic carbocyclyl optionally substituted with substituent group γ, non-aromatic carbocyclyl optionally substituted with substituent group y', aromatic heterocyclyl optionally substituted with substituent group γ, non-aromatic heterocyclyl optionally substituted with substituent group y', aromatic carbocyclyloxy optionally substituted with substituent group γ, non-aromatic carbocyclyloxy optionally substituted with substituent group y', aromatic heterocyclyloxy optionally substituted with substituent group γ, non-aromatic heterocyclyloxy optionally substituted with substituent group y', aromatic carbocyclylcarbonyloxy optionally substituted with substituent group γ, non-aromatic carbocyclylcarbonyloxy optionally substituted with substituent group y', aromatic heterocyclylcarbonyloxy optionally substituted with substituent group γ, non-aromatic heterocyclylcarbonyloxy optionally substituted with substituent group y', aromatic carbocyclylcarbonyl optionally substituted with substituent group γ, non-aromatic carbocyclylcarbonyl optionally substituted with substituent group y', aromatic heterocyclylcarbonyl optionally substituted with substituent group γ, non-aromatic heterocyclylcarbonyl optionally substituted with substituent group y', aromatic carbocyclyloxycarbonyl optionally substituted with substituent group γ, non-aromatic carbocyclyloxycarbonyl optionally substituted with substituent group y', aromatic heterocyclyloxycarbonyl optionally substituted with substituent group γ, non-aromatic heterocyclyloxycarbonyl optionally substituted with substituent group y', aromatic carbocyclylalkyloxy optionally substituted with substituent group γ, non-aromatic carbocyclylalkyloxy optionally substituted with substituent group y', aromatic heterocyclylalkyloxy optionally substituted with substituent group γ, non-aromatic heterocyclylalkyloxy optionally substituted with substituent group y', aromatic carbocyclylalkyloxycarbonyl optionally substituted with substituent group γ, non-aromatic carbocyclylalkyloxycarbonyl optionally substituted with substituent group y', aromatic heterocyclylalkyloxycarbonyl optionally substituted with substituent group γ, non-aromatic heterocyclylalkyloxycarbonyl optionally substituted with substituent group y', aromatic carbocyclylsulfanyl optionally substituted with substituent group γ, non-aromatic carbocyclylsulfanyl optionally substituted with substituent group y', aromatic heterocyclylsulfanyl optionally substituted with substituent group γ, non-aromatic heterocyclylsulfanyl optionally substituted with substituent group y', aromatic carbocyclylsulfinyl optionally substituted with substituent group γ, non-aromatic carbocyclylsulfinyl optionally substituted with substituent group y', aromatic heterocyclylsulfinyl optionally substituted with substituent group γ, non-aromatic heterocyclylsulfinyl optionally substituted with substituent group y', aromatic carbocyclylsulfonyl optionally substituted with substituent group γ, non-aromatic carbocyclylsulfonyl optionally substituted with substituent group y', aromatic heterocyclylsulfonyl optionally substituted with substituent group γ, and non-aromatic heterocyclylsulfonyl optionally substituted with substituent group y'.

Substituent group α: halogen, hydroxy, carboxy, alkyloxy, haloalkyloxy, alkenyloxy, alkynyloxy, sulfanyl and cyano.

Substituent group β: halogen, hydroxy, carboxy, cyano, alkyl optionally substituted with substituent group α, alkenyl optionally substituted with substituent group α, alkynyl optionally substituted with substituent group α, alkylcarbonyl optionally substituted with substituent group α, alkenylcarbonyl optionally substituted with substituent group α, alkynylcarbonyl optionally substituted with substituent group α, alkylsulfanyl optionally substituted with substituent group α, alkenylsulfanyl optionally substituted with substituent group α, alkynylsulfanyl optionally substituted with substituent group α, alkylsulfinyl optionally substituted with substituent group α, alkenylsulfinyl optionally substituted with substituent group α, alkynylsulfinyl optionally substituted with substituent group α, alkylsulfonyl optionally substituted with substituent group α, alkenylsulfonyl optionally substituted with substituent group α, alkynylsulfonyl optionally substituted with substituent group α,
aromatic carbocyclyl optionally substituted with substituent group γ, non-aromatic carbocyclyl optionally substituted with substituent group y', aromatic heterocyclyl optionally substituted with substituent group γ, non-aromatic heterocyclyl optionally substituted with substituent group y', aromatic carbocyclylalkyl optionally substituted with substituent group γ, non-aromatic carbocyclylalkyl optionally substituted with substituent group y', aromatic heterocyclylalkyl optionally substituted with substituent group γ, non-aromatic heterocyclylalkyl optionally substituted with substituent group y', aromatic carbocyclylcarbonyl optionally substituted with substituent group γ, non-aromatic carbocyclylcarbonyl optionally substituted with substituent group y', aromatic heterocyclylcarbonyl optionally substituted with substituent group γ, non-aromatic heterocyclylcarbonyl optionally substituted with substituent group y', aromatic carbocyclyloxycarbonyl optionally substituted with substituent group γ, non-aromatic carbocyclyloxycarbonyl optionally substituted with substituent group y', aromatic heterocyclyloxycarbonyl optionally substituted with substituent group γ, non-aromatic heterocyclyloxycarbonyl optionally substituted with substituent group y', aromatic carbocyclylsulfanyl optionally substituted with substituent group γ, non-aromatic carbocyclylsulfanyl optionally substituted with substituent group y', aromatic heterocyclylsulfanyl optionally substituted with substituent group γ, non-aromatic heterocyclylsulfanyl optionally substituted with substituent group y', aromatic carbocyclylsulfinyl optionally substituted with substituent group γ, non-aromatic carbocyclylsulfinyl optionally substituted with substituent group y', aromatic heterocyclylsulfinyl optionally substituted with substituent group γ, non-aromatic heterocyclylsulfinyl optionally substituted with substituent group y', aromatic carbocyclylsulfonyl optionally substituted with substituent group γ, non-aromatic carbocyclylsulfonyl optionally substituted with substituent group y', aromatic heterocyclylsulfonyl optionally substituted with substituent group γ, and non-aromatic heterocyclylsulfonyl optionally substituted with substituent group y'.

Substituent group γ: substituent group α, alkyl, haloalkyl, cyanoalkyl, hydroxyalkyl, alkenyl, alkynyl, alkylcarbonyl, haloalkylcarbonyl, alkenylcarbonyl, and alkynylcarbonyl.

Substituent group y': substituent group y and oxo.

The substituents on the rings of "aromatic carbocycle" and "aromatic heterocycle", such as "substituted aromatic carbocyclyl", "substituted aromatic heterocyclyl", "substituted aromatic carbocyclyloxy", "substituted aromatic heterocyclyloxy", "substituted aromatic carbocyclylcarbonyloxy", "substituted aromatic heterocyclylcarbonyloxy", "substituted aromatic carbocyclylcarbonyl", "substituted aromatic heterocyclylcarbonyl", "substituted aromatic carbocyclyloxycarbonyl", "substituted aromatic heterocyclyloxycarbonyl", "substituted aromatic carbocyclylsulfanyl", "substituted aromatic heterocyclylsulfanyl", "substituted aromatic carbocyclylsulfinyl", "substituted aromatic heterocyclylsulfinyl", "substituted aromatic carbocyclylsulfonyl", and "substituted aromatic heterocyclylsulfonyl" include the following substituent group B. An atom at any position on the ring may be bonded to one or more group(s) selected from the following substituent group B.

Substituent group B: halogen, hydroxy, carboxy, formyl, formyloxy, sulfanyl, sulfino, sulfo, thioformyl, thiocarboxy, dithiocarboxy, thiocarbamoyl, cyano, nitro, nitroso, azide, hydrazino, ureido, amidino, guanidino, pentafluorothio, trialkylsilyl,
alkyl optionally substituted with substituent group α, alkenyl optionally substituted with substituent group α, alkynyl optionally substituted with substituent group α, alkyloxy optionally substituted with substituent group α, alkenyloxy optionally substituted with substituent group α, alkynyloxy optionally substituted with substituent group α, alkylcarbonyloxy optionally substituted with substituent group α, alkenylcarbonyloxy optionally substituted with substituent group α, alkynylcarbonyloxy optionally substituted with substituent group α, alkylcarbonyl optionally substituted with substituent group α, alkenylcarbonyl optionally substituted with substituent group α, alkynylcarbonyl optionally substituted with substituent group α, alkyloxycarbonyl optionally substituted with substituent group α, alkenyloxycarbonyl optionally substituted with substituent group α, alkynyloxycarbonyl optionally substituted with substituent group α, alkylsulfanyl optionally substituted with substituent group α, alkenylsulfanyl optionally substituted with substituent group α, alkynylsulfanyl optionally substituted with substituent group α, alkylsulfinyl optionally substituted with substituent group α, alkenylsulfinyl optionally substituted with substituent group α, alkynylsulfinyl optionally substituted with substituent group α, alkylsulfonyl optionally substituted with substituent group α, alkenylsulfonyl optionally substituted with substituent group α, alkynylsulfonyl optionally substituted with substituent group α,
amino optionally substituted with substituent group β, imino optionally substituted with substituent group β, carbamoyl optionally substituted with substituent group β, sulfamoyl optionally substituted with substituent group β,
aromatic carbocyclyl optionally substituted with substituent group γ, non-aromatic carbocyclyl optionally substituted with substituent group y', aromatic heterocyclyl optionally substituted with substituent group γ, non-aromatic heterocyclyl optionally substituted with substituent group y', aromatic carbocyclyloxy optionally substituted with substituent group γ, non-aromatic carbocyclyloxy optionally substituted with substituent group y', aromatic heterocyclyloxy optionally substituted with substituent group γ, non-aromatic heterocyclyloxy optionally substituted with substituent group y', aromatic carbocyclylcarbonyloxy optionally substituted with substituent group γ, non-aromatic carbocyclylcarbonyloxy optionally substituted with substituent group y', aromatic heterocyclylcarbonyloxy optionally substituted with substituent group γ, and non-aromatic heterocyclylcarbonyloxy optionally substituted with substituent group y', aromatic carbocyclylcarbonyl optionally substituted with substituent group γ, non-aromatic carbocyclylcarbonyl optionally substituted with substituent group y', aromatic heterocyclylcarbonyl optionally substituted with substituent group γ, non-aromatic heterocyclylcarbonyl optionally substituted with substituent group y', aromatic carbocyclyloxycarbonyl optionally substituted with substituent group γ, non-aromatic carbocyclyloxycarbonyl optionally substituted with substituent group y', aromatic heterocyclyloxycarbonyl optionally substituted with substituent group γ, non-aromatic heterocyclyloxycarbonyl optionally substituted with substituent group y', aromatic carbocyclylalkyl optionally substituted with substituent group γ, non-aromatic carbocyclylalkyl optionally substituted with substituent group y', aromatic heterocyclylalkyl optionally substituted with substituent group γ, non-aromatic heterocyclylalkyl optionally substituted with substituent group y', aromatic carbocyclylalkyloxy optionally substituted with substituent group γ, non-aromatic carbocyclylalkyloxy optionally substituted with substituent group y', aromatic heterocyclylalkyloxy optionally substituted with substituent group γ, non-aromatic heterocyclylalkyloxy optionally substituted with substituent group y', aromatic carbocyclylalkyloxycarbonyl optionally substituted with substituent group γ, non-aromatic carbocyclylalkyloxycarbonyl optionally substituted with substituent group y', aromatic heterocyclylalkyloxycarbonyl optionally substituted with substituent group γ, non-aromatic heterocyclylalkyloxycarbonyl optionally substituted with substituent group y', aromatic carbocyclylalkyloxyalkyl optionally substituted with substituent group γ, non-aromatic carbocyclylalkyloxyalkyl optionally substituted with substituent group y', aromatic heterocyclylalkyloxyalkyl optionally substituted with substituent group γ, non-aromatic heterocyclylalkyloxyalkyl optionally substituted with substituent group y', aromatic carbocyclylsulfanyl optionally substituted with substituent group γ, non-aromatic carbocyclylsulfanyl optionally substituted with substituent group y', aromatic heterocyclylsulfanyl optionally substituted with substituent group γ, non-aromatic heterocyclylsulfanyl optionally substituted with substituent group y', aromatic carbocyclylsulfinyl optionally substituted with substituent group γ, non-aromatic carbocyclylsulfinyl optionally substituted with substituent group y', aromatic heterocyclylsulfinyl optionally substituted with substituent group γ, non-aromatic heterocyclylsulfinyl optionally substituted with substituent group y', aromatic carbocyclylsulfonyl optionally substituted with substituent group γ, non-aromatic carbocyclylsulfonyl optionally substituted with substituent group y', aromatic heterocyclylsulfonyl optionally substituted with substituent group γ, and non-aromatic heterocyclylsulfonyl optionally substituted with substituent group y'.

Cyclic substituents for "non-aromatic carbocycle" and "aromatic heterocycle" of "substituted non-aromatic carbocyclyl", "substituted non-aromatic heterocyclyl", "substituted non-aromatic carbocyclyloxy", "substituted non-aromatic heterocyclyloxy", "substituted non-aromatic carbocyclylcarbonyloxy", "substituted non-aromatic heterocyclylcarbonyloxy", "substituted non-aromatic carbocyclylcarbonyl", "substituted non-aromatic heterocyclylcarbonyl", "substituted non-aromatic carbocyclyloxycarbonyl", "substituted non-aromatic heterocyclyloxycarbonyl", "substituted non-aromatic carbocyclylsulfanyl", "substituted non-aromatic heterocyclylsulfanyl", "substituted non-aromatic carbocyclylsulfinyl", "substituted non-aromatic heterocyclylsulfinyl", "substituted non-aromatic carbocyclylsulfonyl" and "substituted non-aromatic heterocyclylsulfonyl" include the following substituent group C. An atom at an arbitrary position on the ring may be bonded to one or more groups selected from the following substituent group C.

Substituent group C: substituent group B and oxo.

When the "non-aromatic carbocycle", the "non-aromatic heterocycle", the "non-aromatic carbocyclyl" and the "non-aromatic heterocyclyl" are substituted with "oxo", this means a ring in which two hydrogen atoms on a carbon atom are substituted as follows.

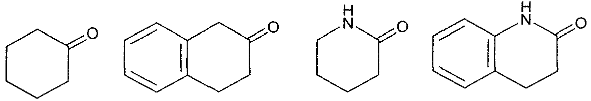

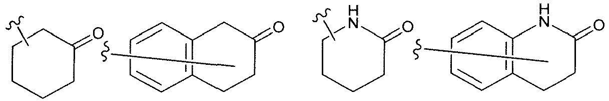

Examples of the substituents for "substituted amino", "substituted imino", "substituted carbamoyl" and "substituted sulfamoyl" include the following substituent group D. These moieties may be substituted with one or two group(s) selected from substituent group D.

Substituent group D: halogen, hydroxy, carboxy, cyano, alkyl optionally substituted with substituent group α, alkenyl optionally substituted with substituent group α, alkynyl optionally substituted with substituent group α, alkylcarbonyl optionally substituted with substituent group α, alkenylcarbonyl optionally substituted with substituent group α, alkynylcarbonyl optionally substituted with substituent group α, alkylsulfanyl optionally substituted with substituent group α, alkenylsulfanyl optionally substituted with substituent group α, alkynylsulfanyl optionally substituted with substituent group α, alkylsulfinyl optionally substituted with substituent group α, alkenylsulfinyl optionally substituted with substituent group α, alkynylsulfinyl optionally substituted with substituent group α, alkylsulfonyl optionally substituted with substituent group α, alkenylsulfonyl optionally substituted with substituent group α, alkynylsulfonyl optionally substituted with substituent group α,
amino optionally substituted with substituent group β, imino optionally substituted with substituent group β, carbamoyl optionally substituted with substituent group β, sulfamoyl optionally substituted with substituent group β,
aromatic carbocyclyl optionally substituted with substituent group γ, non-aromatic carbocyclyl optionally substituted with substituent group y', aromatic heterocyclyl optionally substituted with substituent group γ, non-aromatic heterocyclyl optionally substituted with substituent group y', aromatic carbocyclylalkyl optionally substituted with substituent group γ, non-aromatic carbocyclylalkyl optionally substituted with substituent group y', aromatic heterocyclylalkyl optionally substituted with substituent group γ, non-aromatic heterocyclylalkyl optionally substituted with substituent group y', aromatic carbocyclylcarbonyl optionally substituted with substituent group γ, non-aromatic carbocyclylcarbonyl optionally substituted with substituent group y', aromatic heterocyclylcarbonyl optionally substituted with substituent group γ, non-aromatic heterocyclylcarbonyl optionally substituted with substituent group y', aromatic carbocyclyloxycarbonyl optionally substituted with substituent group γ, non-aromatic carbocyclyloxycarbonyl optionally substituted with substituent group y', aromatic heterocyclyloxycarbonyl optionally substituted with substituent group γ, non-aromatic heterocyclyloxycarbonyl optionally substituted with substituent group y', aromatic carbocyclylsulfanyl optionally substituted with substituent group γ, non-aromatic carbocyclylsulfanyl optionally substituted with substituent group y', aromatic heterocyclylsulfanyl optionally substituted with substituent group γ, non-aromatic heterocyclylsulfanyl optionally substituted with substituent group y', aromatic carbocyclylsulfinyl optionally substituted with substituent group γ, non-aromatic carbocyclylsulfinyl optionally substituted with substituent group y', aromatic heterocyclylsulfinyl optionally substituted with substituent group γ, non-aromatic heterocyclylsulfinyl optionally substituted with substituent group y', aromatic carbocyclylsulfonyl optionally substituted with substituent group γ, non-aromatic carbocyclylsulfonyl optionally substituted with substituent group y', aromatic heterocyclylsulfonyl optionally substituted with substituent group γ, and non-aromatic heterocyclylsulfonyl optionally substituted with substituent group y'.

In formula (II), the bond attached with *1 of -L- is bonded to R³, and the bond attached with *2 of -L- is bonded to an imidazole ring.

A preferred embodiment of each symbol in the compound represented by formula (I) is described below. Examples of the compound represented by formula (I) include embodiments of all combinations of specific examples described below.

A₁ is C(R¹) or N.
A₁ is preferably C(R¹).

R¹ is a hydrogen atom or halogen.
R¹ is preferably a hydrogen atom or a fluorine atom.

R⁸ is a hydrogen atom, halogen, alkyloxy, or 5- or 6-membered aromatic heterocyclyl optionally substituted with halogen or alkyl.

R⁸ is preferably a hydrogen atom, a fluorine atom, methyloxy, or methylpyrazolyl, and more preferably a hydrogen atom, a fluorine atom, or methyloxy.

B₁ is CH or N.
B₁ is preferably N.

Another embodiment of B₁ is CH.

R¹⁰ is a hydrogen atom, cyano, a fluorine atom, a chlorine atom, methyl, difluoromethyl, or trifluoromethyl.

R¹⁰ is preferably a chlorine atom, methyl, difluoromethyl, or trifluoromethyl, and more preferably trifluoromethyl.

Another exemplary embodiment of R¹⁰ is a hydrogen atom, cyano, a fluorine atom, a chlorine atom, methyl, or difluoromethyl.

Another exemplary embodiment of R¹⁰ is a chlorine atom, or difluoromethyl.

Here, (i) when R¹⁰ is a chlorine atom, B₁ is N,
(ii) when R¹⁰ is trifluoromethyl, R³ is 5-membered aromatic heterocyclyl optionally substituted with substituent group F, bicyclic 9- or 10-membered aromatic heterocyclyl optionally substituted with substituent group F, or 5- to 12-membered non-aromatic heterocyclyl optionally substituted with substituent group F, or R⁸ is 5- or 6-membered aromatic heterocyclyl optionally substituted with halogen or alkyl, and
(iii) the compound is other than those below: 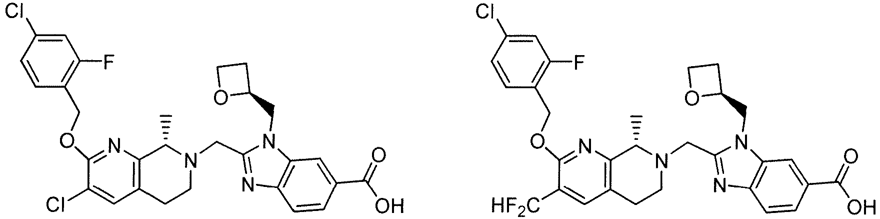 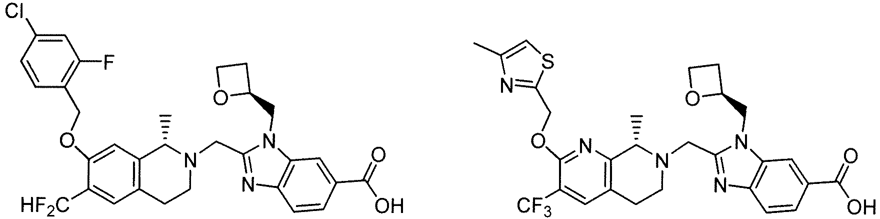 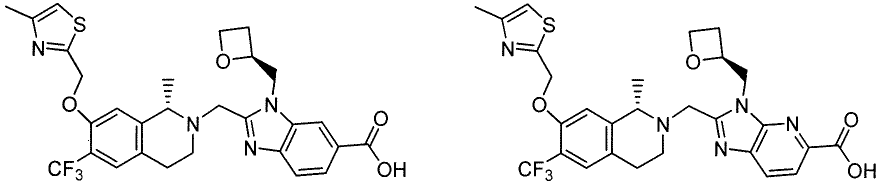 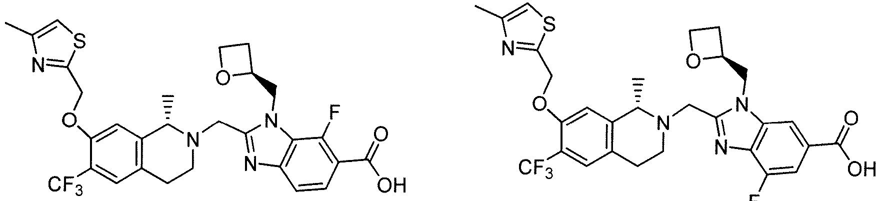 and

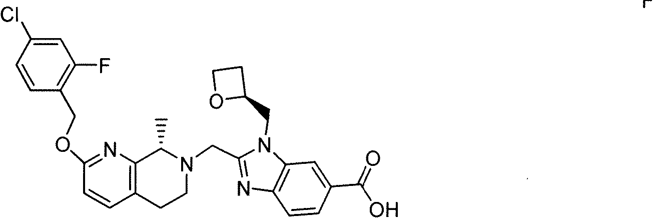

Substituent group F: halogen, cyano, alkyl, haloalkyl, alkyloxy, and haloalkyloxy.

A preferable embodiment of the substituent group F is a fluorine atom, a chlorine atom, cyano, methyl, methyloxy, or difluoromethyloxy.

R³ is phenyl optionally substituted with substituent group F, 5- or 6-membered aromatic heterocyclyl optionally substituted with substituent group F, bicyclic 9- or 10-membered aromatic heterocyclyl optionally substituted with substituent group F, or 5- to 12-membered non-aromatic heterocyclyl optionally substituted with substituent group F.

R³ is preferably the following group: 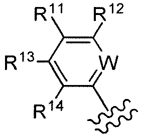 wherein
W is N, or CR¹⁵,
R¹¹ is a hydrogen atom, halogen, cyano, alkyl, haloalkyl, alkyloxy, or haloalkyloxy,
R¹² and R¹³ are each independently a hydrogen atom or halogen,
R¹⁴ and R¹⁵ are each independently a hydrogen atom, halogen, cyano, alkyl, haloalkyl, alkyloxy, or haloalkyloxy,
R¹¹ and R¹² may be taken together to form 5-membered aromatic heterocycle optionally substituted with substituent group F, or 5- to 7-membered non-aromatic heterocycle optionally substituted with substituent group F,
R¹¹ and R¹³ may be taken together to form 5-membered aromatic heterocycle optionally substituted with substituent group F, or 5- to 7-membered non-aromatic heterocycle optionally substituted with substituent group F, and
R¹³ and R¹⁴ may be taken together to form 5-membered aromatic heterocycle optionally substituted with substituent group F, or 5- to 7-membered non-aromatic heterocycle optionally substituted with substituent group F.
R³ is more preferably any one of the following groups: 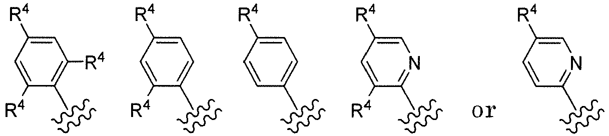 wherein R⁴s are each independently halogen, cyano, alkyl, haloalkyl, alkyloxy, or haloalkyloxy.

R⁴s are preferably each independently a fluorine atom, a chlorine atom, cyano, methyl, methyloxy, or difluoromethyloxy.

When R¹⁰ is trifluoromethyl, another exemplary embodiment of R³ is 5-membered aromatic heterocyclyl optionally substituted with substituent group F, bicyclic 9- or 10-membered aromatic heterocyclyl optionally substituted with substituent group F, or bicyclic 8- to 11-membered non-aromatic heterocyclyl optionally substituted with substituent group F.

A preferred embodiment of each symbol in the compound represented by the formula (II) is described below. Regarding the compound represented by formula (II), embodiments of all the combinations of specific examples shown below are mentioned as examples.

Q is a substituted or unsubstituted benzene ring or substituted or unsubstituted 5- or 6-membered aromatic heterocycle.

Q is more preferably any one of the following groups: 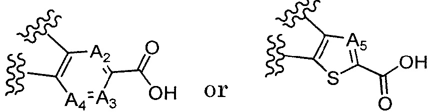 wherein
A₂ is C(R⁵) or N,
A₃ is C(R⁶) or N,
A₄ is C(R⁷) or N,
R⁵, R⁶ and R⁷ are each independently a hydrogen atom, halogen, cyano, substituted or unsubstituted alkyl (wherein the substituent is, for example, halogen), substituted or unsubstituted alkyloxy (wherein the substituent is, for example, halogen), substituted or unsubstituted aromatic heterocyclyl (wherein the substituent is, for example, halogen, alkyl, haloalkyl, alkyloxy, haloalkyloxy), or substituted or unsubstituted non-aromatic carbocyclyl (wherein the substituent is, for example, halogen, alkyl, haloalkyl, alkyloxy, haloalkyloxy),
A₅ is C(R⁹) or N, and
R⁹ is each independently a hydrogen atom, halogen, cyano, substituted or unsubstituted alkyl (wherein the substituent is, for example, halogen), or substituted or unsubstituted alkyloxy (wherein the substituent is, for example, halogen).
Q is preferably a group below: 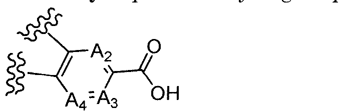 wherein each symbol is as defined above.

A₂, A₃ and A₄ are preferably
(i) A₂ is C(R⁵), A₃ is C(R⁶), and A₄ is C(R⁷),
(ii) A₂ is N, A₃ is C(R⁶), and A₄ is C(R⁷),
(iii) A₂ is C(R⁵), A₃ is C(R⁶), and A₄ is N, or
(iv) A₂ is N, A₃ is C(R⁶), and A₄ is N.

A₂, A₃ and A₄ are more preferably
(i) A₂ is C(R⁵), A₃ is C(R⁶), and A₄ is C(R⁷), or
(ii) A₂ is N, A₃ is C(R⁶), and A₄ is C(R⁷).

R⁵, R⁶ and R⁷ are preferably each independently a hydrogen atom, halogen, alkyl, alkyloxy, or 5- or 6-membered aromatic heterocyclyl optionally substituted with substituent group E.

Substituent group E: halogen, alkyl, haloalkyl, alkyloxy, and haloalkyloxy.

The substituent group E is preferably a group selected from halogen and alkyl.

R⁵ is preferably a hydrogen atom or halogen, and more preferably a hydrogen atom or a fluorine atom.

R⁶ is preferably a hydrogen atom.

R⁷ is preferably a hydrogen atom, halogen, alkyloxy, or methylpyrazolyl, and more preferably a hydrogen atom, a fluorine atom, methyloxy, or methylpyrazolyl.

A₅ is preferably C(R⁹).

R⁹ is preferably a hydrogen atom, halogen, cyano, alkyl, or alkyloxy, and more preferably a hydrogen atom.

R² is substituted or unsubstituted alkyl, or substituted or unsubstituted non-aromatic heterocyclyl.

R² is preferably alkyl, alkyl substituted with non-aromatic heterocyclyl, or alkyl substituted with aromatic heterocyclyl, and more preferably oxetanylalkyl or alkylimidazolylalkyl.

R² is most preferably oxetanylmethyl.

-L- is any one of groups below:

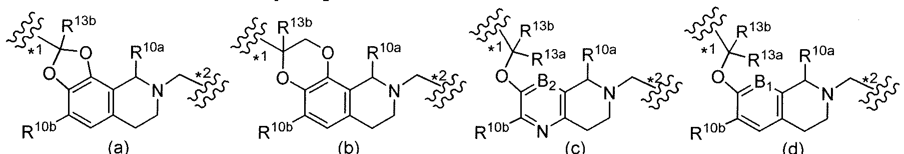

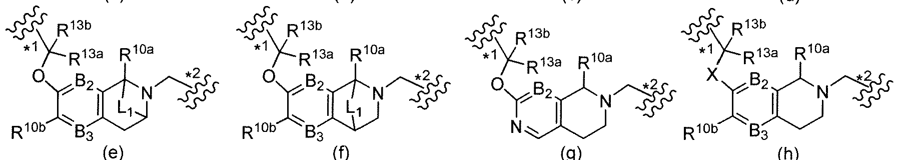

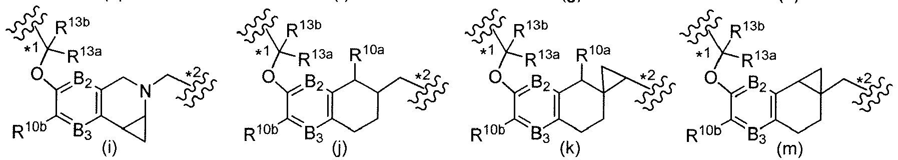

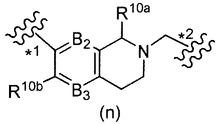

-L- is preferably a group represented by the above (a), (c), (d), (e), (f), (g), (h) or (j), and more preferably the above (a), (c), (d), (g), (h) or (j). -L- is more preferably the above (a), (c), or (d).

Another preferred embodiment of -L- is the above (c) or (h).

Here, (i) when -L- is a group represented by (d), Q is a benzene ring substituted with substituted or unsubstituted aromatic heterocyclyl (wherein the substituent is, for example, halogen, alkyl, haloalkyl, alkyloxy, haloalkyloxy), 6-membered aromatic heterocycle substituted with substituted or unsubstituted aromatic heterocyclyl (wherein the substituent is, for example, halogen, alkyl, haloalkyl, alkyloxy, haloalkyloxy), or substituted or unsubstituted 5-membered aromatic heterocycle (wherein the substituent is, for example, halogen, alkyl, haloalkyl, alkyloxy, haloalkyloxy). Preferably Q is a benzene ring substituted with a substituted or unsubstituted aromatic heterocyclyl (wherein the substituent is, for example, halogen, alkyl, haloalkyl, alkyloxy, haloalkyloxy), or substituted or unsubstituted 5-membered aromatic heterocycle (wherein the substituent is, for example, halogen, alkyl, haloalkyl, alkyloxy, haloalkyloxy).

B₁ is CH or N, and preferably N.

B₂ is C(R⁹) or N, and preferably N.

Another preferred embodiment of B₂ is C(R⁹).

B₃ is CH or N, and preferably CH.

R⁹ is a hydrogen atom or R⁹ and R^{13a} are taken together to form -O- or -CH₂-O-(the right-side bond is bonded to the benzene ring), and R⁹ is preferably a hydrogen atom.

As another embodiment of R⁹, R⁹ and R^{13a} are taken together to form -O-.

R^{13a} is a hydrogen atom or R^{13a} and R⁹ are taken together to form -O- or -CH₂-O- (the right-side bond is bonded to the benzene ring), and R^{13a} is preferably a hydrogen atom.

As another embodiment of R^{13a}, R^{13a} and R⁹ are taken together to form -O-.

R^{13b} is a hydrogen atom, or substituted or unsubstituted alkyl (wherein the substituent is, for example, halogen), and is preferably a hydrogen atom or alkyl.

R^{10a} is a hydrogen atom, or substituted or unsubstituted alkyl (wherein the substituent is, for example, halogen), and preferably methyl.

R^{10b} is a hydrogen atom, cyano, halogen, or substituted or unsubstituted alkyl (wherein the substituent is, for example, halogen), and preferably a hydrogen atom, cyano, fluorine atom, chlorine atom, methyl, difluoromethyl, or trifluoromethyl. R^{10b} is more preferably trifluoromethyl.

-L₁- is alkylene, preferably C1 to C3 alkylene, and more preferably ethylene.

X is S, NH or CH₂, and preferably NH.

Examples of R³ include the same group as in formula (I) above, and preferred groups thereof are also the same.

### (Embodiment 1)

A compound represented by formula (II-1): 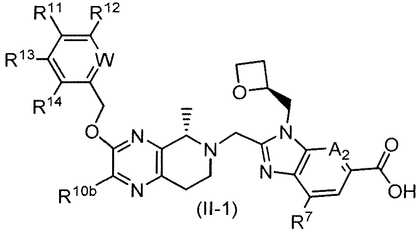 wherein
A₂ is C(R⁵) or N,
R⁵ is a hydrogen atom or halogen,
R⁷ is a hydrogen atom, halogen, alkyloxy, or 5- or 6-membered aromatic heterocyclyl optionally substituted with halogen or alkyl,
R^{10b} is cyano, a fluorine atom, a chlorine atom, methyl, difluoromethyl, or trifluoromethyl,
W is N or CR¹⁵,
R¹¹ is a hydrogen atom, halogen, cyano, alkyl, haloalkyl, alkyloxy, or haloalkyloxy,
R¹² and R¹³ are each independently a hydrogen atom or halogen,
R¹⁴ and R¹⁵ are each independently a hydrogen atom, halogen, cyano, alkyl, haloalkyl, alkyloxy, or haloalkyloxy,
R¹¹ and R¹² may be taken together to form 5-membered aromatic heterocycle optionally substituted with substituent group F, or 5- to 7-membered non-aromatic heterocycle optionally substituted with substituent group F,
R¹¹ and R¹³ may be taken together to form 5-membered aromatic heterocycle optionally substituted with substituent group F, or 5- to 7-membered non-aromatic heterocycle optionally substituted with substituent group F, and
R¹³ and R¹⁴ may be taken together to form 5-membered aromatic heterocycle optionally substituted with substituent group F, or 5- to 7-membered non-aromatic heterocycle optionally substituted with substituent group F,
or a pharmaceutically acceptable salt thereof.

### (Embodiment 2)

A compound represented by formula (II-2): 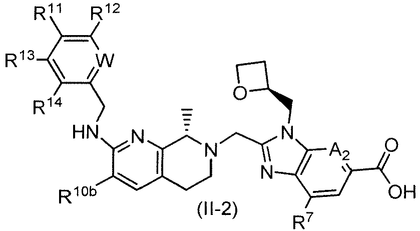 wherein
A₂ is C(R⁵) or N,
R⁵ is a hydrogen atom or halogen,
R⁷ is a hydrogen atom, halogen, alkyloxy, or 5- or 6-membered aromatic heterocyclyl optionally substituted with halogen or alkyl,
R^{10b} is cyano, a fluorine atom, a chlorine atom, methyl, difluoromethyl, or trifluoromethyl,
W is N or CR¹⁵,
R¹¹ is a hydrogen atom, halogen, cyano, alkyl, haloalkyl, alkyloxy, or haloalkyloxy,
R¹² and R¹³ are each independently a hydrogen atom or halogen,
R¹⁴ and R¹⁵ are each independently a hydrogen atom, halogen, cyano, alkyl, haloalkyl, alkyloxy, or haloalkyloxy,
R¹¹ and R¹² may be taken together to form 5-membered aromatic heterocycle optionally substituted with substituent group F, or 5- to 7-membered non-aromatic heterocycle optionally substituted with substituent group F,
R¹¹ and R¹³ may be taken together to form 5-membered aromatic heterocycle optionally substituted with substituent group F, or 5- to 7-membered non-aromatic heterocycle optionally substituted with substituent group F, and
R¹³ and R¹⁴ may be taken together to form 5-membered aromatic heterocycle optionally substituted with substituent group F, or 5- to 7-membered non-aromatic heterocycle optionally substituted with substituent group F,
or a pharmaceutically acceptable salt thereof.

### (Embodiment 3)

The compound according to embodiment 1 or embodiment 2 or a pharmaceutically acceptable salt thereof, wherein R^{10b} is trifluoromethyl.

### (Embodiment 4)

The compound according to any one of embodiment 1 to 3 or a pharmaceutically acceptable salt thereof, wherein R⁷ is alkyloxy, or 5- or 6-membered aromatic heterocyclyl optionally substituted with halogen or alkyl.

The compounds represented by formula (I), formula (II), formula (II-1), or formula (II-2) are not limited to specific isomers, but include all possible isomers (e.g., keto-enol isomers, imine-enamine isomers, diastereoisomers, optical isomers, rotational isomers, tautomers as shown below, etc.), racemates, or mixtures thereof.

One or more hydrogen, carbon, and/or other atoms of the compounds represented by formula (I), formula (II), formula (II-1), or formula (II-2) may be substituted with isotopes of hydrogen, carbon, and/or other atoms, respectively. Examples of such isotopes include hydrogen, carbon, nitrogen, oxygen, phosphorus, sulfur, fluorine, iodine, and chlorine, as in the cases of ²H, ³H, ¹¹C, ¹³C, ¹⁴C, ¹⁵N, ¹⁸O, ¹⁷O, ³¹P, ³²P, ³⁵S, ¹⁸F, ¹²³I, and ³⁶Cl, respectively. The compounds represented by formula (I), formula (II), formula (II-1), or formula (II-2) also include compounds substituted with such isotopes. The compounds substituted with the isotopes are also useful as pharmaceutical products and include all isotopes and radiolabeled forms of the compounds represented by formula (I), formula (II), formula (II-1), or formula (II-2). Furthermore, a "radiolabeling method" for producing the "radiolabeled forms" is also included in the present invention, and the "radiolabeled forms" are useful as tools for metabolic pharmacokinetics studies, studies on binding assay, and/or diagnostics.

Radiolabeled forms of the compounds represented by formula (I), formula (II), formula (II-1), or formula (II-2) can be prepared by methods well known in the pertinent art. For example, a tritium-labeled compound represented by formula (I), formula (II), formula (II-1), or formula (II-2) can be prepared by introducing tritium into a specific compound represented by formula (I), formula (II), formula (II-1), or formula (II-2), by a catalytic dehalogenation reaction using tritium. This method comprises reacting an appropriately-halogenated precursor of the compound represented by formula (I), formula (II), formula (II-1), or formula (II-2) with tritium gas in the presence of an appropriate catalyst, such as Pd/C, and in the presence or absence of a base. Regarding other appropriate methods for preparing tritium-labeled compounds, "Isotopes in the Physical and Biomedical Sciences, Vol. 1, Labeled Compounds (Part A), Chapter 6 (1987)" can be referred to. A ¹⁴C-labeled compound can be prepared by using a raw material having ¹⁴C carbon.

Examples of the pharmaceutically acceptable salts of the compounds represented by formula (I), formula (II), formula (II-1), or formula (II-2) include salts of compounds represented by formula (I), formula (II), formula (II-1), or formula (II-2) with alkaline metal (e.g., lithium, sodium, or potassium), alkaline earth metal (e.g., calcium or barium), magnesium, transition metal (e.g., zinc or iron), ammonia, organic bases (e.g., trimethylamine, triethylamine, dicyclohexylamine, ethanolamine, diethanolamine, triethanolamine, meglumine, ethylenediamine, pyridine, picoline, or quinoline), or amino acids, or salts with inorganic acids (e.g., hydrochloric acid, sulfuric acid, nitric acid, carbonic acid, hydrobromic acid, phosphoric acid, or hydroiodic acid) or organic acids (e.g., formic acid, acetic acid, propionic acid, trifluoroacetic acid, citric acid, lactic acid, tartaric acid, oxalic acid, maleic acid, fumaric acid, succinic acid, mandelic acid, glutaric acid, malic acid, benzoic acid, phthalic acid, ascorbic acid, benzenesulfonic acid, p-toluenesulfonic acid, methanesulfonic acid, ethanesulfonic acid, or trifluoroacetic acid). Particularly, examples of salts include hydrochloric acid, phosphoric acid, tartaric acid, or methanesulfonic acid. These salts can be formed according to methods that are conventionally carried out.

The compounds represented by formula (I), formula (II), formula (II-1), or formula (II-2) of the present invention or pharmaceutically acceptable salts thereof may form solvates (e.g., hydrates), co-crystals, and/or crystal polymorphs. The present invention encompasses those various solvates, co-crystals, and crystal polymorphs. The "solvates" may have the compounds represented by formula (I), formula (II), formula (II-1), or formula (II-2) coordinated with any number of solvent molecules (e.g., water molecules). When the compounds represented by formula (I), formula (II), formula (II-1), or formula (II-2), or pharmaceutically acceptable salts thereof are allowed to stand in the atmosphere, the compounds may absorb water, resulting in attachment of adsorbed water or formation of hydrates. Recrystallization of the compounds represented by formula (I), formula (II), formula (II-1), or formula (II-2), or pharmaceutically acceptable salts thereof may produce crystal polymorphs. The "co-crystal" means that a compound represented by formula (I), formula (II), formula (II-1), or formula (II-2), or a salt thereof and a counter molecule exist in the same crystal lattice, and it can include any number of counter molecules.

The compounds represented by formula (I), formula (II), formula (II-1), or formula (II-2) or pharmaceutically acceptable salts thereof may form prodrugs. The present invention also encompasses such various prodrugs. A prodrug is a derivative of a compound of the present invention having a group that can be chemically or metabolically degraded, and is a compound which becomes a pharmaceutically active compound of the present invention in vivo as a result of solvolysis or under physiological conditions. Prodrugs include compounds that are converted to the compounds represented by formula (I), formula (II), formula (II-1), or formula (II-2) through enzymatic oxidation, reduction, hydrolysis, or the like under physiological conditions in vivo, compounds that are converted to the compounds represented by formula (I), formula (II), formula (II-1), or formula (II-2) through hydrolysis by gastric acid etc., and the like. Methods for selecting and preparing suitable prodrug derivatives are described in, for example, "Design of Prodrugs, Elsevier, Amsterdam, 1985". A prodrug may have activity per se.

When the compounds represented by formula (I), formula (II), formula (II-1), or formula (II-2) or pharmaceutically acceptable salts thereof have hydroxyl group(s), prodrugs include acyloxy derivatives and sulfonyloxy derivatives that are prepared by, for example, reacting compounds having hydroxyl group(s) with suitable acyl halide, suitable acid anhydride, suitable sulfonyl chloride, suitable sulfonyl anhydride, and mixed anhydride, or with a condensing agent. Examples include CH₃COO-, C₂H₅COO-, tert-BuCOO-, C₁₅H₃₁COO-, PhCOO-, (m-NaOOCPh)COO-, NaOOCCH₂CH₂COO-, CH₃CH(NH₂)COO-, CH₂N(CH₃)₂COO-, CH₃SO₃-, CH₃CH₂SO₃-, CF₃SO₃-, CH₂FSO₃-, CF₃CH₂SO₃-, p-CH₃O-PhSO₃-, PhSO₃-, and p-CH₃PhSO₃-.

### (Method for producing compound of present invention)

The compounds represented by formula (I), formula (II), formula (II-1), or formula (II-2) can be produced by, for example, the general synthesis method described below. Starting materials and reaction reagents used in such synthesis are commercially available or can be synthesized according to methods well known in the art using compounds commercially available. Regarding extraction, purification, and the like, the treatments carried out in ordinary experiments of organic chemistry may be carried out.

The compounds of the present invention can be synthesized with reference to methods known in the art.

In the following steps, when a substituent which interferes with the reaction, e.g. hydroxy, mercapto, amino, formyl, carbonyl, carboxy, is possessed, the substituent is protected by the method such as those described in Protective Groups in Organic Synthesis, Theodora W Greene (John Wiley & Sons) in advance, and the protective group may be removed at a desirable step.

In addition, in the all steps below, an order of steps to be implemented may be appropriately changed, and each intermediate may be isolated, and used in a next step. All of reaction time, reaction temperature, solvents, reagents, protecting groups, etc. are mere exemplification and not limited as long as they do not cause an adverse effect on a reaction.

General procedures for the synthesis of the compounds of the present invention are described below. Starting materials and reaction reagents used in such synthesis are commercially available or can be synthesized according to methods well known in the art using compounds commercially available.

For example, the compounds represented by formula (I), formula (II), formula (II-1), or formula (II-2) of the present invention can be prepared by the general synthetic methods described below.

### General Synthetic Method 1

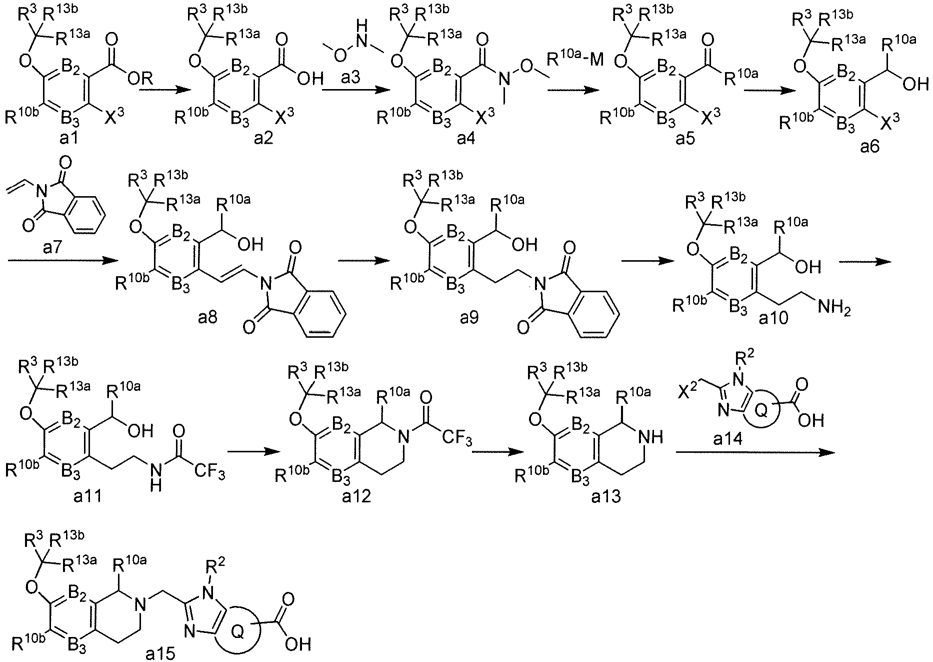

wherein R is alkyl, X² and X³ are leaving groups such as halogen, M is Li, MgCl, MgBr, or the like, and other symbols are the same as those described above, respectively.

Although the case where X is O is described as an example, similar synthesis is possible when X is S.

### Step 1

Compound a1 can be obtained by reacting Compound a2 with a basic aqueous solution.

The reaction temperature is 0°C to 70°C, preferably 0°C to 50°C.

The reaction time is 0.5 hours to 12 hours, and preferably 1 hour to 6 hours.

Examples of the base include sodium carbonate, potassium carbonate, cesium carbonate, carbonate, cesium carbonate, lithium hydroxide, sodium hydroxide, and potassium hydroxide, and the base can be used at 1 to 10 molar equivalents relative to compound a1.

Examples of the reaction solvent include methanol, ethanol, water, acetone, acetonitrile, and THF, and one of or a mixture of these can be used.

### Step 2

Compound a4 can be obtained by reacting Compound a2 with Compound a3 in the presence of a condensing agent.

Examples of the condensing agent include dicyclohexylcarbodiimide, carbonyldiimidazole, dicyclohexylcarbodiimide-N-hydroxybenzotriazole, EDC, and HATU. These can be used at 1 to 5 molar equivalents relative to Compound a2.

Examples of the base include triethylamine, diisopropylethylamine, para-dimethylaminopyridine or the like, and can be used at 1 to 10 molar equivalents relative to Compound a2.

Compound a3 can be used at 1 to 5 molar equivalents relative to Compound a2.

The reaction temperature is -20°C to 80°C, preferably 10°C to 70°C.

The reaction time is 0.1 hours to 24 hours, and preferably 1 hour to 12 hours.

Examples of the reaction solvent include THF, dichloromethane, and DMF, and one of or a mixture of these can be used.

### Step 3

Compound a5 can be obtained by reacting Compound a4 with an organometallic reagent R^{10a}-M.

Examples of the organometallic reagent include Grignard reagent, and organolithium reagent, and the organometallic reagent can be used at 1 to 10 molar equivalents relative to compound a4.

The reaction temperature is -40°C to 60°C, preferably -20°C to 40°C.

The reaction time is 0.5 hours to 24 hours, and preferably 1 hour to 12 hours.

Examples of the reaction solvent include THF, diethyl ether, and dioxane, and one of or a mixture of these can be used.

### Step 4

Compound a6 can be obtained by reacting Compound a5 with formic acid and amine in the presence of a ruthenium catalyst.

Examples of the ruthenium catalyst include [(R,R)-N-(2-amino-1,2-diphenylethyl)-p-toluenesulfonamide]chloro(p-cymene)ruthenium and [(S,S)-N-(2-amino-1,2-diphenylethyl)-p-toluenesulfonamide]chloro(p-cymene)ruthenium, and the ruthenium catalyst can be used at 0.05 to 1 molar equivalents relative to Compound a5.

Examples of the base include triethylamine, and the base can be used at 1 to 5 molar equivalents relative to Compound a5.

The reaction temperature is -10°C to 80°C, preferably 10°C to 60°C.

The reaction time is 0.5 hours to 24 hours, and preferably 1 hour to 12 hours.

As the reaction solvent, acetonitrile or the like can be used.

### Step 5

In the presence of a metal catalyst and a base, tetrabutylammonium bromide or the like is added as necessary, and Compounds a6 and a7 are reacted with each other, whereby Compound a8 can be obtained.

Examples of the metal catalyst include palladium acetate, bis(dibenzylideneacetone)palladium, tetrakis(triphenylphosphine)palladium, bis(triphenylphosphine)palladium (II) dichloride, and bis(tri-tert-butylphosphine)palladium, and the metal catalyst can be used at 0.001 to 0.5 molar equivalents relative to Compound a6.

Examples of the base include dicyclohexylamine, potassium tert-butoxide, sodium carbonate, and potassium carbonate, and the base can be used at 1 to 10 molar equivalents relative to Compound a6.

Compound a7 can be used at 1 to 5 molar equivalents relative to Compound a6.

The reaction temperature may be 20°C to reflux temperature of solvent, and if necessary, by a microwave irradiation.

The reaction time is 0.1 hours to 48 hours, and preferably 0.5 hours to 12 hours.

Examples of the reaction solvent include THF, toluene, DMF, dioxane, and water, and one of or a mixture of these can be used.

### Step 6

Compound a9 can be obtained by reacting compound a8 with hydrogen gas in the presence of a metal catalyst.

Examples of the metal catalyst include palladium-carbon, platinum oxide, rhodium-aluminum oxide, and chlorotris(triphenylphosphine)rhodium(I), and the metal catalyst can be used at 0.01 to 100 weight percent relative to Compound a8.

The hydrogen pressure can be 1 to 50 atm. As the hydrogen source, cyclohexene, 1,4-cyclohexadiene, formic acid, ammonium formate, or the like can also be used.

The reaction temperature is 0°C to the reflux temperature, preferably 20°C to 40°C.

The reaction time is 0.5 to 72 hours, preferably 1 to 12 hours.

Examples of the reaction solvent include methanol, ethanol, propanol, isopropanol, butanol, THF, diethyl ether, toluene, ethyl acetate, acetic acid, and water, and one of or a mixture of these can be used.

### Step 7

Compound a10 can be obtained by reacting hydrazine monohydrate with Compound a9.

Hydrazine monohydrate and the like can be used at 1 to 20 molar equivalent(s) relative to Compound a9.

The reaction temperature is 0°C to 100°C, preferably 20°C to 80°C.

The reaction time is 0.5 hours to 24 hours, and preferably 1 hour to 12 hours.

Examples of the reaction solvent include ethanol.

### Step 8

Compound a11 can be obtained by reacting Compound a10 with trifluoroacetic anhydride.

Trifluoroacetic anhydride can be used at 1 to 10 molar equivalent(s) relative to Compound a10.

The reaction temperature is -10°C to 80°C, preferably 0°C to 40°C.

The reaction time is 0.5 hours to 24 hours, and preferably 1 hour to 12 hours.

Examples of the reaction solvent include dichloromethane, THF, dioxane, and acetonitrile, and one of or a mixture of these can be used.

### Step 9

Compound a12 can be obtained by reacting Compound a11 with triphenylphosphine and a condensing agent.

Examples of the condensing agent include DEAD and DIAD, and the condensing agent can be used at 1 to 5 molar equivalents relative to Compound a11.

Triphenylphosphine can be used at 1 to 5 molar equivalent(s) relative to Compound a11.

The reaction temperature is 0°C to 60°C, preferably 10°C to 40°C.

The reaction time is 0.1 hours to 12 hours, and preferably 0.2 hours to 6 hours.

Examples of the reaction solvent include THF, dioxane, ethyl acetate, toluene, and acetonitrile, and one of or a mixture of these can be used.

### Step 10

Compound a13 can be obtained by reacting Compound a12 with a base.

The reaction temperature is 0°C to 80°C, preferably 10°C to 60°C.

The reaction time is 0.5 hours to 12 hours, and preferably 1 hour to 10 hours.

Examples of the base include sodium carbonate, potassium carbonate, cesium carbonate, carbonate, cesium carbonate, lithium hydroxide, sodium hydroxide, and potassium hydroxide, and the base can be used in an amount of 1 to 10 molar equivalents relative to compound a12.

Examples of the reaction solvent include methanol, ethanol, water, acetone, acetonitrile, and THF, and one of or a mixture of these can be used.

### Step 11

Compound a15 can be obtained by allowing Compound a14 to act on Compound a13 in the presence of a base.

Compound a14 can be used at 0.9 to 3 molar equivalents relative to Compound a13.

The reaction temperature is 0°C to the reflux temperature of the solvent.

The reaction time is 0.5 hours to 12 hours, and preferably 1 hour to 6 hours.

Examples of the base include potassium tert-butoxide, sodium tert-butoxide, sodium carbonate, potassium carbonate, and cesium carbonate, and the base can be used at 1 to 10 molar equivalents with respect to Compound a13.

Examples of the reaction solvent include methanol, ethanol, acetonitrile, THF, and dimethylformamide, and one of or a mixture of these can be used.

### General Synthetic Method 2

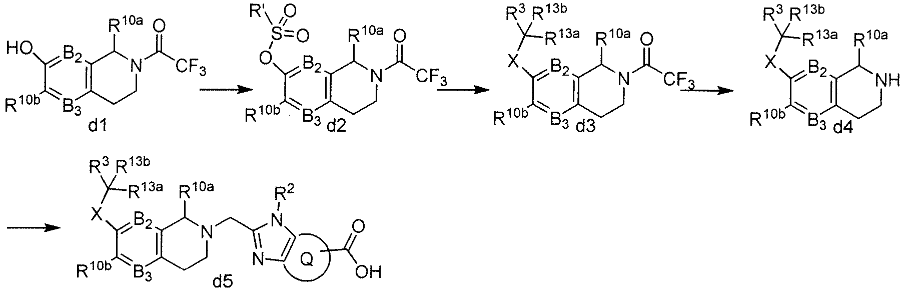

wherein R' is alkyl or haloalkyl, and other symbols are the same as those described above, respectively.

### Step 1

Compound d2 can be obtained by allowing sulfonic anhydride ester or sulfonic acid chloride to act on Compound d1 in the presence of a base.

The sulfonic anhydride ester or the sulfonic acid chloride can be used at 1 to 5 molar equivalents with respect to Compound d1.

Examples of the base include triethylamine, diisopropylethylamine, and pyridine, and 1 to 10 molar equivalents with respect to Compound d1 can be used.

The reaction temperature is -40°C to 60°C, preferably -20°C to 40°C.

The reaction time is 0.1 hours to 48 hours, and preferably 1 hour to 24 hours.

### Step 2

Compound d3 can be obtained by allowing amine or alkyl boronic acid to act on Compound d2 in the presence of a base, a metal catalyst, and ligand.

Examples of the metal catalyst include palladium acetate, bis(dibenzylideneacetone)palladium, tetrakis(triphenylphosphine)palladium, bis(triphenylphosphine)palladium (II) dichloride, [1,1'-bis(diphenylphosphino)ferrocene]dichloropalladium (II), and bis(tri-tert-butylphosphine)palladium, and the metal catalyst can be used at 0.001 to 0.5 molar equivalents relative to Compound d2.

Examples of the ligand include BINAP, DPPF, and Xantphos, and the ligand can be used at 0.001 to 1 molar equivalents relative to Compound d2.

Examples of the base include lithium hydroxide, sodium hydroxide, potassium hydroxide, potassium tert-butoxide, sodium tert-butoxide, sodium carbonate, potassium carbonate, sodium hydrogen carbonate, sodium phosphate, sodium hydrogen phosphate, potassium phosphate, and potassium hydrogen phosphate, and the base can be used at 1 to 10 molar equivalent(s) relative to Compound d2.

The amine or alkyl boronic acid can be used at 1 to 10 molar equivalents relative to Compound d2.

The reaction temperature may be 20°C to reflux temperature of solvent, and if necessary, by a microwave irradiation.

The reaction time is 0.1 hours to 48 hours, and preferably 0.5 hours to 12 hours.

Examples of the reaction solvent include THF, toluene, DMF, dioxane, and water, and one of or a mixture of these can be used.

### Step 3

Compound d4 can be obtained by reacting Compound d3 with a base.

The reaction temperature is 0°C to 80°C, preferably 10°C to 60°C.

The reaction time is 0.5 hours to 12 hours, and preferably 1 hour to 10 hours.

Examples of the base include sodium carbonate, potassium carbonate, cesium carbonate, carbonate, cesium carbonate, lithium hydroxide, sodium hydroxide, and potassium hydroxide, and the base can be used in an amount of 1 to 10 molar equivalents relative to compound d3.

Examples of the reaction solvent include methanol, ethanol, water, acetone, acetonitrile, and THF, and one of or a mixture of these can be used.

### Step 4

Compound d5 can be obtained by allowing Compound d4 to react in the same manner as step 11 of General Synthetic Method 1.

### General Synthetic Method 3

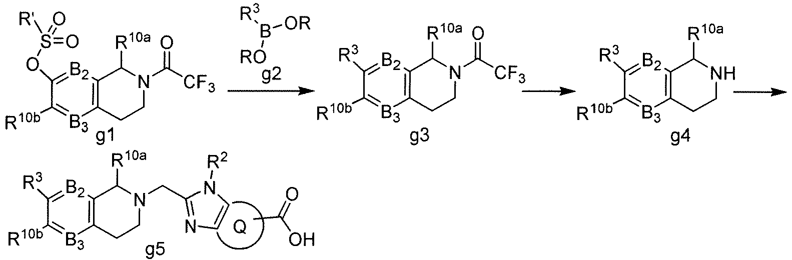

wherein each symbol is as defined above.

### Step 1

Compound g3 can be obtained by allowing Compound g1 and Compound g2 to react in the presence of a metal catalyst and a base.

Examples of the metal catalyst include palladium acetate, bis(dibenzylideneacetone)palladium, tetrakis(triphenylphosphine)palladium, bis(triphenylphosphine)palladium (II) dichloride, and bis(tri-tert-butylphosphine)palladium, and the metal catalyst can be used at 0.001 to 0.5 molar equivalents relative to Compound g1.

Examples of the base include lithium hydroxide, sodium hydroxide, potassium hydroxide, potassium tert-butoxide, sodium tert-butoxide, sodium carbonate, potassium carbonate, sodium hydrogen carbonate, sodium phosphate, sodium hydrogen phosphate, potassium phosphate, and potassium hydrogen phosphate, and the base can be used at 1 to 10 molar equivalent(s) relative to Compound g1.

Compound g2 can be used at 1 to 10 molar equivalents relative to Compound g1.

The reaction temperature may be 20°C to reflux temperature of solvent, and if necessary, by a microwave irradiation.

The reaction time is 0.1 hours to 48 hours, and preferably 0.5 hours to 12 hours.

Examples of the reaction solvent include THF, toluene, DMF, dioxane, and water, and one of or a mixture of these can be used.

### Step 2

Compound g4 can be obtained by reacting Compound g3 with a base.

The reaction temperature is 0°C to 80°C, preferably 10°C to 60°C.

The reaction time is 0.5 hours to 12 hours, and preferably 1 hour to 10 hours.

Examples of the base include sodium carbonate, potassium carbonate, cesium carbonate, carbonate, cesium carbonate, lithium hydroxide, sodium hydroxide, and potassium hydroxide, and the base can be used in an amount of 1 to 10 molar equivalents relative to compound g3.

Examples of the reaction solvent include methanol, ethanol, water, acetone, acetonitrile, and THF, and one of or a mixture of these can be used.

### Step 3

Compound g5 can be obtained by allowing Compound g4 to react in the same manner as step 11 of General Synthetic Method 1.

### General Synthetic Method 4

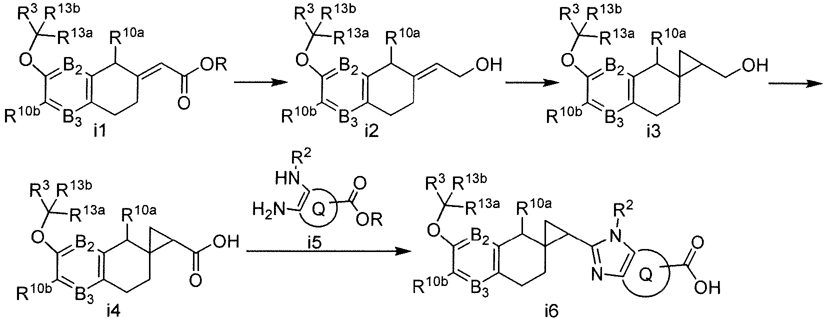

wherein each symbol is as defined above.

### Step 1

Compound i2 can be obtained by reacting Compound i1 with a reducing agent.

Examples of the reducing agent include diisobutylaluminum hydride and lithium borohydride, and the reducing agent can be used in an amount of 1 to 5 molar equivalents relative to Compound i1.

The reaction temperature is -40°C to 50°C, preferably -20°C to 30°C.

The reaction time is 0.1 hours to 24 hours, and preferably 0.5 hours to 12 hours.

Examples of the reaction solvent include dichloromethane, THF, and toluene, and one of or a mixture of these can be used.

### Step 2

Compound i3 can be obtained by allowing diiodomethane to act on compound i2 in the presence of diethyl zinc and a Lewis acid.

Diethylzinc can be used at 1 to 10 molar equivalents relative to Compound i2.

Diiodomethane can be used at 1 to10 molar equivalents relative to Compound i2.

Examples of the Lewis acid include boron trifluoride diethyl ether, and can be used at 1 to 5 molar equivalents relative to Compound i2.

The reaction temperature is -10°C to 50°C, and preferably 0°C to 30°C.

The reaction time is 1 hour to 48 hours, and preferably 3 hours to 24 hours.

Examples of the reaction solvent include dichloromethane, THF, and dioxane, and one of or a mixture of these can be used.

### Step 3

Compound i4 can be obtained by reacting Compound i3 with a radical initiator and an oxidizing agent in the presence of a base.

Examples of the base include sodium hydrogen carbonate, sodium carbonate, sodium hydroxide, potassium carbonate, and calcium carbonate, and the base can be used at 1 to 2 molar equivalents relative to Compound i3.

Examples of the radical initiator include TEMPO, AZADO, and TEMPOL, and and the radical initiator can be used at 0.01 to 1 molar equivalents relative to Compound i3.

Examples of the oxidizing agent include sodium chlorite, sodium hypochlorite, tert-butyloxy chloride, NCS, and meta-chloroperbenzoic acid, and the oxidizing agent can be used at 2 to 5 molar equivalents relative to Compound i3.

The reaction temperature is -20°C to 50°C, and preferably 0°C to 30°C.

The reaction time is 0.1 hours to 24 hours, and preferably 0.5 hours to 6 hours.

Examples of the reaction solvent include acetonitrile, water, dichloromethane, THF, DMF, and DMA, and one of or a mixture of these can be used.

### Step 4

Compound i6 can be obtained by reacting Compound i4 with a condensing agent and Compound i5 in the presence/absence of a base, followed by a reaction in an acid solution.

Compound i5 can be used at 1 to 5 molar equivalents relative to Compound i4.

Examples of the condensing agent include dicyclohexylcarbodiimide, carbonyldiimidazole, dicyclohexylcarbodiimide-N-hydroxybenzotriazole, EDC, 4-(4,6-dimethoxy-1,3,5-triazin-2-yl)-4-methylmorpholinium chloride, and HATU. The condensing agent can be used at 1 to 5 molar equivalents relative to Compound i4.

Examples of the base include triethylamine, diisopropylethylamine, para-dimethylaminopyridine or the like, and can be used at 1 to 10 molar equivalents relative to Compound i4.

Examples of the acid include hydrochloric acid and acetic acid, and the acid can be used at 1 to 20 molar equivalents relative to Compound i4.

The reaction temperature is -20°C to 60°C, preferably 0°C to 30°C for the condensation reaction, and 10°C to 80°C for the subsequent reaction in the acid solution.

The reaction time is 0.1 hours to 24 hours, preferably 1 hour to 12 hours for the condensation reaction, and 0.5 hours to 10 hours for the subsequent reaction in the acid solution.

### General Synthetic Method 5

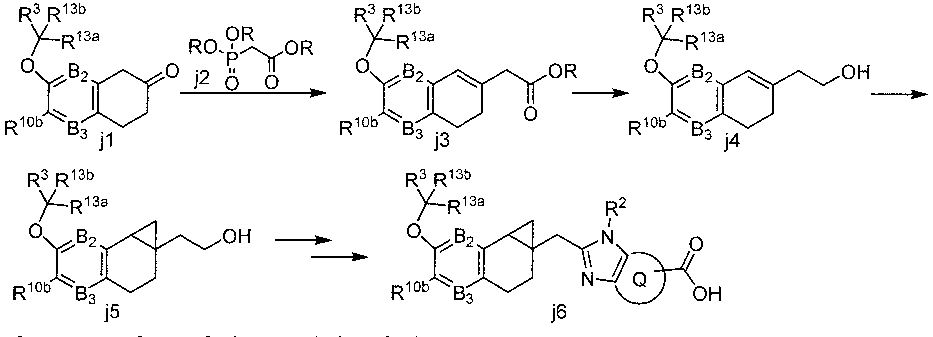

wherein each symbol is as defined above.

### Step 1

Compound j3 can be obtained by reacting Compound j1 with Compound j2 in the presence of a base.

Compound j2 can be used at 1 to 5 molar equivalents relative to Compound j1.

Examples of the base include sodium hydride, sodium methoxide, and LHMDS, and the base can be used at 1 to 10 molar equivalents relative to Compound j1.

The reaction temperature is -40°C to 50°C, preferably -20°C to 30°C.

The reaction time is 0.1 hours to 24 hours, and preferably 0.5 hours to 12 hours.

Examples of the reaction solvent include THF and dimethoxyethane, and one of or a mixture of these can be used.

### Step 2

Compound j4 can be obtained by reacting Compound j3 with a reducing agent.

Examples of the reducing agent include diisobutylaluminum hydride and lithium borohydride, and the reducing agent can be used in an amount of 1 to 5 molar equivalents relative to Compound j3.

The reaction temperature is -40°C to 50°C, preferably -20°C to 30°C.

The reaction time is 0.1 hours to 24 hours, and preferably 0.5 hours to 12 hours.

Examples of the reaction solvent include dichloromethane, THF, and toluene, and one of or a mixture of these can be used.

### Steps 3 and 4

Compound j6 can be obtained by allowing Compound j4 to react in the same manner as steps 2 to 4 of General Synthetic Method 4.

### General Synthetic Method 6

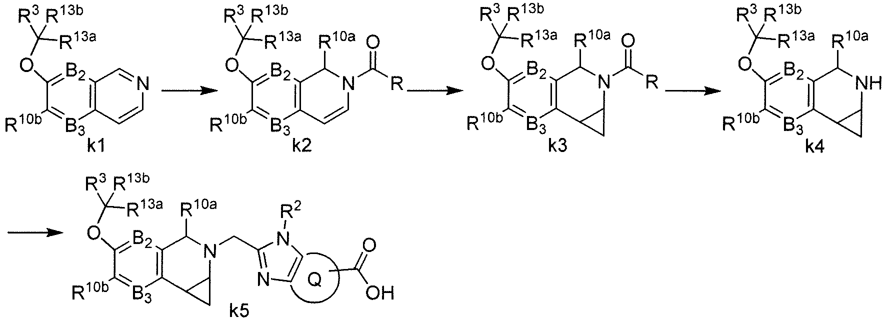

### Step 1

Compound k2 can be obtained by allowing an acid chloride to act on Compound k1 and thereafter reacting Compound k1 with an organometallic reagent R¹⁰-M (when R^{10a} is other than a hydrogen atom) or hydride (when R^{10a} is a hydrogen atom).

Examples of the acid chloride include acetyl chloride and benzoyl chloride, and the acid chloride can be used at 1 to 10 molar equivalents relative to Compound k1.

Examples of the organometallic reagent include Grignard reagent, and organolithium reagent, and the organometallic reagent can be used at 1 to 10 molar equivalents relative to compound k1.

Examples of the hydride include sodium borohydride, and the hydride can be used at 1 to 10 molar equivalents relative to Compound k1.

The reaction temperature is 0°C to 80°C, preferably 10°C to 60°C.

The reaction time is 0.5 hours to 12 hours, and preferably 1 hour to 10 hours.

Examples of the reaction solvent include THF and dichloromethane, and one of or a mixture of these can be used.

### Step 2

Compound k3 can be obtained by allowing Compound k2 to react in the same manner as step 2 of General Synthetic Method 4.

### Step 3

Compound k4 can be obtained by reacting Compound k3 with a base.

The reaction temperature is 0°C to 80°C, preferably 10°C to 60°C.

The reaction time is 0.5 hours to 12 hours, and preferably 1 hour to 10 hours.

Examples of the base include sodium carbonate, potassium carbonate, cesium carbonate, carbonate, cesium carbonate, lithium hydroxide, sodium hydroxide, and potassium hydroxide, and the base can be used at 1 to 10 molar equivalents relative to compound k3.

Examples of the reaction solvent include methanol, ethanol, water, acetone, acetonitrile, and THF, and one of or a mixture of these can be used.

### Step 4

Compound k5 can be obtained by allowing Compound k4 in the same manner as step 11 of General Synthetic Method 1.

### General Synthetic Method 7

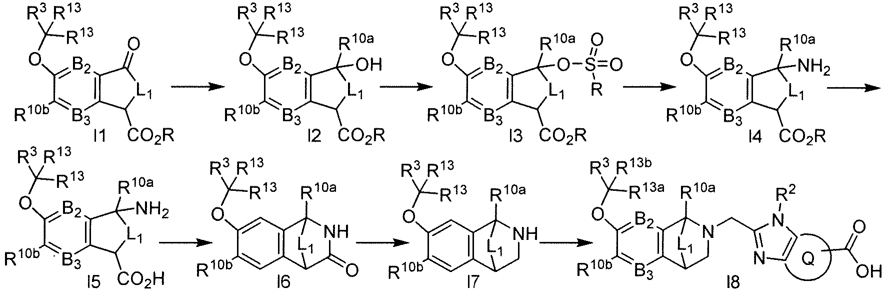

wherein each symbol is as defined above.

### Step 1

Compound l2 can be obtained by reacting an organometallic reagent R^{10a}-M (when R^{10a} is other than a hydrogen atom) or hydride (when R^{10a} is a hydrogen atom) with Compound 11.

Examples of the organometallic reagent include Grignard reagent, and organolithium reagent, and the organometallic reagent can be used at 1 to 5 molar equivalents relative to compound 11.

Examples of the hydride include sodium borohydride, and the hydride can be used at 1 to 10 molar equivalents relative to Compound 11.

The reaction temperature is -40°C to 60°C, preferably -20°C to 50°C.

The reaction time is 0.5 hours to 12 hours, and preferably 1 hour to 6 hours.

Examples of the reaction solvent include THF, dioxane, and dimethoxyethane, and one of or a mixture of these can be used.

### Step 2

Compound 13 can be obtained by allowing sulfonic anhydride ester or sulfonic acid chloride to act on Compound l2 in the presence of a base.

The sulfonic anhydride ester or the sulfonic acid chloride can be used at 1 to 10 molar equivalents with respect to Compound l2.

Examples of the base include triethylamine, diisopropylethylamine, and pyridine, and 1 to 10 molar equivalents with respect to Compound l2 can be used.

The reaction temperature is -40°C to 60°C, preferably -20°C to 40°C.

The reaction time is 0.1 hours to 48 hours, and preferably 1 hour to 24 hours.

Examples of the reaction solvent include dichloromethane, THF, DMF, and acetonitrile, and one of or a mixture of these can be used.

### Step 3

Compound l4 can be obtained by reacting Compound l3 with sodium azide and thereafter reacting the same with triphenylphosphine and water.

The sodium azide can be used at 1 to 10 molar equivalents relative to Compound 13.

The reaction temperature in the first stage is 0°C to 80°C, and preferably 10°C to 60°C.

The reaction time in the first stage is 0.5 hours to 24 hours, and preferably 1 hour to 12 hours.

Examples of the reaction solvent in the first stage include THF, dichloromethane, DMF, and acetonitrile, and one of or a mixture of these can be used.

Triphenylphosphine can be used at 1 to 10 molar equivalents relative to Compound 13.

The reaction temperature in the second stage is 0°C to 80°C,and preferably 10°C to 60°C.

The reaction time in the second stage is 0.5 hours to 48 hours, and preferably 1 hour to 24 hours.

Examples of the reaction solvent in the second stage include THF.

### Step 4

Compound 15 can be obtained by reacting Compound l4 with a base.

The reaction temperature is 0°C to 80°C, preferably 10°C to 60°C.

The reaction time is 0.5 hours to 12 hours, and preferably 1 hour to 10 hours.

Examples of the base include sodium carbonate, potassium carbonate, cesium carbonate, carbonate, cesium carbonate, lithium hydroxide, sodium hydroxide, and potassium hydroxide, and the base can be used at 1 to 10 molar equivalents relative to compound l4.

Examples of the reaction solvent include methanol, ethanol, water, and THF, and one of or a mixture of these can be used.

### Step 5

Compound 16 can be obtained by reacting Compound 15 with a condensing agent in the presence/absence of a base.

Examples of the condensing agent include dicyclohexylcarbodiimide, carbonyldiimidazole, dicyclohexylcarbodiimide-N-hydroxybenzotriazole, EDC, and HATU, and the condensing agent can be used at 1 to 5 molar equivalents relative to Compound 15.

Examples of the base include triethylamine, diisopropylethylamine, and para-dimethylaminopyridine, and the base can be used at 1 to 10 molar equivalents relative to Compound l5.

The reaction temperature is -20°C to 80°C, preferably 10°C to 70°C.

The reaction time is 0.1 hours to 24 hours, and preferably 1 hour to 12 hours.

Examples of the reaction solvent include THF, dichloromethane, and DMF, and one of or a mixture of these can be used.

### Step 6

Compound 17 can be obtained by reacting Compound l6 with a reducing agent.

Examples of the reducing agent include lithium aluminum hydride, and the reducing agent can be used at 1 to 20 molar equivalents relative to Compound l6.

The reaction temperature is 0°C to the reflux temperature of the solvent.

The reaction time is 0.5 hours to 24 hours, and preferably 1 hour to 12 hours.

As the reaction solvent, THF or the like can be also used.

### Step 7

Compound l8 can be obtained by allowing Compound 17 to react in the same manner as step 11 of General Synthetic Method 1.

### General Synthetic Method 8

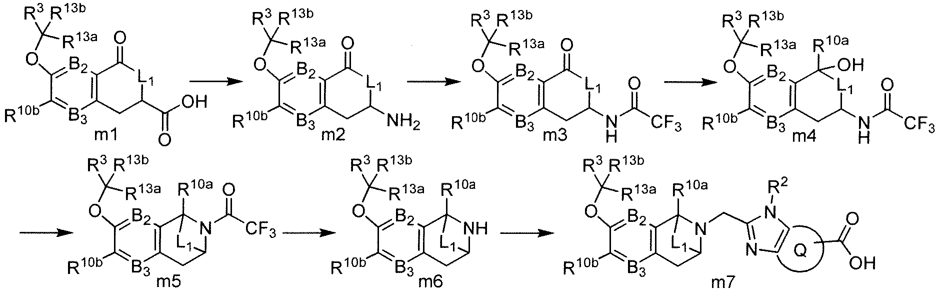

wherein each symbol is as defined above.

### Step 1

Compound m2 can be obtained by reacting Compound m1 with diphenylphosphoryl azide in the presence of a base, and then reacting the same with water.

Diphenylphosphoryl azide can be used at 1 to 10 molar equivalents relative to Compound m1.

Examples of the base include triethylamine, diisopropylethylamine, and pyridine, and the base can be used at 1 to 10 molar equivalents relative to Compound m1.

Regarding the reaction temperature, the temperature for the transfer reaction is -10°C to the reflux temperature of the solvent, and preferably 20°C to the reflux temperature of the solvent, and the temperature for the hydrolysis reaction is -20°C to 60°C, and preferably 0°C to 40°C.

Regarding the reaction time, the time for the transfer reaction is 0.5 to 48 hours, and preferably 1 to 24 hours, and the time for the hydrolysis reaction is 0.1 to 24 hours, and preferably 0.1 to 6 hours.

Examples of the reaction solvent include toluene and THF, and one of or a mixture of these can be used.

### Step 2

Compound m3 can be obtained by allowing Compound m2 to react in the same manner as step 8 of General Synthetic Method 1.

### Step 3

Compound m4 can be obtained by allowing Compound m3 in the same manner as step 1 of General Synthetic Method 7.

### Step 4

Compound m5 can be obtained by allowing Compound m4 to react in the same manner as step 9 of General Synthetic Method 1.

### Step 5

Compound m6 can be obtained by allowing Compound m5 to react in the same manner as step 10 of General Synthetic Method 1.

### Step 6

Compound m7 can be obtained by allowing Compound m6 to react in the same manner as step 11 of General Synthetic Method 1.

As the compounds according to the present invention have GLP-1 receptor agonist activity, they are useful as a therapeutic and/or preventive agent for diseases associated with the GLP-1 receptor.

When the term "therapeutic agent and/or prophylactic agent" is used in the present invention, this also encompasses a symptom ameliorating agent.

Examples of the disease associated with the GLP-1 receptor include non-insulin-dependent diabetes mellitus (type 2 diabetes mellitus), hyperglycemia, impaired glucose tolerance, insulin-dependent diabetes mellitus (type 1 diabetes mellitus), diabetic complications, obesity, hypertension, dyslipidemia, arteriosclerosis, myocardial infarction, coronary heart disease, cerebral infarction, non-alcoholic steatohepatitis, Parkinson's disease, and dementia.

In the present invention, "diabetes" means a disease or condition in which the metabolism in the production and utilization of glucose is abnormal due to the inability to maintain an appropriate blood glucose level in the body, and encompasses insulin-dependent diabetes (type 1 diabetes mellitus) and non-insulin-dependent diabetes mellitus (type 2 diabetes mellitus).

"Hyperglycemia" refers to a state in which the plasma glucose level is higher than a normal value (for example, in humans, 80 to 110 mg/dL on an empty stomach) at fasting and after glucose load, and is also one of representative symptoms of diabetes.

Impaired glucose tolerance includes insulin resistant impaired glucose tolerance and insulin hyposecretion.

Diabetic complication means a complication caused by diabetes or hyperglycemia, and may be either an acute complication or a chronic complication. Examples of the "acute complications" include ketoacidosis and infections (for example, skin infections, soft tissue infections, biliary tract infections, respiratory infections, urinary tract infections), and examples of the "chronic complications" include microangiopathies (for example, nephropathy, retinopathy), neurological disorders (for example, a sensory nerve disorder, a motor nerve disorder, or an autonomic nerve disorder), and leg/foot gangrene. Examples of the diabetic complications include diabetic retinopathy, diabetic nephropathy, and diabetic neuropathy. The "coronary heart disease" encompasses myocardial infarction, angina pectoris, and the like.

Examples of the "dementia" include Alzheimer's disease, vascular dementia, and diabetic dementia. The compound of the present invention has not only GLP-1 receptor agonist activity but also is useful as a medicine, and has any or all of the following excellent characteristics:
a) Inhibitory action against CYP enzymes (e.g., CYP1A2, CYP2C9, CYP2C19, CYP2D6, and CYP3A4) is weak.
b) Satisfactory pharmacokinetics such as high bioavailability and moderate clearance are exhibited.
c) High metabolic stability is exhibited.
d) Irreversible inhibitory action is not exhibited against CYP enzymes (e.g., CYP3A4) within the concentration range of the measurement conditions described in the present specification.
e) Mutagenicity is not exhibited.
f) Low cardiovascular risk is exhibited.
g) Low risk of hematotoxicity is exhibited.
h) High solubility is exhibited.

The pharmaceutical composition of the present invention can be administered by either an oral method or a parenteral method. Examples of a parenteral administration method include percutaneous, subcutaneous, intravenous, intraarterial, intramuscular, intraperitoneal, transmucosal, inhalation, transnasal, ocular instillation, ear instillation, and intravaginal administration.

In the case of oral administration, the pharmaceutical composition may be prepared into any dosage form that is commonly used, such as a solid preparation for internal use (for example, a tablet, a powder preparation, a granular preparation, a capsule, a pill, or a film preparation), or a liquid preparation for internal use (for example, a suspension, an emulsion, an elixir, a syrup, a limonade, a spirit preparation, an aromatic water preparation, an extraction, a decoction, or a tincture) and administered. The tablet may be a dragee, a film-coated tablet, an enteric-coated tablet, a sustained release tablet, a troche, a sublingual tablet, a buccal tablet, a chewable tablet, or an orally disintegrating tablet; the powder preparation and granular preparation may be dry syrups; and the capsule may be a soft capsule, a microcapsule, or a sustained release capsule.

In the case of parenteral administration, the pharmaceutical composition can be suitably administered in any dosage form that is commonly used, such as an injectable preparation, an infusion, or a preparation for external use (for example, an eye drop, a nasal drop, an ear drop, an aerosol, an inhalant, a lotion, an impregnating agent, a liniment, a gargling agent, an enema, an ointment, a plaster, a jelly, a cream, a patch, a poultice, a powder preparation for external use, or a suppository). The injectable preparation may be an emulsion of O/W type, W/O type, O/W/O type, W/O/W type, or the like.

A pharmaceutical composition can be obtained by mixing an effective amount of the compound of the present invention with various pharmaceutical additives appropriate for the dosage form, such as an excipient, a binder, a disintegrating agent, and a lubricating agent, as necessary. Furthermore, the pharmaceutical composition can be prepared into a pharmaceutical composition for use for a child, an elderly person, a patient with a serious case, or a surgical operation, by appropriately changing the effective amount of the compound of the present invention, the dosage form, and/or various pharmaceutical additives. The pharmaceutical composition for children is preferably administered to patients under the age of 12 or 15. Also, the pharmaceutical composition for children may be administered to patients under 27 days after birth, of 28 days to 23 months after birth, or 2 years to 11 years, of 12 years to 17 years, or of 18 years of age. The pharmaceutical composition for the elderly is preferably administered to patients of 65 years of age or older.

It is desirable to set the amount of administration of the pharmaceutical composition of the present invention, after considering the age and body weight of the patient, the type and degree of the disease, the route of administration, and the like; however, in the case of oral administration, the amount of administration is usually 0.05 to 100 mg/kg/day and is preferably in the range of 0.1 to 10 mg/kg/day. Although varying depending on the administration route, the dose in the case of parenteral administration is within a range of usually 0.005 to 10 mg/kg/day, preferably 0.01 to 1 mg/kg/day. This may be administered once a day or several times a day.

The compound of the present invention can be used in combination of a combined drug, to increase the activity of the compound or reduce the dose of the compound, or the like. At this time, the timing of administration for the compound of the present invention and the concomitant drug is not limited, and these may be administered simultaneously to the target of administration or may be administered with a time difference.

The concomitant drug is not limited to a commercially available agent or an agent under development. Examples of the commercially available concomitant drug or the concomitant drug under development include: orlistat, cetilistat, phentermine, mazindol, benzphetamine, amfepramone, methamphetamine, phentermine hydrochloride/topiramate, naltrexone hydrochloride/bupropion hydrochloride, setmelanotide (RM-493), metreleptin, topiramate, naltrexone, bupropion, acarbose, voglibose, miglitol, ipragliflozin, dapagliflozin, luseogliflozin, tofogliflozin, canagliflozin, empagliflozin, ertugliflozin, bexagliflozin, enavogliflozin henagliflozin, janagliflozin, sotagliflozin, insulin aspart, insulin lispro, insulin glulisine, biosynthetic human neutral insulin, human insulin, biosynthetic human isophene insulin, human isophene insulin, intermediate insulin lispro, insulin detemir, insulin glargine, insulin degludec, glibenclamide, gliclazide, glimepiride, glipizide, gliquidone, nateglinide, mitiglinide calcium hydrate, repaglinide, metformin hydrochloride, buformin hydrochloride, pioglitazone hydrochloride, rosiglitazone, lobeglitazone, sitagliptin phosphate, vildagliptin, alogliptin benzoate, linagliptin, teneligliptin hydrobromide, anagliptin, saxagliptin, trelagliptin succinate, omarigliptin, gemigliptin, evogliptin, imegurimin, sitagliptin phosphate/ipragliflozin, pioglitazone hydrochloride/metformin, pioglitazone hydrochloride/glimepiride, teneligliptin hydrobromide/canagliflozin, alogliptin benzoate/pioglitazone hydrochloride, alogliptin benzoate/metformin hydrochloride, vildagliptin/metformin hydrochloride, mitiglinide calcium/voglibose, pravastatin, simvastatin, fluvastatin, atorvastatin, pitavastatin, rosuvastatin, lovastatin, clinofibrate, clofibrate, bezafibrate, fenofibrate, ciprofibrate, pemafibrate, gemfibrozil, colestimide, colestyramine, colesevelam hydrochloride, colestipol, ezetimibe, blobcall, nicomol, tocopherol nicotinic acid ester, niceritrol, ethyl icosapentate, ω-3 fatty acid ethyl, evolocumab, alirocumab, nifedipine, amlodipine, efonidipine, cilnidipine, nicardipine, nisoldipine, nitrendipine, nilvadipine, barnidipine, felodipine, benidipine, manidipine, azelnidipine, alanidipine, diltiazem, trichlormethiazide, benzylhydrochlorothiazide, hydrochlorothiazide, macrone, indapamide, tripamide, mefluside, furosemide, triamterene, spironolactone, eplerenone, tolvaptan, torasemide, hydrochlorothiazide, bumetanide, chlortalidone, isosorbide, metolazone,losartan, candesartan, valsartan, telmisartan, olmesartan, irbesartan, azilsartan, captopril, enalapril, alacepril, delapril, cilazapril, lisinopril, benazepril, imidapril, temocapril, quinapril, trandolapril, perindopril erbumine, urapidil, terazosin, prazosin, doxazosin, bunazosin, atenolol, bisoprolol, metoprolol, acebutolol, celiprolol, propranolol, nadolol, nipradilol, carteolol, pindolol, nebivolol, carvedilol, labetalol, sotalol, landiolol, arotinolol, amosulalol, arotinolol, carvedilol, labetalol, bevantolol, clonidine, guanabenz, methyldopa, reserpine, hydralazine, nitroprusside, aliskiren, kallidinogenase, alprostadil alfadex, dihydroergotoxine, doxazosin, urapidil, hydralazine, prazosin, moxonidine, guanfacine, rilmenidine, amlodipine/atrovastatin, losartan/hydrochlorothiazide, valsartan/hydrochlorothiazide, candesartan/hydrochlorothiazide, telmisartan/hydrochlorothiazide, irbesartan/trichlorothiazide, valsartan/amlodipine, olmesartan/azelnidipine, candesartan/amlodipine, telmisartan/amlodipine, irbesartan/amlodipine, valsartan/cilnidipine, azilsartan/amlodipine, carbetocin (LV-101), PYY-1562 (NNC0165-1562), PYY-1875 (NNC0165-1875), NGM395, YH34160, SCO-267, SCO-792, diazoxide, tesofensine, namodenoson, ERX1000, ASC41, Xla1, HDV Biotin, EMP16, metoprolol/tesofensine, RZL-012, CB4211, BI1356225, AMG171, NO-13065, bardoxolone methyl, HSG4112, YHC2129, YHC2134, KTX-0200, obeticholic acid, cilofexor (GS-9674), tropifexor (LJN452), EDP-305, EYP-001, resmetirom, VK-2809, cenicriviroc, saroglitazar, lanifibranor, selonsertib, PF-06835919, pegbelfermin, efrugxifermin, aldafermin, aramcol, MK-3655, MSDC-0602K, belapectin, firsocostat (GS-0976), PF-05221304, ervogastat, ION-224, AXA-1125, HU-6, MET-409, MET-642, TERN-101, TERN-501, LPCN-1144, denifanstat, fruxifermin, leronlimab, pegozafermin, rencofilstat, retatrutide, tipelukast, S-237648, and S-723595, and BMS-963272, as well as compounds shown below and acceptable salts thereof.

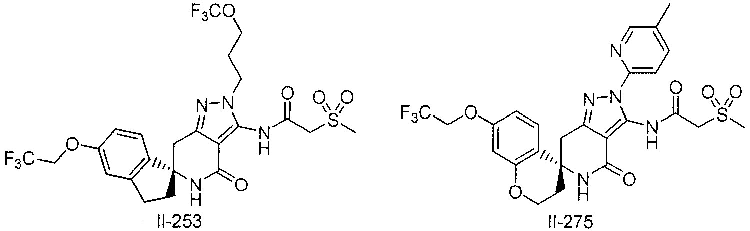

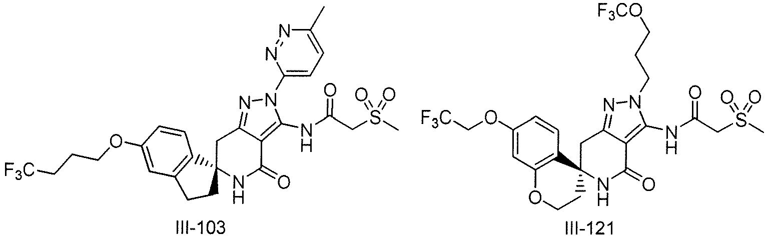

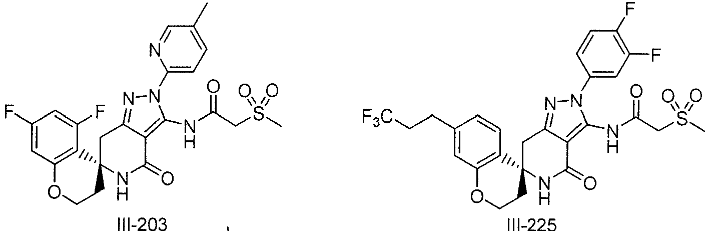

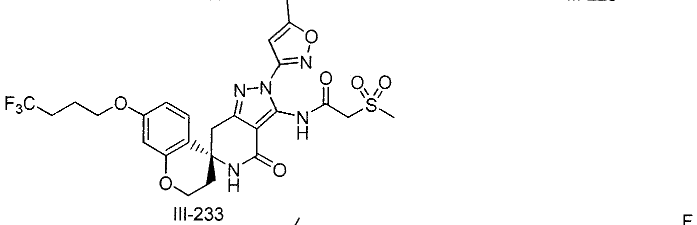

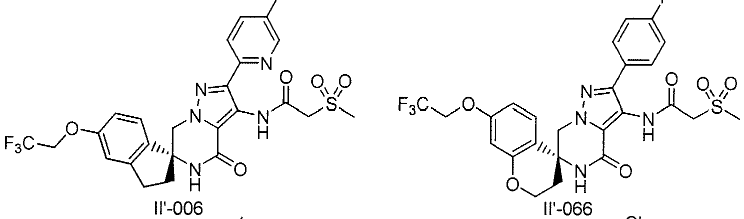

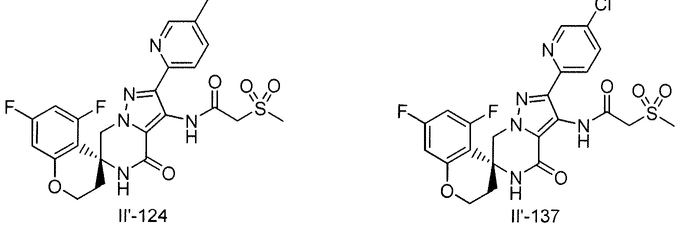

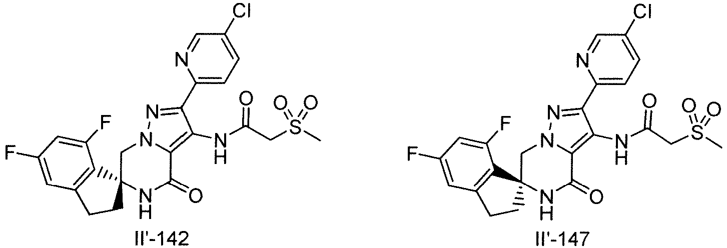

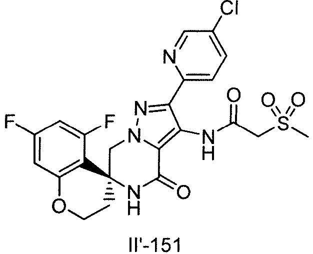

Examples of the same preferably include the following compounds or pharmaceutically acceptable salts thereof.

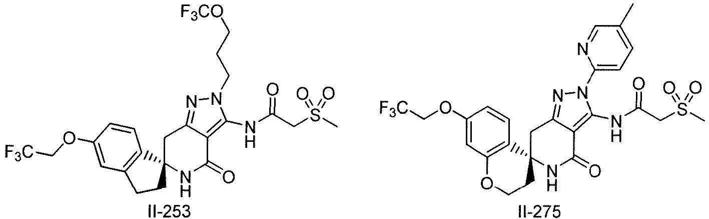

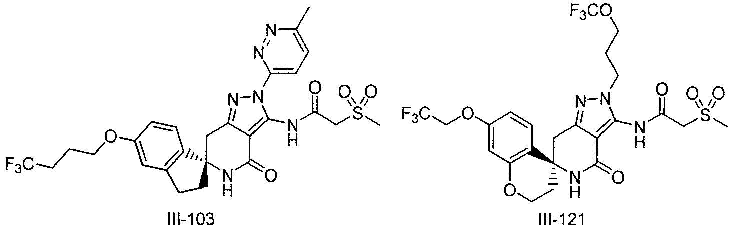

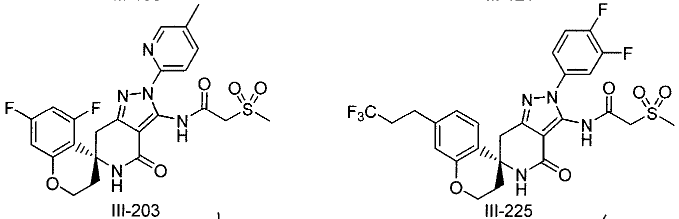

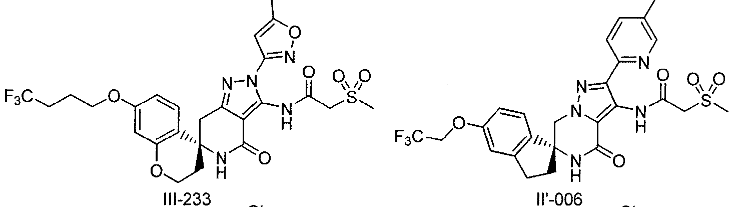

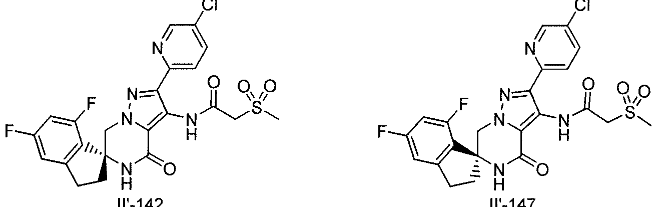

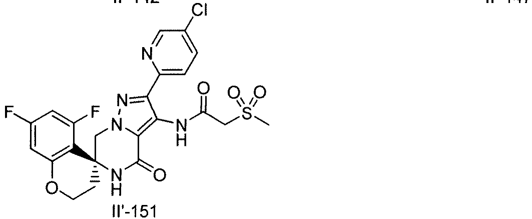

The amount of administration of the concomitant drug can be appropriately selected based on the clinically used dosage. Furthermore, the blending ratio of the compound of the present invention and the concomitant drug can be appropriately selected according to the target of administration, the route of administration, the target disease, symptoms, combination, and the like. For example, when the target of administration is a human, 0.01 to 100 parts by weight of the concomitant drug may be used with respect to 1 part by weight of the compound of the present invention.

The pharmaceutical composition of the present invention can be used in combination with other anti-obesity agent(s) (the pharmaceutical composition comprising compounds having anti-obesity effect, or the medicinal agent for obesity or for the weight management for obesity). For example, a combination treatment with a pharmaceutical composition comprising a compound having an anti-obesity effect and the compound of the present invention can be used for the prevention and/or treatment of obesity or the weight management for obesity. A combination treatment with the pharmaceutical composition comprising the compound of the present invention and a pharmaceutical composition(s) comprising a compound having an anti-obesity effect can be used for the prevention and/or treatment of obesity or the weight management for obesity. Furthermore, a method of treatment by administering the pharmaceutical composition of the invention can be used in combination of the diet therapy, drug therapy, exercise and the like.

### [EXAMPLES]

Hereinafter, the present invention will be described in more detail by way of Examples, Reference Examples, and Test Examples; however, the present invention is not intended to be limited by these.

Furthermore, abbreviations used in the present specification denote the following meanings.
CHCl₃: chloroform
CDCl₃: deuterated chloroform
MeOH: methanol
DMSO-d₆: deuterated dimethyl sulfoxide
DMSO: dimethyl sulfoxide
DMA: dimethylacetamide
DMF: dimethylformamide
THF: tetrahydrofuran
EDC: 1-ethyl-3-(3-dimethylaminopropyl)carbodiimide
HATU: 2-(7-azabenzotriazol-1-yl)-1,1,3,3-tetramethyluronium hexafluorophosphate
DIAD: diisopropyl azodicarboxylate
DEAD: diethyl azodicarboxylate
TBDPS: tert-butyldiphenylsilyl
TEMPO: 2,2,6,6-tetramethylpiperidine 1-oxyl
TEMPOL: 4-hydroxy-2,2,6,6-tetramethylpiperidine-1-oxyl
AZADO: 2-azaadamantane N-oxyl
NCS: N-chlorosuccinimide
BINAP: 2,2'-bis(diphenylphosphino)-1,1'-binaphthyl
DPPF: 1,1-ferrocenediyl-bis(diphenylphosphino)
Xantphos: 4,5-bis(diphenylphosphino)-9,9-dimethylxanthene
TLC: thin layer chromatography
SFC: supercritical fluid chromatography
ODS: octadecylsilyl
M: mol/L

### (Method for identifying compound)

The NMR analysis obtained in each Example was performed at 400 MHz, and measurement was made using DMSO-d₆, or CDCl₃. Furthermore, when NMR data are shown, there are occasions in which all the measured peaks are not described.

In Examples, "No." represents compound number, "Structure" means a chemical structure, and "MS" represents a mass in LC/MS (liquid chromatography/mass spectrometry). MS (m/z) can be measured under the following measurement conditions, though the measurement conditions are not limited to these.

### (Measurement conditions 1)

Column: ACQUITY UPLC BEH C18 (1.7 µm i.d. 2.1 × 50 mm) (Waters)
Flow rate: 0.8 mL/min
UV detection wavelength: 254 nm
Mobile phase: [A] was 0.1% formic acid-containing aqueous solution, and [B] was 0.1% formic acid-containing acetonitrile solution
Gradient: A linear gradient of 5% to 100% solvent [B] was carried out for 3.5 minutes, and then 100% solvent [B] was maintained for 0.5 minutes.

### (Measurement conditions 2)

Column: Shim-pack XR-ODS (2.2 µm, i.d. 3.0 × 50 mm) (Shimadzu)
Flow rate: 1.6 mL/min; UV detection wavelength: 254 nm;
Mobile phase: [A] was 0.1% formic acid-containing aqueous solution, and [B] was 0.1% formic acid-containing acetonitrile solution
Gradient: linear gradient of 10% to 100% solvent [B] was performed in 3 minutes, and 100% solvent [B] was kept for 0.5 minutes.

### (Measurement conditions 3)

Column: ACQUITY UPLC BEH C18 (1.7 µm i.d. 2.1 × 50 mm) (Waters)
Flow rate: 0.8 mL/min; UV detection wavelength: 254 nm;
Mobile phase: [A] was 0.1% formic acid-containing aqueous solution, and [B] was 0.1% formic acid-containing acetonitrile solution
Gradient: linear gradient of 5% to 100% solvent [B] for 3.5 minutes was performed, and then 100% solvent [B] was maintained for 0.5 minute.

### (Measurement conditions 4)

Column: ACQUITY UPLC BEH C18 (1.7 µm i.d. 2.1 × 50 mm) (Waters)
Flow rate: 0.8 mL/min
UV detection wavelength: 254 nm
Mobile phase: [A] is 10mM ammonium carbonate in aqueous solution, and [B] is acetonitrile
Gradient: A linear gradient of 5% to 100% solvent [B] was carried out for 3.5 minutes, and then 100% solvent [B] was maintained for 0.5 minutes.

### (Measurement conditions 5)

Column: L-column 2 ODS (3 µm i.d. 3 × 50 mm) (manufactured by Chemicals Evaluation and Research Institute)
Flow rate: 1.5 mL/min
UV detection wavelength: 220 nm
Mobile phase: [A] was 0.05% trifluoroacetic acid-containing aqueous solution, and [B] was 0.05% trifluoroacetic acid-containing acetonitrile solution
Gradient: linear gradient of 5% to 95% solvent [B] was performed in 3.5 minutes, and 95% solvent [B] was kept for 2 minutes.

### Example 1

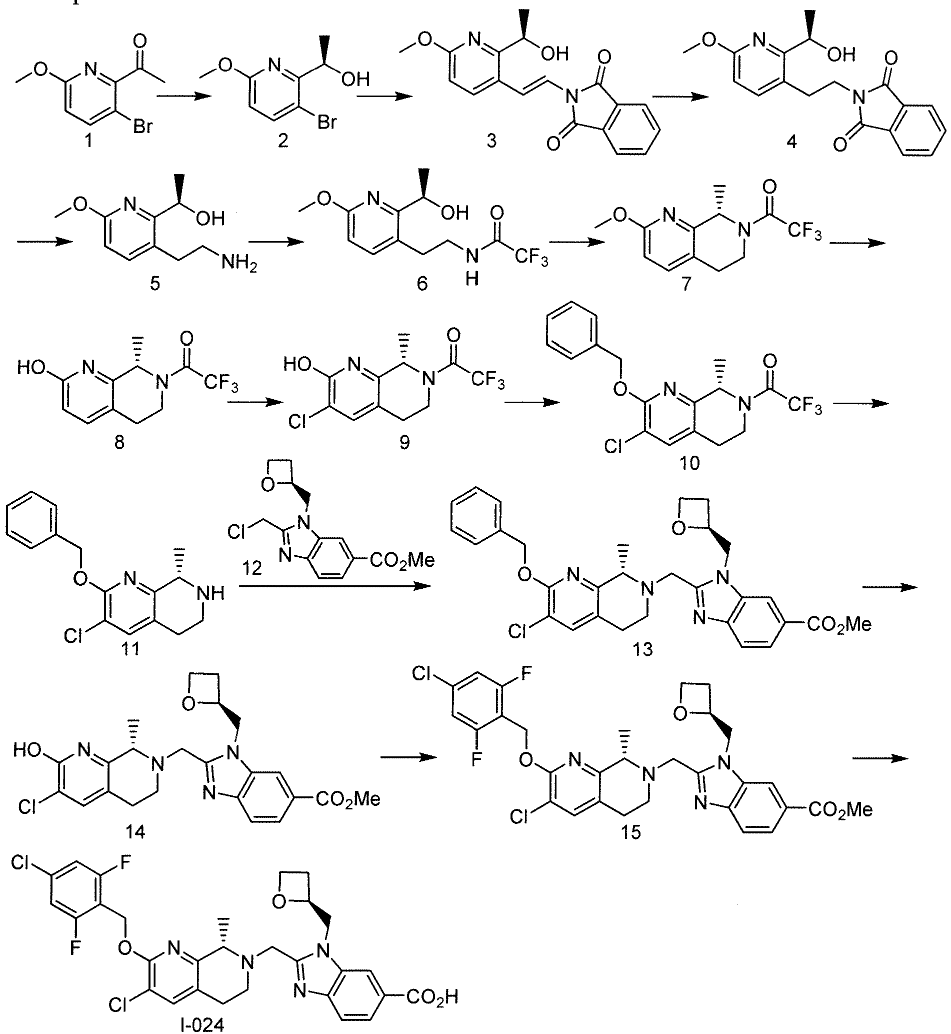

### Step 1

Formic acid (2.45 mL, 63.8 mmol), triethylamine (5.15mL, 37.1 mmol) and [(R,R)-N-(2-amino-1,2-diphenylethyl)-p-toluenesulfonamide]chloro(p-cymene)ruthenium (II) (0.19g, 0.30 mmol) were added to an acetonitrile (35 mL) solution of Compound 1 (3.42 g,14.85 mmol), and the mixture was stirred at room temperature for 6 hours. Water was added to the reaction solution, and the solution was extracted with ethyl acetate. After the organic layer was washed with an aqueous sodium hydrogen carbonate solution and dried over anhydrous sodium sulfate, the solvent was evaporated under reduced pressure, and the obtained residue was purified by silica gel column chromatography (hexane-ethyl acetate) to give Compound 2 (3.44 g, yield 100%).
¹H-NMR (CDCl₃) 6: 1.45 (3H, d, J = 6.4 Hz), 3.96 (3H, s), 4.03 (1H, d, J = 8.3 Hz), 5.01-5.08 (1H, m), 6.59 (1H, d, J = 8.7 Hz), 7.67 (1H, d, J = 8.7 Hz).
MS (m/z) = 232 [M+H]+

### Step 2

N-vinylphthalimide (2.62 g, 14.82 mmol), tetrabutylammonium bromide (4.78 g, 14.82 mmol), palladium acetate (0.33 g, 1.48 mmol), and N,N-dicyclohexyl-N-methylamine (4.72 mL, 22.23 mmol) were added to a solution of Compound 2 (3.44 g, 14.82 mmol) in DMF (27.5 mL). After the reaction container was degassed under reduced pressure and purged with nitrogen, the reaction solution was stirred at 110 °C for 1 hour. Water was added to the reaction solution, and the precipitated solids were filtered. The obtained solid was washed with diisopropyl ether to give Compound 3 (3.87 g, yield 81%).
¹H-NMR (CDCl₃) 6: 1.45 (3H, d, J = 6.4 Hz), 3.99 (3H, s), 4.54 (1H, d, J = 7.7 Hz), 5.09-5.16 (1H, m), 6.71 (1H, d, J = 8.5 Hz), 7.17 (1H, d, J = 14.9 Hz), 7.72 (1H, d, J = 14.9 Hz), 7.77-7.81 (3H, m), 7.90-7.93 (2H, m).
MS (m/z) = 325 [M+H]+

### Step 3

After 10% palladium-carbon (containing 50% of water) (2.54 g, 1.2 mmol) was added to a methanol (25 mL)-THF (50 mL) mixture solution of Compound 3 (3.87 g, 11.9 mmol), the mixture was stirred at room temperature for 6 hours and 30 minutes under a hydrogen atmosphere (1 atm). Insolubles were removed by filtration, and then the solvent was evaporated under reduced pressure to give Compound 4 (1.55 g, yield 40%).
¹H-NMR (CDCl₃) δ: 1.47 (3H, d, J = 6.4 Hz), 2.82-2.99 (2H, m), 3.84-3.89 (2H, m), 3.94 (3H, s), 4.27 (1H, d, J = 8.0 Hz), 5.04-5.11 (1H, m), 6.62 (1H, d, J = 8.4 Hz), 7.50 (1H, d, J = 8.4 Hz), 7.71-7.75 (2H, m), 7.83-7.87 (2H, m).
MS (m/z) = 327 [M+H]+

### Step 4

Hydrazine monohydrate (1.15 mL, 23.75 mmol) was added to an ethanol (30 mL) solution of Compound 4 (1.55 g, 4.75 mmol), and then the mixture was stirred at 80°C for 35 minutes. Insolubles were removed by filtration, and the solvent was evaporated under reduced pressure. Dichloromethane was added to the obtained residue, precipitated insolubles were removed by filtration, and the solvent was evaporated under reduced pressure to give a crude product (1.05 g) of Compound 5. ¹H-NMR (CDCl₃) δ: 1.45 (3H, d, J = 6.4 Hz), 2.62-2.78 (2H, m), 2.86-3.02 (2H, m), 3.95 (3H, s), 5.01 (1H, q, J = 6.4 Hz), 6.63 (1H, d, J = 8.4 Hz), 7.41 (1H, d, J = 8.4 Hz). MS (m/z) = 197 [M+H]+

### Step 5

Trifluoroacetic anhydride (2.0 mL, 14.25 mmol) was added dropwise to a dichloromethane (10 mL) solution of the crude product (4.75 mmol) of Compound 5 under ice cooling, and the mixture was stirred at room temperature for 45 minutes. After the solvent was evaporated under reduced pressure, the obtained residue was dissolved in toluene, and the solvent was evaporated under reduced pressure again. The obtained residue was dissolved in ethyl acetate, slowly poured into an aqueous sodium hydrogen carbonate solution, and stirred at 35°C for 9 hours. The reaction solution was extracted with ethyl acetate, the organic layer was dried over anhydrous magnesium sulfate, and then, the solvent was evaporated under reduced pressure. The obtained residue was purified by silica gel column chromatography (hexane-ethyl acetate) to give Compound 6 (952 mg, yield 69%).
¹H-NMR (CDCl₃) δ: 1.48 (3H, d, J = 6.4 Hz), 2.80-2.92 (2H, m), 3.50-3.64 (2H, m), 3.82 (1H, d, J = 7.0 Hz), 3.95 (3H, s), 4.95-5.02 (1H, m), 6.67 (1H, d, J = 8.4 Hz), 6.77 (1H, s), 7.41 (1H, d, J = 8.4 Hz).
MS (m/z) = 293 [M+H]+

### Step 6

Triphenylphosphine (1.28 g, 4.89 mmol) was added to a THF (30 mL) solution of Compound 6 (952 mg, 3.26 mmol), DIAD (0.95 mL, 4.89 mmol) was added dropwise thereto under water cooling, and the mixture was stirred at room temperature for 30 minutes. The solvent of the reaction solution was evaporated under reduced pressure, and the obtained residue was purified by silica gel column chromatography (hexane-ethyl acetate) to give Compound 7 (776 mg, yield 87%).
¹H-NMR (CDCl₃) δ: 1.59 (2.1H, d, J = 6.9 Hz), 1.65 (0.9H, d, J = 6.9 Hz), 2.64-2.73 (1H, m), 2.87-2.98 (1H, m), 3.14-3.21 (0.3H, m), 3.44-3.51 (0.7H, m), 3.90 (2.1H, s), 3.90 (0.9H, s), 4.09-4.15 (0.7H, m), 4.69 (0.3H, dd, J = 13.2, 5.6 Hz), 5.01 (0.3H, q, J = 6.8 Hz), 5.43 (0.7H, q, J = 6.8 Hz), 6.57-6.61 (1H, m), 7.30-7.34 (1H, m).
MS (m/z) = 275 [M+H]+

### Step 7

Sodium iodide (1.27 g, 8.49 mmol) and trimethylsilyl chloride (1.085 mL, 8.49 mmol) were added to an acetonitrile (10 mL) solution of Compound 7 (776 mg, 2.83 mmol), and the mixture was stirred at 45°C for 6 hours. An aqueous sodium hydrogen carbonate solution and an aqueous sodium thiosulfate solution were added to the reaction solution. The solution was extracted with ethyl acetate. The organic layer was dried over anhydrous sodium sulfate, and the solvent was evaporated under reduced pressure to give a crude product (747 mg) of Compound 8.
¹H-NMR (CDCl₃) δ: 1.65 (2.4H, d, J = 6.8 Hz), 1.73 (0.6H, d, J = 6.8 Hz), 2.50-2.58 (1H, m), 2.73-2.84 (1H, m), 3.14-3.22 (0.2H, m), 3.44-3.52 (0.8H, m), 4.08-4.13 (0.8H, m), 4.68 (0.2H, dd, J = 13.4, 5.8 Hz), 4.96 (0.2H, q, J = 6.6 Hz), 5.43 (0.8H, q, J = 6.8 Hz), 6.48-6.52 (1H, m), 7.26-7.22 (1H, m), 12.82 (1H, s).
MS (m/z) = 261 [M+H]+

### Step 8

N-chlorosuccinimide (497 mg, 3.72 mmol) was added to a DMF (8 mL) solution of Compound 8 (800 mg, 3.07 mmol), and at 45°C, then the mixture was stirred at the same temperature for 10 hours. After the reaction solution was cooled to room temperature, an aqueous sodium thiosulfate solution and an aqueous sodium hydrogen carbonate solution were added thereto, and a solid obtained was collected by filtration to give Compound 9 (527 mg, yield 58%).
MS (m/z) = 295.1 [M+H]+
¹H-NMR (DMSO-D₆) δ: 1.46 (2.4H, d, J = 6.8 Hz), 1.55 (0.6H, d, J = 6.5 Hz), 2.58-2.67 (2H, m), 3.54-3.62 (1H, m), 3.93 (0.8H, d, J = 12.9 Hz), 4.39 (0.2H, dd, J = 13.8, 4.5 Hz), 4.85 (0.2H, q, J = 6.4 Hz), 5.17 (0.8H, dd, J = 6.7, 13.5 Hz), 7.62-7.65 (1H, m).

### Step 9

1,4-dioxane (20 mL) was added to Compound 9 (1.97 g, 6.68 mmol), silver carbonate (2.76, 10.01 mmol), and benzyl bromide (1.26 g, 7.34 mmol), and the mixture was stirred at 65°C for 9 hours. The reaction solution was cooled to room temperature, and insolubles were removed by filtration and washed with methanol. The solvent was evaporated under reduced pressure to give a crude product (2.31 g) of Compound 10.
MS (m/z) = 385.2 [M+H]+

### Step 10

A 1 M aqueous sodium hydroxide solution (9.0 mL, 9.0 mmol) was added to a THF (13 mL)-methanol (13 mL) mixture solution of Compound 10 (2.3 g, 5.98 mmol), and the mixture was stirred at room temperature for 1 hour. Water was added to the reaction solution, and the solution was extracted with ethyl acetate. The solvent was evaporated under reduced pressure to give a crude product (1.77 g) of Compound 11.
MS (m/z) = 289.2 [M+H]+
¹H-NMR (CDCl₃) δ: 1.46 (3H, d, J = 6.7 Hz), 2.63 (1H, dt, J = 16.0, 4.1 Hz), 2.75-2.83 (1H, m), 2.95-3.01 (1H, m), 3.20-3.27 (1H, m), 3.97 (1H, dd, J = 6.7, 12.7 Hz), 5.44 (2H, dd, J = 18.4, 12.7 Hz), 7.28-7.31 (1H, m), 7.34-7.37 (3H, m), 7.48 (2H, d, J = 7.3 Hz).

### Step 11

Acetonitrile (2.4 mL) was added to Compound 11 (245 mg, 0.85 mmol), Compound 12 (251 mg, 0.85 mmol), and potassium carbonate (235 mg, 1.70 mmol), and the mixture was stirred at 65°C for 3.5 hours. The reaction solution was cooled to room temperature, water was added thereto, and the solution was extracted with ethyl acetate. The solvent was evaporated under reduced pressure, and the obtained residue was purified by silica gel column chromatography (hexane-ethyl acetate) to give Compound 13 (440 mg, yield 95%).
MS (m/z) = 547.4 [M+H]+
¹H-NMR (CDCl₃) δ: 1.44 (3H, d, J = 6.7 Hz), 2.36-2.42 (1H, m), 2.56-2.78 (4H, m), 2.95-3.02 (1H, m), 3.80 (1H, dd, J = 6.6, 13.5 Hz), 3.95 (3H, s), 4.06-4.15 (1H, m), 4.30-4.39 (2H, m), 4.55-4.63 (1H, m), 4.68 (1H, dd, J = 15.4, 5.8 Hz), 4.75 (1H, dd, J = 15.3, 3.0 Hz), 5.20 (1H, ddd, J = 13.2, 7.2, 2.9 Hz), 5.38-5.47 (2H, m), 7.28-7.31 (1H, m), 7.33-7.38 (3H, m), 7.47 (2H, d, J = 7.3 Hz), 7.77 (1H, d, J = 8.5 Hz), 7.98 (1H, dd, J = 8.5, 1.4 Hz), 8.14 (1H, d, J = 0.9 Hz).

### Step 12

Methanol (4.5 mL) was added to Compound 13 (439 mg, 0.80 mmol) and 10% palladium carbon (128 mg, 0.12 mmol), and the mixture was stirred at room temperature under a hydrogen atmosphere for 2.5 hours. Insolubles were removed by filtration, the solvent of the filtrate was evaporated under reduced pressure, and the obtained residue was purified by silica gel column chromatography (chloroform-methanol) to give Compound 14 (230 mg, yield 63%).
MS (m/z) = 457.3 [M+H]+
¹H-NMR (CDCl₃) δ: 1.50 (3H, d, J = 6.8 Hz), 2.32-2.46 (2H, m), 2.70-2.81 (3H, m), 3.02-3.10 (1H, m), 3.83 (1H, dd, J = 6.6, 13.6 Hz), 3.95 (3H, s), 4.18 (1H, d, J = 13.6 Hz), 4.27 (1H, d, J = 13.7 Hz), 4.35-4.41 (1H, m), 4.55-4.64 (2H, m), 4.77 (1H, dd, J = 15.4, 6.6 Hz), 5.15-5.21 (1H, m), 7.39 (1H, s), 7.69 (1H, d, J = 8.4 Hz), 7.95 (1H, dd, J = 8.5, 1.4 Hz), 8.11 (1H, d, J = 1.0 Hz).

### Step 13

1,4-dioxane (0.5 mL) was added to Compound 14 (20 mg, 0.044 mmol), 2,6-difluoro-4-chlorobenzyl bromide (13 mg, 0.053 mmol), and silver carbonate (18 mg, 0.066 mmol), and the mixture was stirred at 65°C for 8 hours. The reaction solution was cooled to room temperature, insolubles were removed by filtration, water was added to the filtrate, and the solution was extracted with chloroform. The solvent of the organic layer was evaporated under reduced pressure, and the obtained residue was purified by reverse phase chromatography to give Compound 15 (15 mg, yield 55%).
MS (m/z) = 617.2 [M+H]+

### Step 14

A 1 M aqueous sodium hydroxide solution (0.5 mL, 0.50 mmol) was added to a methanol (0.5 mL)-THF (0.5 mL) solution of Compound 15 (15 mg, 0.024 mmol), and the mixture was stirred at room temperature for 2 hours. An aqueous citric acid solution was added to the reaction solution to adjust pH to about 7, and the solution was extracted with chloroform. The solvent of the organic layer was evaporated under reduced pressure, and the obtained residue was purified by reverse phase chromatography to give Compound I-024 (12 mg, yield 79%).
MS (m/z) = 603.1 [M+H]+
¹H-NMR (DMSO-D₆) δ: 1.37 (3H, d, J = 6.7 Hz), 2.30-2.39 (1H, m), 2.57-2.79 (4H, m), 2.93-2.99 (1H, m), 3.79 (1H, q, J = 6.7 Hz), 4.06 (1H, d, J = 13.8 Hz), 4.18 (1H, d, J = 13.9 Hz), 4.27-4.32 (1H, m), 4.46 (1H, dd, J = 13.4, 7.9 Hz), 4.65-4.79 (2H, m), 5.09 (1H, ddd, J = 13.8, 6.7, 2.8 Hz), 5.35 (1H, d, J = 12.3 Hz), 5.44 (1H, d, J = 12.2 Hz), 7.38-7.43 (2H, m), 7.65-7.68 (2H, m), 7.81 (1H, dd, J = 8.5, 1.4 Hz), 8.25 (1H, s).

### Example 2

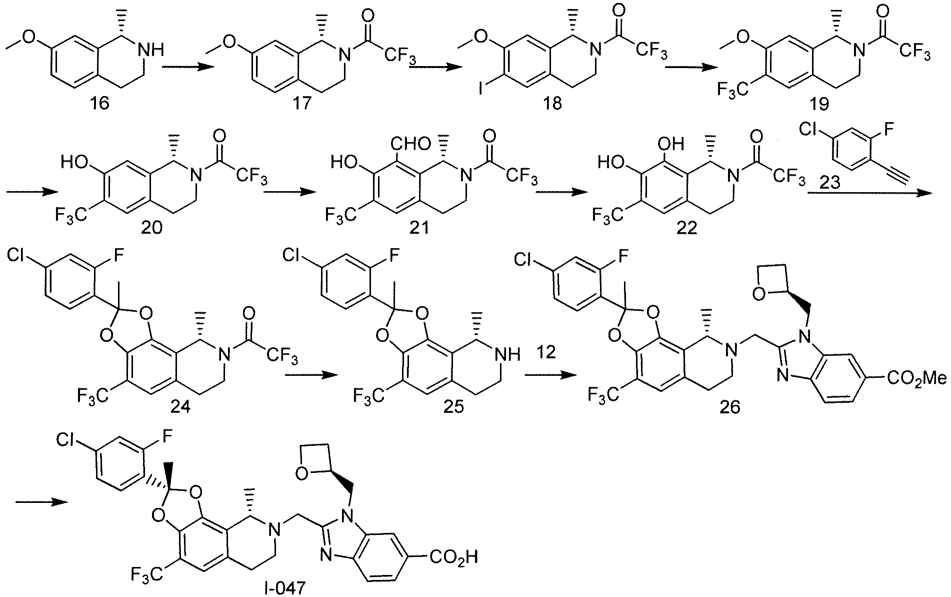

### Step 1

Trifluoroacetic anhydride (2.5 mL, 16.7 mmol) was added to a dichloromethane (19 mL) solution of Compound 16 (WO 2019200120A) (1.90 g, 10.7 mmol), and the mixture was stirred at room temperature for 4.5 hours. Water was added to the reaction solution, and the solution was extracted with dichloromethane. The solvent was evaporated under reduced pressure, and the obtained residue was purified by silica gel column chromatography (hexane-ethyl acetate) to give Compound 17 (2.25 g, yield 74%).
MS (m/z) = 274.2 [M+H]+
¹H-NMR (CDCl₃) δ: 1.53 (2.4H, d, J = 6.8 Hz), 1.61 (0.6H, d, J = 6.7 Hz), 2.74-2.82 (1H, m), 2.91-3.00 (1H, m), 3.26 (0.2H, td, J = 12.5, 4.4 Hz), 3.56 (0.8H, td, J = 12.5, 4.3 Hz), 3.79 (2.4H, s), 3.80 (0.6H, s), 4.04 (0.8H, d, J = 12.8 Hz), 4.59 (0.2H, dd, J = 12.8, 5.6 Hz), 5.10 (0.2H, q, J = 6.8 Hz), 5.53 (0.8H, q, J = 6.8 Hz), 6.62 (0.2H, d, J = 2.5 Hz), 6.66 (0.8H, d, J = 2.5 Hz), 6.75-6.80 (1H, m), 7.03-7.07 (1H, m).

### Step 2

Silver trifluoroacetate (1.74 g, 7.87 mmol) and iodine (1.99 g, 7.84 mmol) were added to a dichloromethane (19 mL) solution of Compound 17 (1.95 g, 7.13 mmol), and the mixture was stirred at room temperature for 1 hour. Insolubles in the reaction solution were removed by filtration, an aqueous sodium thiosulfate solution was added to the filtrate, and the solution was extracted with dichloromethane. The organic layer was washed with water and dried over anhydrous sodium sulfate, and then the solvent was evaporated under reduced pressure. The obtained residue was purified by silica gel column chromatography (hexane-ethyl acetate) to give Compound 18 (2.00 g, yield 70%).
MS (m/z) = 400.2 [M+H]+
¹H-NMR (CDCl₃) 6: 1.53 (2.4H, d, J = 6.8 Hz), 1.61 (0.6H, d, J = 6.8 Hz), 2.71-2.79 (1H, m), 2.89-2.97 (1H, m), 3.23 (0.2H, td, J = 12.7, 4.5 Hz), 3.50-3.57 (0.8H, m), 3.86 (2.4H, s), 3.88 (0.6H, s), 4.01-4.05 (0.8H, m), 4.59 (0.2H, dd, J = 13.3, 5.3 Hz), 5.08 (0.2H, q, J = 6.5 Hz), 5.52 (0.8H, q, J = 6.8 Hz), 6.51 (0.2H, s), 6.55 (0.8H, s), 7.55 (0.8H, s), 7.57 (0.2H, s).

### Step 3

Methyl difluoro(fluorosulfonyl)acetate (3.15 mL, 24.93 mmol) and copper iodide (1.14 g, 5.98 mmol) were added to a DMF (20 mL) solution of Compound 18 (1.99 g, 4.99 mmol), and the mixture was stirred at 100°C for 3 hours under a nitrogen atmosphere. Water was added to the reaction solution, and the solution was extracted with ethyl acetate. The organic layer was washed with water and dried over anhydrous sodium sulfate, and then the solvent was evaporated under reduced pressure. The obtained residue was purified by silica gel column chromatography (hexane-ethyl acetate) to give Compound 19 (1.56 g, yield 94%).
MS (m/z) = 342.2 [M+H]+
¹H-NMR (CDCl₃) 6: 1.56 (2.4H, d, J = 6.9 Hz), 1.64 (0.6H, d, J = 6.8 Hz), 2.77-2.86 (1H, m), 2.92-3.02 (1H, m), 3.26 (0.2H, td, J = 12.3, 5.2 Hz), 3.52-3.60 (0.8H, m), 3.89 (2.4H, s), 3.90 (0.6H, s), 4.02-4.13 (0.8H, m), 4.65 (0.2H, dd, J = 13.0, 5.6 Hz), 5.14 (0.2H, q, J = 6.8 Hz), 5.59 (0.8H, q, J = 6.9 Hz), 6.69 (0.2H, s), 6.74 (0.8H, s), 7.33 (0.8H, s), 7.35 (0.2H, s).

### Step 4

A 1 M boron tribromide dichloromethane solution (13.2 mL, 13.2 mmol) was added a dichloromethane (7.8 mL) solution of Compound 19 (1.55 g, 4.41 mmol) under ice cooling, and the mixture was stirred at room temperature for 2.5 hours. Methanol (7.8 mL) was added to the reaction solution under ice cooling, water was added thereto, and the solution was extracted with dichloromethane. The solvent of the organic layer was evaporated under reduced pressure, and the obtained residue was purified by silica gel column chromatography (hexane-ethyl acetate) to give Compound 20 (1.20 g, yield 78%).
MS (m/z) = 326.2 [M-H]-
¹H-NMR (CDCl₃) δ: 1.53 (2.4H, d, J = 6.9 Hz), 1.61 (0.6H, d, J = 6.8 Hz), 2.77-2.86 (1H, m), 2.91-3.01 (1H, m), 3.24 (0.2H, td, J = 12.6, 4.1 Hz), 3.50-3.58 (0.8H, m), 4.07-4.12 (0.8H, m), 4.64 (0.2H, dd, J = 13.4, 5.8 Hz), 5.10 (0.2H, q, J = 6.8 Hz), 5.50-5.57 (1.6H, m), 6.73 (0.2H, s), 6.76 (0.8H, s), 7.29 (0.8H, s), 7.30 (0.2H, s).

### Step 5

Compound 20 (490.6 mg, 1.50 mmol) was dissolved in trifluoroacetic acid (10 mL), and hexamethylenetetramine (315 mg, 2.25 mmol) was added, then the mixture was stirred at 85°C for 7 hours. Water was added to the reaction solution, the mixture was stirred at room temperature for 10 minutes, and the solution was extracted with ethyl acetate. The organic layer was washed with sodium bicarbonate water, and dried over anhydrous sodium sulfate, and then the solvent was evaporated under reduced pressure. The obtained residue was purified by silica gel column chromatography (hexane-ethyl acetate) to give Compound 21 (411.8 mg, yield 77%).
MS (m/z) = 356.0 [M+H]+
¹H-NMR (CDCl₃) δ: 12.70 (0.8H, s), 12.64 (0.2H, s), 10.39 (0.8H, s), 10.32 (0.2H, s), 7.62 (0.2H, s), 7.60 (0.8H, s), 6.31 (1H, q, J = 6.8 Hz), 4.13-4.06 (1H, m), 3.79-3.71 (1H, m), 3.11-2.86 (2H, m), 1.73 (0.6H, d, J = 6.8 Hz), 1.64 (2.4H, d, J = 6.9 Hz).

### Step 6

A THF (10 mL) solution of 30% aqueous hydrogen peroxide solution (1.62 mL, 15.8 mmol), boric acid (2.23 g, 36.0 mmol), and 1M sulfuric acid (0.192 mL, 3.60 mmol) was stirred at room temperature for 30 minutes. A THF (15 mL) solution of Compound 21 (2.56 g, 7.20 mmol) was added to the reaction solution, and the mixture was stirred at 60°C for 8 hours and 30 minutes. A 10% aqueous sodium bisulfite solution was added to the reaction solution under ice cooling, and the solution was extracted with ethyl acetate. The organic layer was washed with sodium bicarbonate water, and dried over anhydrous sodium sulfate, and then the solvent was evaporated under reduced pressure. The obtained residue was purified by silica gel column chromatography (hexane-ethyl acetate) to give Compound 22 (1.53 g, yield 62%).
MS (m/z) = 344.2 [M+H]+
¹H-NMR (CDCl₃) δ: 6.85 (1H, s), 6.22 (1H, br s), 5.99 (0H, br s), 5.84 (0.7H, q, J = 6.4 Hz), 5.67 (1H, br s), 5.37 (0.3H, q, J = 6.6 Hz), 4.64-4.60 (0.3H, m), 4.08-4.03 (0.7H, m), 3.66-3.58 (0.7H, m), 3.30-3.27 (0.3H, m), 2.98-2.93 (1H, m), 2.87-2.78 (1H, m), 1.61 (1H, d, J = 6.8 Hz), 1.54 (2H, d, J = 6.8 Hz).

### Step 7

Compound 22 (162.1 mg, 0.472 mmol) and a hexane solution of Compound 23 (57 wt%, 128 mg, 0.472 mmol) were dissolved in toluene (2.5 mL), and triruthenium dodecacarbonyl (6.04 mg, 9.45 µmol) was added, then the mixture was stirred at 100°C for 18 hours. The reaction solution was diluted with ethyl acetate, and the solvent was evaporated under reduced pressure. The obtained residue was purified by silica gel column chromatography (hexane-ethyl acetate) to give Compound 24 (55.4 mg, yield 24%).
MS (m/z) = 498.2 [M+H]+
¹H-NMR (CDCl₃) δ: 7.50-7.47 (1H, m), 7.18-7.12 (2H, m), 6.84 (0.3H, s), 6.82 (0.7H, s), 5.68 (0.35H, q, J = 7.2 Hz), 5.60 (0.35H, q, J = 6.5 Hz), 5.21-5.19 (0.15H, m), 4.64-4.61 (0.15H, m), 4.10-4.07 (1H, m), 3.54-3.45 (1H, m), 2.89-2.85 (2H, m), 2.15 (1.5H, s), 2.11 (1.5H, s), 1.64 (0.45H, d, J = 6.9 Hz), 1.56 (1.05H, d, J = 6.9 Hz), 1.52 (0.45H, d, J = 6.8 Hz), 1.45 (1.05H, d, J = 6.8 Hz).

### Step 8

Potassium carbonate (30.8 mg, 0.223 mmol) was added to a methanol (1 mL)-THF (1 mL) mixture solution of Compound 24 (55.4 mg, 0.111 mmol), and the mixture was stirred at 50°C for 7 hours. The reaction solution was cooled to room temperature, water was added thereto, and the solution was extracted with ethyl acetate. The organic layer was dried over anhydrous sodium sulfate, and the solvent was evaporated under reduced pressure to give a crude product (46.8 mg g) of Compound 25.
MS (m/z) = 402.2 [M+H]+
¹H-NMR (CDCl₃) 6: 7.51-7.48 (1H, m), 7.16-7.10 (2H, m), 6.77 (1H, s), 4.22-4.15 (1H, m), 3.19-3.15 (1H, m), 2.98-2.93 (1H, m), 2.74-2.62 (2H, m), 2.11 (1.5H, s), 2.10 (1.5H, s), 1.51 (1.5H, d, J = 6.7 Hz), 1.42 (1.5H, d, J = 6.8 Hz).

### Step 9

A mixture (58.9 mg) of Compound 26 and impurities was obtained by performing the same reaction as in Step 11 of Example 1.
MS (m/z) = 660.4 [M+H]+

### Step 10

A 1 M aqueous sodium hydroxide solution (0.382 mL, 0.382 mmol) was added to a methanol (1.5 mL)-THF (1.5 mL) mixture solution of a mixture (58.9 mg) of Compound 26 and impurities, and it was stirred at 45°C for 12 hours. The reaction solution was cooled to room temperature, 2M hydrochloric acid was added to adjust the pH to 4, and the solution was extracted with ethyl acetate. The organic layer was dried over anhydrous sodium sulfate, and then the solvent was evaporated under reduced pressure. The obtained residue was purified by reverse-phase column chromatography (acetonitrile-water) to give a diastereomeric mixture.

The obtained diastereomeric mixture was separated by SFC to give Compound 1-047 (12.2 mg, 16% yield).

### SFC Conditions

Column: P4VP column (manufactured by Daicel Corporation) 4.6×250 mm
Analysis time: 15 min
Mobile phase: A is carbon dioxide and B is 0.1% diethylamine MeOH solution
Fractionation conditions: the solution was sent with a compositional ratio of B/(A + B) = 20% being kept
Flow rate: 2.0 mL/min
Detection wavelength: 220 nm

MS (m/z) = 646.4 [M+H]+
¹H-NMR (CDCl₃) δ: 8.20 (1H, s), 8.04 (1H, d, J = 9.0 Hz), 7.81 (1H, d, J = 8.5 Hz), 7.50 (1H, t, J = 8.4 Hz), 7.15-7.12 (2H, m), 6.80 (1H, s), 5.24-5.17 (1H, m), 4.84-4.58 (3H, m), 4.46-4.19 (3H, m), 4.15-4.03 (1H, m), 3.08-2.98 (1H, m), 2.96-2.84 (1H, m), 2.83-2.55 (3H, m), 2.47-2.38 (1H, m), 2.11 (3H, s), 1.35 (3H, d, J = 6.5 Hz).

### Example 3

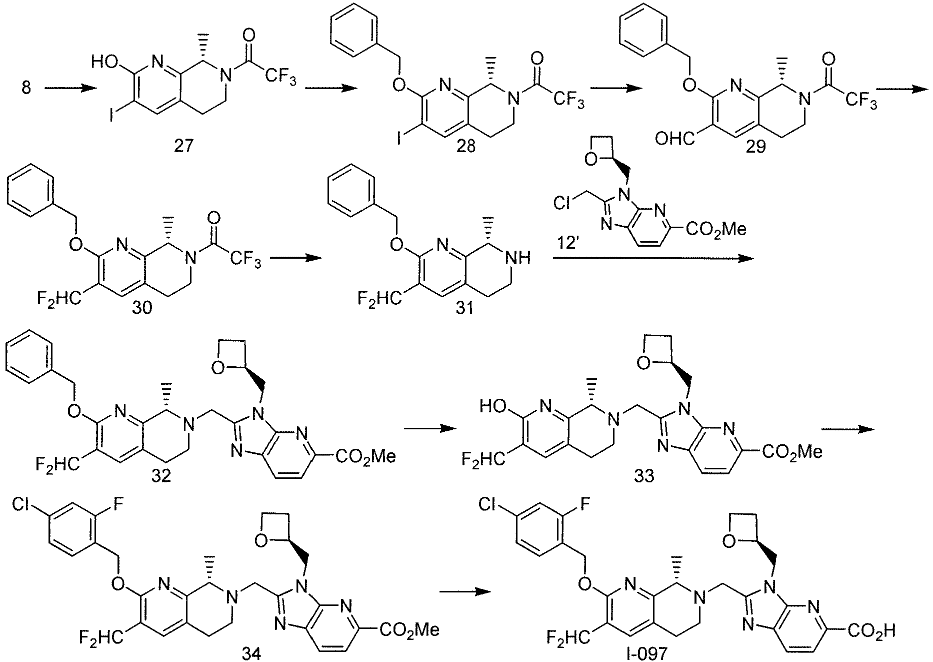

### Step 1

N-iodosuccinimide (2.12 g, 9.42 mmol) was added to a DMF (12 mL) solution of Compound 8 (1.17 g, 3.14 mmol), and the mixture was stirred at room temperature for 7 hours. An aqueous sodium thiosulfate solution was added to the reaction solution, and the solution was extracted with ethyl acetate. The solvent of the organic layer was evaporated under reduced pressure to give Compound 27 (1.98 g). MS (m/z) = 386.9 [M+H]+

### Step 2

Compound 28 (7.59 mg, yield 94%) was obtained by performing the same reaction as in Step 9 of Example 1.
MS (m/z) = 476.9 [M+H]+
¹H-NMR (CDCl₃) δ: 1.58-1.52 (3H, m), 2.71-2.63 (1H, m), 2.93-2.87 (1H, m), 3.46-3.09 (1H, m), 4.70-4.05 (1H, m), 5.45-4.94 (3H, m), 7.31-7.30 (1H, m), 7.39-7.36 (2H, m), 7.49-7.47 (2H, m), 7.83-7.81 (1H, m).

### Step 3

Palladium acetate (206 mg, 0.92 mmol), 2-(di-tert-butylphosphino)biphenyl (366 mg, 1.23 mmol), sodium carbonate (3.25 g, 30.6 mmol), triethylsilane (5.34 g, 46.0 mmol), and 2-isocyano-2-methylpropane (2.55 g, 30.6 mmol) were added to a DMF (60 mL) solution of Compound 28 (5.95 g, 12.3 mmol), and the mixture was stirred at 65°C for 3.5 hours. The reaction solution was cooled to room temperature, hydrochloric acid was added to adjust the pH to about 4, and thereafter, the solution was extracted with ethyl acetate. The solvent was evaporated under reduced pressure, and the obtained residue was purified by silica gel column chromatography (hexane-ethyl acetate) to give Compound 29 (2.37 g, yield 50%).
MS (m/z) = 379.0 [M+H]+

¹H-NMR (CDCl₃) δ: 1.65-1.57 (3H, m), 2.83-2.74 (1H, m), 3.02-2.91 (1H, m), 3.51-3.14 (1H, m), 4.75-4.12 (1H, m), 5.55-5.04 (3H, m), 7.39-7.34 (3H, m), 7.48-7.46 (2H, m), 7.92-7.90 (1H, m), 10.39 (1H, s).

### Step 4

N,N-diethylaminosulfur trifluoride (3.95 g, 26.3 mmol) was added to a dichloromethane (28 mL) solution of Compound 29 (2.84 g, 7.28 mmol) in an ice bath, and the mixture was stirred at room temperature for 2.5 hours. An aqueous sodium hydrogen carbonate solution was added to the reaction solution, and the solution was extracted with chloroform. The solvent was evaporated under reduced pressure, and the obtained residue was purified by silica gel column chromatography (hexane-ethyl acetate) to give Compound 30 (2.55 g, yield 85%).
MS (m/z) = 401.0 [M+H]+
¹H-NMR (CDCl₃) δ: 1.62-1.54 (3H, m), 2.79-2.71 (1H, m), 3.01-2.91 (1H, m), 3.51-3.13 (1H, m), 4.74-4.12 (1H, m), 5.47-5.01 (3H, m), 6.86 (1H, t, J = 55.2 Hz), 7.39-7.29 (3H, m), 7.44-7.42 (2H, m), 7.65-7.62 (1H, m).

### Step 5

A 1 M aqueous sodium hydroxide solution (12.5 mL, 12.5 mmol) was added to a methanol (12 mL)-THF (12 mL) mixture solution of Compound 30 (2.0 g, 5.0 mmol), and the mixture was stirred at room temperature for 1 hour. An aqueous citric acid solution was added to the reaction solution to adjust pH to about 7, and the solution was extracted with chloroform. The solvent of the organic layer was evaporated under reduced pressure, and the obtained residue was purified by silica gel column chromatography (hexane-ethyl acetate) to give Compound 31 (1.49 g, yield 98%).
MS (m/z) = 305.1 [M+H]+
¹H-NMR (CDCl₃) δ: 1.49 (3H, d, J = 6.8 Hz), 2.68 (1H, dt, J = 16.0, 4.1 Hz), 2.87-2.79 (1H, m), 3.03-2.97 (1H, m), 3.26 (1H, ddd, J = 12.5, 5.3, 4.3 Hz), 4.01 (1H, q, J = 6.6 Hz), 5.40 (1H, d, J = 12.5 Hz), 5.45 (1H, d, J = 12.8 Hz), 6.87 (1H, t, J = 55.6 Hz), 7.32-7.28 (1H, m), 7.36 (2H, t, J = 7.4 Hz), 7.43 (2H, d, J = 7.3 Hz), 7.56 (1H, s).

### Step 6

Compound 12' (277 mg, 0.936 mmol) and potassium carbonate (272 mg, 1.97 mmol) were added to an acetonitrile (3 mL) solution of Compound 31 (300 mg, 0.936 mmol), and the mixture was stirred at 60°C for 5 hours. An aqueous citric acid solution was added to the reaction solution to adjust pH to about 7, and the solution was extracted with chloroform. The solvent was evaporated under reduced pressure to give Compound 32 (410 mg).
MS (m/z) = 564.2 [M+H]+

### Step 7

THF (5 mL) and Methanol (5 mL) were added to Compound 32 (410 mg, 0.883 mmol) and 10% palladium carbon (155 mg, 0.088 mmol), and the mixture was stirred at room temperature under a hydrogen atmosphere for 3 hours. Insolubles were removed by filtration, and the solvent of the filtrate was evaporated under reduced pressure. The obtained residue was purified by silica gel column chromatography (hexane-ethyl acetate) to give Compound 33 (320 mg, yield 93%).
MS (m/z) = 474.0 [M+H]+
¹H-NMR (CDCl₃) 6: 1.51 (3H, d, J = 6.8 Hz), 2.38-2.53 (2H, m), 2.69-2.84 (3H, m), 3.05-3.12 (1H, m), 3.85-3.90 (1H, m), 4.01 (3H, s), 4.25-4.39 (3H, m), 4.60 (1H, q, J = 7.2 Hz), 4.81 (1H, dd, J = 14.8, 3.0 Hz), 4.92 (1H, dd, J = 14.8, 6.5 Hz), 5.18-5.24 (1H, m), 6.72 (1H, t, J = 55.3 Hz), 7.53 (1H, d, J = 9.3 Hz), 8.06 (1H, d, J = 8.3 Hz), 8.11 (1H, d, J = 8.3 Hz).

### Step 8

Silver carbonate (17 mg, 0.063 mmol) and 1-(bromomethyl)-4-chloro-2-fluorobenzene (11 mg, 0.051 mmol) were added to a dioxane (0.2 mL) solution of Compound 33 (20 mg, 0.042 mmol), and the mixture was stirred at 65°C for 3 hours. The mixture was cooled to room temperature, and insolubles were removed by filtration and washed with ethyl acetate. The solvent of the organic layer was evaporated under reduced pressure, and the obtained residue was purified by reverse phase chromatography to give Compound 34 (11 mg, yield 43%).
MS (m/z) = 616.2 [M+H]+

### Step 9

A 1 M aqueous sodium hydroxide solution (0.06 mL, 0.06 mmol) was added to a methanol (0.25 mL)-THF (0.25 mL) solution of Compound 34 (11 mg, 0.018 mmol), and the mixture was stirred at room temperature for 3 hours. An aqueous citric acid solution was added to the reaction solution to adjust pH to about 4, and the solution was extracted with chloroform. The solvent of the organic layer was evaporated under reduced pressure, and the obtained residue was purified by reverse phase chromatography to give Compound 1-097 (10.1 mg, yield 93%).
MS (m/z) = 602.1 [M+H]+
¹H-NMR (CDCl₃) δ: 1.47 (3H, d, J = 6.8 Hz), 2.39-2.44 (1H, m), 2.64-2.85 (4H, m), 3.03-3.08 (1H, m), 3.82-3.87 (1H, m), 4.18-4.23 (1H, m), 4.30-4.34 (1H, m), 4.42-4.46 (1H, m), 4.59-4.64 (1H, m), 4.79-4.91 (2H, m), 5.20-5.25 (1H, m), 5.40 (1H, d, J = 13.1 Hz), 5.47 (1H, d, J = 13.1 Hz), 6.84 (1H, t, J = 55.6 Hz), 7.09-7.13 (2H, m), 7.39 (1H, t, J = 8.3 Hz), 7.59 (1H, s), 8.19-8.24 (2H, m).

### Example 4

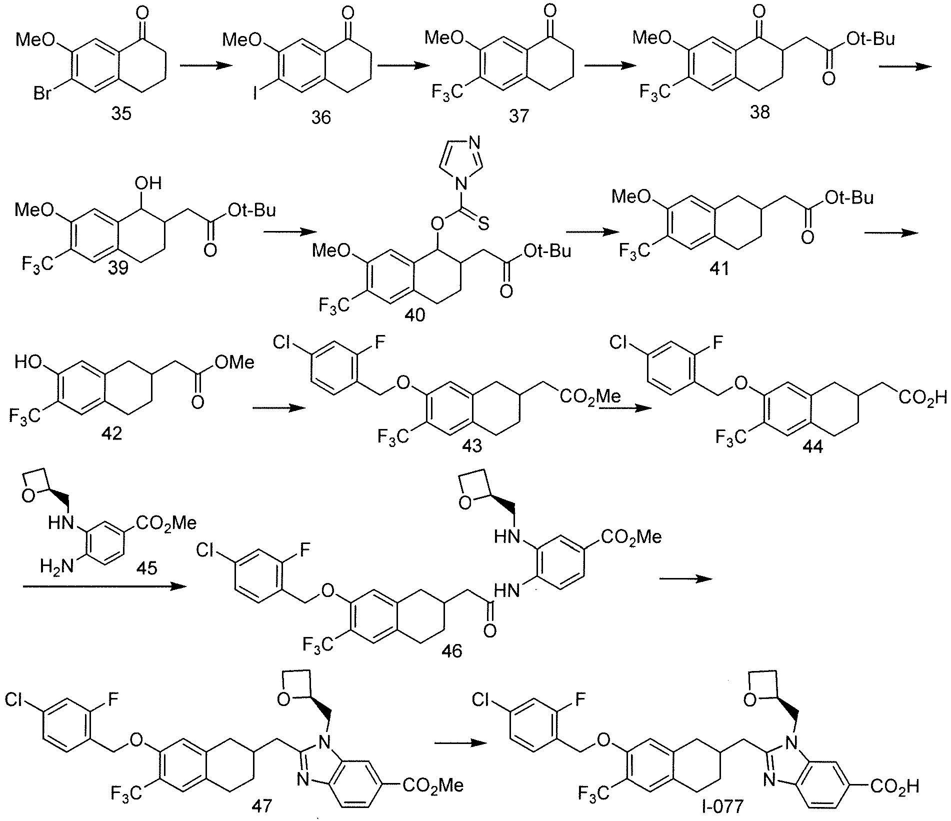

### Step 1

Copper iodide (0.659 g, 3.46 mmol) and sodium iodide (5.20 g, 34.7 mmol) were added to a dioxane (66 mL) solution of Compound 35 (4.42 g, 17.3 mmol), (±)-trans-N,N'-dimethylaminocyclohexane (0.999 g, 7.02 mmol), under a nitrogen atmosphere, and the mixture was heated and stirred at 100°C for 9 hours. The reaction solution was cooled to room temperature, then diluted with ethyl acetate, and filtered through Celite (registered trademark). The filtrate was washed with a 3% aqueous ammonia solution and water, and the solvent was evaporated under reduced pressure. The obtained residue was purified by silica gel column chromatography (hexane-ethyl acetate) to give Compound 36 (4.79 g, yield 86.4%).
¹H-NMR (CDCl₃) 6: 2.08-2.14 (2H, m), 2.63 (2H, t, J = 6.3 Hz), 2.87 (2H, t, J = 6.3 Hz), 3.92 (3H, s), 7.40 (1H, s), 7.75 (1H, s).
MS (m/z) = 302.8 [M+H]+

### Step 2

Copper iodide (2.68 g, 14.1 mmol) and methyl 2,2-difluoro-2-(fluorosulfonyl) acetate (7.42 mL, 58.7 mmol) were added to a DMF (56 mL) solution of Compound 36 (3.76 g, 11.7 mmol), under a nitrogen atmosphere, and the mixture was heated and stirred at 100°C for 2 hours. The reaction solution was cooled to room temperature, then diluted with ethyl acetate, and filtered through Celite (registered trademark). The filtrate was washed by sodium bicarbonate water and water, and the solvent was evaporated under reduced pressure. The obtained residue was purified by silica gel column chromatography (hexane-ethyl acetate) to give Compound 37 2.79 g, yield 92%).
¹H-NMR (CDCl₃) δ: 2.12-2.18 (2H, m), 2.68 (2H, t, J = 6.4 Hz), 2.94 (2H, t, J = 6.4 Hz), 3.94 (3H, s), 7.49 (1H, s), 7.62 (1H, s).
MS (m/z) = 244.8 [M+H]+

### Step 3

A THF solution (1.24 mL, 1.24 mmol) of 1 M lithium bis(trimethylsilyl)amide was added dropwise to a 2-methyltetrahydrofuran (4 mL) solution of Compound 37 (252 mg, 1.03 mmol) and hexamethylphosphoric triamide (0.197 mL, 1.13 mmol) at - 15°C, and the mixture was stirred for 30 minutes at the same temperature. t-Butyl 2-bromoacetate (0.167 mL, 1.13 mmol) was added to the reaction solution, and the mixture was further stirred for 1 hour. Water was added to the reaction solution, and the solution was extracted with ethyl acetate. The organic layer was washed with water, and the solvent was evaporated under reduced pressure. The obtained residue was purified by silica gel column chromatography (hexane-ethyl acetate) to give Compound 38 (252 mg, yield 59%).
¹H-NMR (CDCl₃) δ: 1.48 (9H, s), 1.97 (1H, ddd, J = 25.8, 12.9, 4.5 Hz), 2.22-2.28 (1H, m), 2.40 (1H, dd, J = 16.4, 6.7 Hz), 2.84-3.11 (4H, m), 3.93 (3H, s), 7.47 (1H, s), 7.61 (1H, s).
MS (m/z) = 303.0 [M-tBu+H]+

### Step 4

Sodium borohydride (74.1mg,1.96mmol) was added to a solution of Compound 38 (788mg, 1.96mmol) in an aqueous 2-propanol solution (90%, 8 mL) at 0°C, and the mixture was stirred at the same temperature for 30 minutes. A 0.4 M aqueous hydrochloric acid solution (5 mL) was added to the reaction solution, and the mixture was extracted with ethyl acetate. The organic layer was washed with water, and the solvent was evaporated under reduced pressure. The obtained residue was solidified with hexane, and the obtained solid was collected by filtration to give Compound 39 (542 mg, yield 77%).
¹H-NMR (CDCl₃) δ: 1.48 (9H, s), 1.58-1.64 (1H, m), 1.95-2.00 (1H, m), 2.16 (1H, dt, J = 10.7, 3.9 Hz), 2.36 (1H, dd, J = 15.7, 6.0 Hz), 2.61 (1H, dd, J = 15.7, 7.0 Hz), 2.73 (1H, d, J = 7.5 Hz), 2.76-2.83 (1H, m), 3.89 (3H, s), 4.44 (1H, t, J = 8.2 Hz), 7.24 (1H, s), 7.27 (1H, s).

### Steps 5 to 6

1,1'-Thiocarbonyldiimidazole (379 mg, 2.13 mmol) and 4-dimethylaminopyridine (18.3 mg, 0.150 mmol) were added to a suspension of Compound 39 (540 mg, 1.50 mmol) in toluene (12 mL), and the mixture was stirred at room temperature for 20.5 hours. The solvent of the reaction solution was evaporated under reduced pressure. The obtained residue was purified by silica gel column chromatography (hexane-ethyl acetate) to give Compound 40 (540 mg).

Azobisisobutyronitrile (9.4 mg, 0.057 mmol) and tributyltin hydride (423 mg, 1.45 mmol) were added to a toluene (10 mL) solution of Compound 40 (540 mg), and the mixture was heated at 100°C for 3 hours under a nitrogen atmosphere. The solvent of the reaction solution was evaporated under reduced pressure, and the obtained residue was purified by silica gel column chromatography (hexane-ethyl acetate).

Palladium catalyst on activated carbon (NX type, 10% palladium, 50% water) (88.8 mg, 0.042 mmol) was added to an ethyl acetate (10 mL) solution of the obtained compound (284 mg), and the mixture was stirred at room temperature for 9 hours under a hydrogen stream of 1 atm. The reaction solution was filtered through Celite (registered trademark), and the solvent was evaporated under reduced pressure. The obtained residue was purified by silica gel column chromatography (hexane-ethyl acetate) to give Compound 41 (racemic mixture, 276 mg, 3 steps yield 54%). ¹H-NMR (CDCl₃) δ: 1.39-1.51 (1H, m), 1.48 (9H, s), 1.96 (1H, t, J = 6.5 Hz), 2.26 (3H, tt, J = 15.5, 6.1 Hz), 2.51 (1H, dd, J = 16.8, 10.0 Hz), 2.78 (2H, d, J = 4.8 Hz), 2.91 (1H, dd, J = 16.7, 4.0 Hz), 3.85 (3H, s), 6.67 (1H, s), 7.26 (1H, s).

### Steps 7 to 8

A 1 M boron tribromide dichloromethane solution (2.38 mL, 2.38 mmol) was added to a dichloromethane (5.5 mL) solution of Compound 41(273 mg, 0.793 mmol) at -15°C, and the mixture was stirred at room temperature for 2 hours. The reaction solution was added to an aqueous solution of sodium bicarbonate (1.33 g) and ice (24 g), and a 2 M aqueous hydrochloric acid solution was added to adjust the pH to 4. The obtained reaction solution was extracted with ethyl acetate, washed with water, and dried over anhydrous sodium sulfate, and then, the solvent was evaporated under reduced pressure. Methanol (8 mL) was added to the obtained residue, and the solvent was evaporated under reduced pressure. The obtained residue was purified by silica gel column chromatography (chloroform-methanol) to give Compound 42 (101 mg).

Cesium carbonate (168 mg, 0.517 mmol) was added to a DMF (2 mL) solution of Compound 42 (99 mg) and 1-(bromomethyl)-4-chloro-2-fluorobenzene (85.0 mg, 0.379 mmol), and the mixture was stirred at room temperature for 11 hours. The reaction solution was diluted with ethyl acetate and washed with water. The solvent of the organic layer was evaporated under reduced pressure, and the obtained residue was purified by silica gel column chromatography (hexane-ethyl acetate) to give Compound 43 (racemic mixture, 101 mg, 2 steps yield 29%).
¹H-NMR (CDCl₃) δ:1.39-1.52 (1H, m), 1.91-2.01 (1H, m), 2.19-2.32 (1H, m), 2.37 (2H, dt, J = 16.0, 7.8 Hz), 2.50 (1H, dd, J = 16.8, 10.5 Hz), 2.80 (2H, d, J = 4.8 Hz), 2.91 (1H, dd, J = 16.8, 4.5 Hz), 3.71 (3H, s), 5.13 (2H, s), 6.72 (1H, s), 7.11 (1H, dd, J = 9.8, 2.0 Hz), 7.18 (1H, dd, J = 8.2, 1.6 Hz), 7.29 (1H, s), 7.51 (1H, t, J = 8.2 Hz).

### Step 9

A 1 M aqueous sodium hydroxide solution (0.702 mL, 0.702 mmol) was added to a THF (1.4 mL)-methanol (0.7 mL) solution of Compound 43 (101 mg, 0.234 mmol), and the mixture was stirred at room temperature for 1 hour. A 2 M aqueous hydrochloric acid solution (0.409 mL, 0.819 mmol) was added to the reaction solution, and the mixture was concentrated, then diluted with ethyl acetate. The organic layer was washed with water, the solvent was evaporated under reduced pressure, and the obtained residue was purified by silica gel column chromatography (chloroform-methanol) to give Compound 44 (racemic mixture, 95.6 mg, yield 98%). ¹H-NMR (CDCl₃) δ: 1.43-1.57 (1H, m), 1.97-2.06 (1H, m), 2.28 (1H, dd, J = 7.0, 3.0 Hz), 2.44 (2H, dd, J = 7.0, 2.6 Hz), 2.53 (1H, dd, J = 16.8, 10.5 Hz), 2.81 (2H, d, J = 5.0 Hz), 2.96 (1H, dd, J = 16.8, 4.3 Hz), 5.13 (2H, s), 6.73 (1H, s), 7.11 (1H, dd, J = 9.7, 1.6 Hz), 7.18 (1H, d, J = 8.3 Hz), 7.30 (1H, s), 7.51 (1H, t, J = 8.3 Hz).
MS (m/z) = 414.9 [M-H]-

### Step 10

A 50% ethyl acetate solution of 2,4,6-tripropyl-1,3,5,2,4,6-trioxatriphosphorinane-2,4,6-trioxide was added to a pyridine (2 mL) solution of

Compound 44 (93.4 mg, 0.224 mmol), and Compound 45 (54.8 mg, 0.232 mmol), and the mixture was stirred at room temperature for 30 minutes. Toluene was added to the reaction solution, and the mixture was concentrated and diluted with ethyl acetate. The organic layer was washed with water, and the solvent was evaporated under reduced pressure. The obtained residue was purified by silica gel column chromatography (chloroform-methanol) to give Compound 46 (diastereomeric mixture (1 : 1), 95.6 mg, yield 98%).
MS (m/z) = 635.0 [M+H]+

### Step 11

An acetic acid (1.35 mL) solution of Compound 46 (36.6 mg, 0.058 mmol) was heated and stirred at 60°C for 3.5 hours and further at 80°C for 2.5 hours. Toluene was added to the reaction solution, and the mixture was concentrated, then neutralized with sodium bicarbonate water, and extracted with ethyl acetate. The organic layer was washed with water, the solvent was evaporated under reduced pressure, and the obtained residue was purified by preparatory TLC (toluene-ethyl acetate) to give Compound 47 (diastereomeric mixture (1 : 1), 69.1 mg, yield 64%). ¹H-NMR (CDCl₃) δ: 1.52-1.67 (1H, m), 2.02-2.04 (1H, m), 2.35-2.37 (1H, m), 2.60-2.83 (5H, m), 2.98-3.07 (3H, m). 3.95 (3H, s), 4.31-4.48 (3H, m), 4.61 (1H, q, J = 7.3 Hz), 5.10 (2H, s), 5.10 (2H, s), 5.16-5.20 (1H, m), 6.71 (1H, d, J = 3.6 Hz), 7.10 (1H, dd, J = 9.8, 1.8 Hz), 7.17 (1H, d, J = 8.3 Hz), 7.30 (1H, s), 7.50 (1H, t, J = 8.3 Hz), 7.75 (1H, d, J = 8.5 Hz), 7.98 (1H, dd, J = 8.5, 1.3 Hz), 8.09 (1H, s).
MS (m/z) = 617.0 [M+H]+

### Step 12

A 1 M aqueous sodium hydroxide solution (0.450 mL, 0.450 mmol) was added to a THF (1.4 mL)-methanol (0.7 mL) solution of Compound 47 (66.5 mg, 0.108 mmol), and the mixture was stirred at 50°C for 1.5 hours. A 2 M aqueous hydrochloric acid solution (0.225 mL, 0.450 mmol) was added to the reaction solution, and the mixture was concentrated. Thereafter, water was added thereto, and the resulting solid was collected by filtration, dried under vacuum at 50°C, to give I-077 (diastereomeric mixture (1 : 1), 57.0 mg, 88% yield).
¹H-NMR (CDCl₃) δ: 1.58-1.68 (1H, m), 2.02-2.14 (1H, m), 2.35-2.50 (1H, m), 2.54-2.88 (5H, m), 2.94-3.05 (1H, m), 3.11 (2H, d, J = 5.9 Hz), 4.29-4.52 (3H, m), 4.62 (1H, q, J = 7.2 Hz), 5.09 (2H, s), 5.14-5.23 (1H, m), 6.71 (1H, d, J = 3.9 Hz), 7.09 (1H, dd, J = 9.8, 1.8 Hz), 7.17 (1H, d, J = 8.3 Hz), 7.30 (1H, s), 7.49 (1H, t, J = 8.1 Hz), 7.83 (1H, d, J = 8.5 Hz), 8.06 (1H, d, J = 8.5 Hz), 8.17 (1H, s).
MS (m/z) = 603.0 [M+H]+

### Example 5

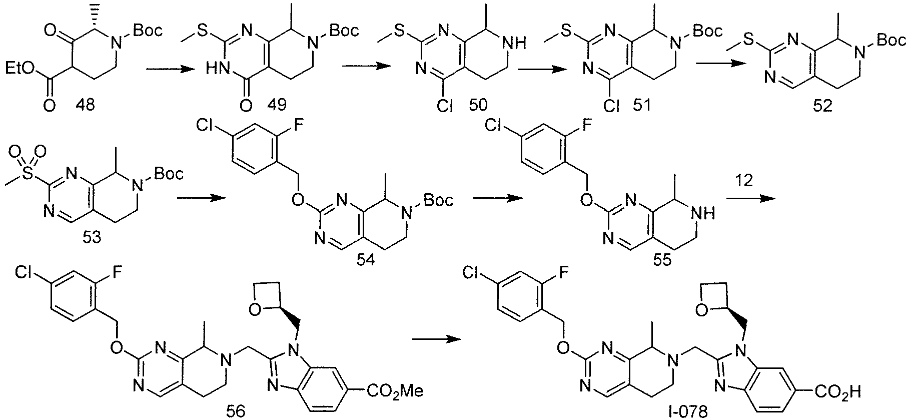

### Step 1

Thiourea (1.07 g, 14.0 mmol) and a 20% ethanol solution of sodium ethoxide were added to Compound 48 (800 mg, 3.07 mmol), and the mixture was stirred at 90°C for 2 hours. The reaction solution was cooled to room temperature, methyl iodide (1.49 g, 10.5 mmol) was added, and the mixture was stirred at room temperature for 1 hour. DOWEX 50Wx8 100-200 Mesh (H) Cation Exchange Resin was added to the reaction solution, and the solid was removed by filtration. The solvent of the filtrate was evaporated under reduced pressure, and the obtained residue was purified by silica gel column chromatography (hexane-ethyl acetate) to give Compound 49 (1.98 g, yield 91%).
MS (m/z) = 312.0 [M+H]+
¹H-NMR (CDCl₃) δ: 1.43 (3H, d, J = 6.8 Hz), 1.49 (9H, s), 2.58-2.50 (5H, m), 2.92-2.90 (1H, m), 4.31-4.18 (1H, m), 4.94-4.76 (1H, m), 10.86 (1H, s).

### Step 2

Phosphorus oxychloride (1.95 g, 12.7 mmol) was added to an acetonitrile (20 mL) solution of the compound 49 (1.98 g, 6.36 mmol), and the mixture was stirred at 90°C for 1 hour. The solvent was evaporated under reduced pressure to give Compound 50.
MS (m/z) = 229.9 [M+H]+

### Step 3

Di-tert-butyl dicarbonate (2.77 g, 12.7 mmol) and sodium hydrogen carbonate (2.67 g, 31.8 mmol) were added to a THF (15 mL)-water (15 mL) mixture solution of Compound 50 (1.46 g, 6.36 mmol), and the mixture was stirred at room temperature for 1 hour. Water was added to the reaction solution, and the solution was extracted with ethyl acetate. The solvent was evaporated under reduced pressure, and the obtained residue was purified by silica gel column chromatography (hexane-ethyl acetate) to give Compound 51 (1.66 g, yield 79%).
¹H-NMR (CDCl₃) δ: 1.51-1.49 (12H, m), 2.55 (3H, s), 2.76-2.74 (2H, m), 3.02 (1H, br s), 4.37 (1H, br s), 5.06 (1H, br s).
MS (m/z) = 330.0 [M+H]+

### Step 4

A THF (6 mL) solution of Compound 51 (400 mg, 1.21 mmol), 10% palladium carbon (400 mg, 0.38 mmol), and triethylamine (368 mg, mmol to 3.64) was stirred under a hydrogen atmosphere at room temperature for 6 hours. Insolubles in the reaction solution were removed by filtration, and the solvent of the filtrate was evaporated under reduced pressure. The obtained residue was purified by silica gel column chromatography (hexane-ethyl acetate) to give Compound 52 (360 mg, yield 100%).
MS (m/z) = 297.0 [M+H]+
¹H-NMR (CDCl₃) δ: 1.50-1.49 (12H, m), 2.55 (3H, s), 2.63 (1H, d, J = 15.6 Hz), 2.82 (1H, td, J = 15.2, 5.4 Hz), 3.01 (1H, s), 4.34 (1H, br s), 5.06 (1H, br s), 8.28 (1H, s).

### Step 5

Compound 52 (360 mg, 1.21 mmol) was dissolved in dichloromethane (5.4 mL), and 70% meta-chloroperbenzoic acid (600 mg, 2.42 mmol) was added, then the mixture was stirred at room temperature for 1 hour. An aqueous sodium hydrogen carbonate solution and a sodium thiosulfate aqueous solution were added to the reaction solution, and the solution was extracted with ethyl acetate. The solvent was evaporated under reduced pressure to give Compound 53.
MS (m/z) = 328.0 [M+H]+

### Step 6

60% Sodium hydride (58.2 mg, 2.42 mmol) and Compound 53 (397 mg, 1.21 mmol) were added to a THF (4 mL) solution of (4-chloro-2-fluorophenyl)methanol (389 mg, 2.42 mmol), and the mixture was stirred at room temperature for 1 hour. Water was added to the reaction solution, and the solution was extracted with chloroform. The solvent was evaporated under reduced pressure, and the obtained residue was purified by silica gel column chromatography (hexane-ethyl acetate) to give Compound 54 (410 mg, yield 83%).
MS (m/z) = 408.0 [M+H]+
¹H-NMR (CDCl₃) δ: 1.49 (9H, s), 1.57 (3H, d, J = 5.0 Hz), 2.64 (1H, d, J = 15.3 Hz), 2.81 (1H, t, J = 11.2 Hz), 3.01 (1H, s), 4.34 (1H, br s), 5.06 (1H, br s), 5.46-5.41 (2H, m), 7.13-7.10 (2H, m), 7.48 (1H, t, J = 7.9 Hz), 8.28 (1H, s).

### Step 7

Trifluoroacetic acid (0.2 mL) was added to a dichloromethane (0.6 mL) solution of Compound 54(40 mg, 0.098 mmol), and the mixture was stirred at room temperature for 1 hour. The solvent was evaporated under reduced pressure, and the obtained residue was purified by silica gel column chromatography (hexane-ethyl acetate) to give Compound 55 (28 mg, yield 93%).
MS (m/z) = 308.0 [M+H]+
¹H-NMR (CDCl₃) δ: 1.50 (3H, d, J = 6.5 Hz), 2.69-2.65 (1H, m), 2.79-2.77 (1H, m), 2.99-2.96 (1H, m), 3.29-3.27 (1H, m), 3.99-3.97 (1H, m), 5.40 (1H, d, J = 13.1 Hz), 5.46 (1H, d, J = 12.8 Hz), 7.11 (2H, t, J = 8.4 Hz), 7.48 (1H, t, J = 7.5 Hz), 8.23 (1H, s).

### Step 8

Compound 12 (26 mg, 0.086 mmol) and potassium carbonate (25 mg, 0.182 mmol) were added to an acetonitrile (0.3 mL) solution of Compound 55 (28 mg, 0.091 mmol), and the mixture was stirred at 60°C for 3 hours. An aqueous hydrochloric acid solution was added to the reaction solution to adjust pH to about 7, and the solution was extracted with chloroform. The solvent of the organic layer was evaporated under reduced pressure, and the obtained residue was purified by silica gel column chromatography (hexane-ethyl acetate) to give Compound 56 (36 mg, yield 70%).
MS (m/z) = 566.0 [M+H]+
¹H-NMR (CDCl₃) δ: 1.57-1.50 (3H, m), 2.48-2.35 (1H, m), 2.80-2.60 (4H, m), 3.07-3.00 (1H, m), 3.87-3.77 (1H, m), 3.95 (3H, s), 4.13-4.06 (1H, m), 4.38-4.34 (2H, m), 4.87-4.57 (3H, m), 5.21-5.19 (1H, m), 5.40 (1H, d, J = 12.5 Hz), 5.46 (1H, d, J = 13.1 Hz), 7.13-7.10 (2H, m), 7.48 (1H, t, J = 8.0 Hz), 7.76 (1H, dd, J = 8.5, 3.3 Hz), 7.99 (1H, d, J = 8.5 Hz), 8.13 (1H, s), 8.26 (1H, s).

### Step 9

A 1 M aqueous sodium hydroxide solution (0.12 mL, 0.12 mmol) was added to a methanol (0.15 mL)-THF (0.15 mL) solution of Compound 56 (36 mg, 0.064 mmol), and the mixture was stirred at room temperature for 19 hours. An aqueous citric acid solution was added to the reaction solution to adjust pH to about 7, and the solution was extracted with chloroform. The solvent was evaporated under reduced pressure, and the obtained residue was purified by silica gel column chromatography (chloroform-methanol) to give Compound I-078 (diastereomeric mixture, 28 mg, yield 80%).
MS (m/z) = 552.0 [M+H]+
¹H-NMR (CDCl₃) δ: 1.54 (3H, dd, J = 26.2, 6.7 Hz), 2.50-2.38 (1H, m), 2.80-2.61 (4H, m), 3.07-3.04 (1H, m), 3.84-3.80 (1H, m), 4.16-4.09 (1H, m), 4.45-4.31 (2H, m), 4.89-4.56 (3H, m), 5.21-5.19 (1H, m), 5.39 (1H, d, J = 13.1 Hz), 5.46 (1H, d, J = 13.1 Hz), 7.13-7.10 (2H, m), 7.48 (1H, t, J = 7.9 Hz), 7.82 (1H, dd, J = 8.5, 3.3 Hz), 8.05 (1H, d, J = 8.5 Hz), 8.20 (1H, s), 8.27 (1H, s).

### Example 6

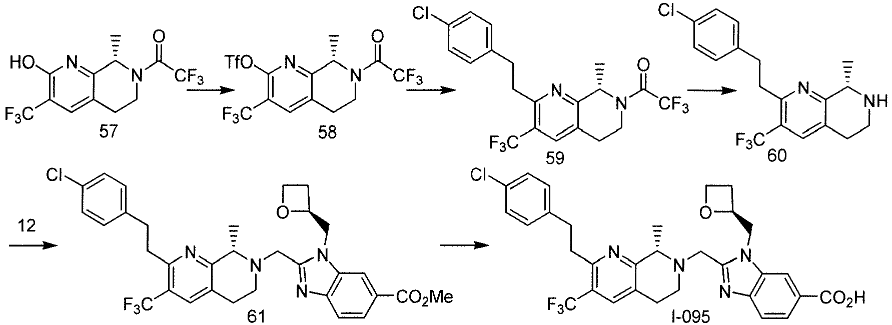

### Step 1

Trifluoromethanesulfonic anhydride (386 µL, 2.29 mmol) and triethylamine (422 µL, 3.05 mmol) were added to a dichloromethane (7.5 mL) solution of Compound 57 (500 mg, 1.52 mmol) under ice cooling, and then, the mixture was stirred under ice cooling for 1 hour. An aqueous sodium hydrogen carbonate solution was added to the reaction solution, and extracted with ethyl acetate. The organic layer was dried over anhydrous sodium sulfate, and the solvent was evaporated under reduced pressure. The obtained residue was purified by silica gel column chromatography (hexane-ethyl acetate) to give Compound 58 (717 mg, yield 97%).
¹H-NMR (CDCl₃) δ: 1.62 (2.25H, d, J = 6.9 Hz), 1.69 (0.75H, d, J = 6.8 Hz), 2.90-2.99 (1H, m), 3.04-3.24 (1.25H, m), 3.47-3.55 (0.75H, m), 4.24 (0.75H, dd, J = 14.2, 4.4 Hz), 4.81 (0.25H, dd, J = 13.2, 5.3 Hz), 5.16 (0.25H, q, J = 6.6 Hz), 5.60 (0.75H, q, J = 6.9 Hz), 7.92 (0.75H, s), 7.94 (0.25H, s).
MS (m/z) = 461 [M+H]+

### Step 2

2-(4-Chlorophenyl) ethylboronic acid pinacol ester (127 mg, 0.48 mmol), palladium (19.5 mg, 0.02 mmol), cesium carbonate (156 mg, 0.48 mmol) and water (100 µL) were added to a solution of Compound 58 (110 mg, 0.24 mmol) in 1,4-dioxane (1 mL), and the mixture was stirred at 100°C for 2 hours under a nitrogen atmosphere. Water was added to the reaction solution, and the solution was extracted with ethyl acetate. The organic layer was dried over anhydrous sodium sulfate, the solvent was evaporated under reduced pressure, and the obtained residue was purified by silica gel column chromatography (hexane-ethyl acetate) to give Compound 59 (12 mg, yield 11%).
¹H-NMR (CDCl₃) δ: 1.60 (2.1H, d, J = 6.9 Hz), 1.66 (0.9H, d, J = 6.8 Hz), 2.81-2.89 (1H, m), 2.98-3.10 (3H, m), 3.13-3.25 (2.3H, m), 3.48-3.55 (0.7H, m), 4.18 (0.7H, dd, J = 14.2, 4.1 Hz), 4.75 (0.3H, dd, J = 13.4, 5.8 Hz), 5.18 (0.3H, q, J = 6.8 Hz), 5.60 (0.7H, q, J = 6.9 Hz), 7.13-7.24 (4H, m), 7.66 (0.7H, s), 7.69 (0.3H, s).
MS (m/z) = 451 [M+H]+

### Step 3

Compound 60 (12 mg) was obtained by performing the same reaction as in Step 10 of Example 1.
¹H-NMR (CDCl₃) δ: 1.55 (3H, d, J = 6.8 Hz), 2.79 (1H, dt, J = 16.4, 4.0 Hz), 2.91-2.99 (1H, m), 3.02-3.10 (3H, m), 3.13-3.24 (2H, m), 3.32 (1H, dt, J = 12.5, 5.0 Hz), 4.16 (1H, q, J = 6.4 Hz), 7.16 (2H, d, J = 8.3 Hz), 7.24 (2H, d, J = 8.3 Hz), 7.58 (1H, s).
MS (m/z) = 355 [M+H]+

### Step 4

Compound 61 (14 mg, yield 85%) was obtained by performing the same reaction as in Step 11 of Example 1.
¹H-NMR (CDCl₃) δ: 1.53 (3H, br s), 2.36-2.45 (1H, m), 2.65-2.89 (4H, m), 3.00-3.24 (4H, m), 3.95 (3H, s), 4.00 (1H, br s), 4.18 (1H, s), 4.32-4.37 (1H, m), 4.41 (1H, br s), 4.60 (1H, dd, J = 13.8, 7.9 Hz), 4.72 (2H, br s), 5.17-5.23 (1H, m), 7.16 (2H, d, J = 8.4 Hz), 7.24 (2H, d, J = 8.4 Hz), 7.62 (1H, s), 7.77 (1H, d, J = 8.3 Hz), 7.99 (1H, d, J = 8.7 Hz), 8.14 (1H, s).
MS (m/z) = 613 [M+H]+

### Step 5

A 2 M aqueous sodium hydroxide solution (57 µL, 0.11 mmol) was added to a methanol (0.15 mL)-THF (0.15 mL) solution of Compound 61 (14 mg, 0.02 mmol), and the mixture was stirred at 45°C for 2 hours. The solvent was evaporated under reduced pressure, the residue was diluted with water, then diluted sulfuric acid was added thereto until the pH reached about 4, and the solution was extracted with ethyl acetate twice. The organic layer was dried over anhydrous sodium sulfate, and the solvent was evaporated under reduced pressure. The obtained residue was purified by silica gel column chromatography (chloroform-methanol) to give Compound I-095 (8 mg, yield 59%).
¹H-NMR (CDCl₃) δ: 1.52 (3H, br s), 2.35-2.45 (1H, m), 2.65-3.24 (9H, m), 4.01 (1H, br s), 4.21 (1H, br s), 4.32-4.37 (1H, m), 4.40 (1H, br s), 4.61 (1H, dd, J = 14.0, 7.6 Hz), 4.74 (2H, br s), 5.18-5.23 (1H, br m), 7.15 (2H, d, J = 8.4 Hz), 7.23 (2H, d, J = 8.4 Hz), 7.62 (1H, s), 7.82 (1H, d, J = 8.5 Hz), 8.04 (1H, d, J = 8.5 Hz), 8.19 (1H, s).
MS (m/z) = 599 [M+H]+

### Example 7

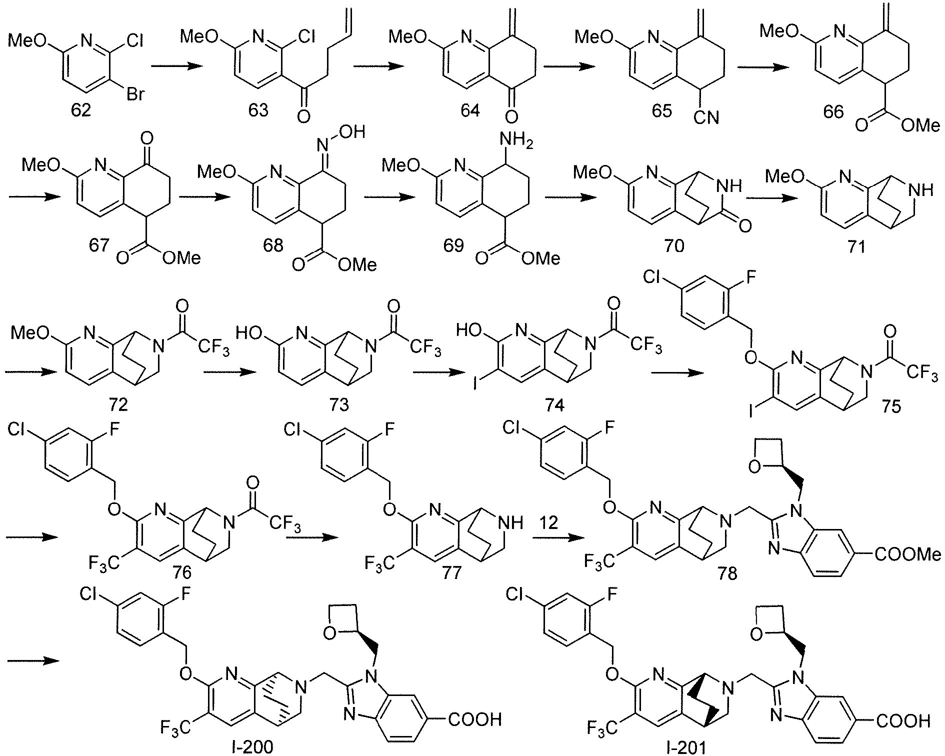

### Step 1

An isopropylmagnesium chloride lithium chloride complex THF solution (20.8 mL, 27.0 mmol) was added dropwise to a THF (30 mL) solution of Compound 62 (5.00 g, 22.5 mmol) at -20°C, and the mixture was stirred at -10°C for 1 hour. 4-Pentenoyl chloride (3.7 mL, 33.7 mmol) was added to the reaction solution, and the mixture was stirred at 0°C for 5 hours. An aqueous hydrochloric acid solution was added to the reaction solution, and the mixture was extracted with ethyl acetate twice. The organic layer was washed with water and brine, and the solvent was evaporated under reduced pressure. The obtained residue was purified by silica gel column chromatography (hexane-ethyl acetate) to give Compound 63 (4.81 g, yield 94%). MS (m/z) = 225.9 [M+H]+

### Step 2

Palladium (II) acetate (762 mg, 3.39 mmol), BINAP(4.23 g, 6.79 mmol), and acetonitrile (80 mL) were added to Compound 63 (4.81 g, 17.0 mmol), and the mixture was stirred at 85°C for 17.5 hours. The reaction solution was cooled to room temperature, water was added thereto, and the solution was extracted with ethyl acetate twice. The organic layer was dried over anhydrous sodium sulfate, and the solvent was evaporated under reduced pressure. The obtained residue was purified by silica gel column chromatography (hexane-ethyl acetate) to give Compound 64 (3.12 g, yield 97%).
¹H-NMR (CDCl₃) δ: 2.70-2.74 (2H, m), 2.88-2.92 (2H, m), 4.04 (3H, s), 5.43 (1H, q, J = 1.6 Hz), 6.35 (1H, s), 6.73 (1H, d, J = 8.7 Hz), 8.16 (1H, d, J = 8.7 Hz).
MS (m/z) = 189.9 [M+H]+

### Step 3

THF (62 mL) and ethanol (6 mL) were added to Compound 64 (3.12 g, 16.5 mmol), and the mixture was ice-cooled. p-Toluenesulfonylmethyl isocyanide (4.83 g, 24.7 mmol) and potassium-tert-butoxide (2.78 g, 24.7 mmol) were added, and the mixture was stirred at room temperature for 6 hours. In the middle of stirring, at the timings of 2.5 hours and 4.5 hours, p-toluenesulfonylmethyl isocyanide (4.83 g, 24.7 mmol) and potassium-tert-butoxide (2.78 g, 24.7 mmol) were further added. An aqueous ammonium chloride solution was added to the reaction solution, and the solution was extracted with ethyl acetate twice. The solvent of the organic layer was evaporated under reduced pressure, and the obtained residue was purified by silica gel column chromatography (hexane-ethyl acetate) to give Compound 65 (1.41 g, yield 43%).
MS (m/z) = 201.0 [M+H]+

### Step 4

Methanol (35 mL) and trimethylsilyl chloride (27.0 mL, 211 mmol) were added to Compound 65 (1.41 g, 7.0 mmol), and the mixture was stirred at 50°C for 5 hours. An aqueous sodium hydrogen carbonate solution was added to the reaction solution, and the solution was extracted with ethyl acetate twice. The solvent of the organic layer was evaporated under reduced pressure, and the obtained residue was purified by silica gel column chromatography (hexane-ethyl acetate) to give Compound 66 (563 mg, yield 34%).
MS (m/z) = 234.0 [M+H]+

### Step 5

Methanol (11 mL) and dichloromethane (11 mL) were added to Compound 66 (563 mg, 2.42 mmol), and the mixture was stirred at room temperature to perform an ozonolysis reaction. After the reaction, triphenylphosphine (1266 mg, 4.83 mmol) was added to the reaction solution, and the mixture was stirred at room temperature. The reaction solution was concentrated, and the obtained residue was purified by silica gel column chromatography (hexane-ethyl acetate) to give Compound 67 (482 mg, yield 85%).
MS (m/z) = 236.0 [M+H]+

### Step 6

Ethanol (17 mL), water (1 mL), hydroxylamine hydrochloride (185 mg, 2.66 mmol) and triethylamine (0.43 mL, 3.07 mmol) were added to Compound 67 (482 mg, 2.05 mmol), and the mixture was stirred at room temperature for 30 minutes. Water was added to the reaction solution, and the solution was extracted with ethyl acetate twice. The organic layer was washed with aqueous hydrochloric acid solution, water, and brine, and the organic layer was concentrated to give an extraction residue of a crude product of Compound 68 (424 mg, 83%).
MS (m/z) = 251.0 [M+H]+

### Step 7

Methanol (12 mL), acetic acid (1.5 mL) and 10% palladium-carbon (361 mg, 0.34 mmol) were added to the total amount (1.69 mmol) of the crude product of Compound 68, and the mixture was stirred for 1 hour under a hydrogen atmosphere. The reaction solution was filtered through Celite, and washed with methanol. The solvent of the filtrate was evaporated under reduced pressure to give the crude product of Compound 69.
MS (m/z) = 237.0 [M+H]+

### Step 8

Methanol (25 mL) was added to the total amount (1.69 mmol) of the crude product of Compound 69, and the mixture was stirred under ice cooling. 55% Sodium hydride (163 mg, 3.73 mmol) was added to the mixture solution, and the mixture was stirred at 50°C for 8.5 hours. The solvent of the reaction solution was evaporated under reduced pressure, and the obtained residue was purified by silica gel column chromatography (chloroform-methanol) to give Compound 70 (200.4 mg, yield 58%). ¹H-NMR (CDCl₃) δ: 1.61-1.69 (1H, m), 1.71-1.79 (1H, m), 2.09-2.16 (1H, m), 2.19-2.27 (1H, m), 3.78 (1H, q, J = 2.5 Hz), 3.92 (3H, s), 4.63-4.66 (1H, m), 6.21 (1H, s), 6.61 (1H, d, J = 8.1 Hz), 7.46 (1H, d, J = 8.1 Hz).
MS (m/z) = 205.0 [M+H]+

### Step 9

Borane dimethyl sulfide complex (1.86 mL, 19.6 mmol) was added to a THF (8 mL) solution of Compound 70 (200 mg, 0.98 mmol), and the mixture was stirred at 65°C for 14 hours. Methanol (10 mL) was added to the reaction solution, and after 1 hour at 65°C, a 2 mol/L aqueous hydrochloric acid solution (2 mL) was added, and the mixture was stirred at 70°C for 1 hour. The solvent of the reaction solution was evaporated under reduced pressure to give a crude product of Compound 71.

### Step 10

Compound 72 (338.7 mg) was obtained by performing the same reaction as in Step 1 of Example 2.
MS (m/z) = 287.0 [M+H]+

### Step 11

Sodium iodide (0.28 g, 1.89 mmol), trimethylsilyl chloride (0.24 mL, 1.89 mmol), and acetonitrile (5 mL) were added to Compound 72 (180 mg, 0.63 mmol), and the mixture was stirred at 50°C for 16 hours. In the middle of stirring, sodium iodide and trimethylsilyl chloride were appropriately added in order to allow the reaction to proceed. Sodium hydrogen carbonate and an aqueous sodium thiosulfate solution were added to the reaction solution, and the solution was extracted with ethyl acetate. The organic layer was dried over anhydrous sodium sulfate, and the solvent was evaporated under reduced pressure to give a crude product of Compound 73.
MS (m/z) = 273.1 [M+H]+

### Step 12

N,N-formamide (5 mL) and N-iodosuccinimide (212 mg, 0.94 mmol) were added to the total amount (0.63 mmol) of the crude product of Compound 73, and the mixture was stirred at room temperature for 20 minutes. After iced water was added to the reaction solution, the solution was extracted with ethyl acetate. The organic layer was dried over anhydrous sodium sulfate, and the solvent was evaporated under reduced pressure to give a crude product of Compound 74.

### Step 13

1,4-Dioxane (5 mL), silver (I) carbonate (346 mg, 1.26 mmol), and 4-chloro-2-fluorobenzyl bromide (154 mg, 0.69 mmol) were added to the whole amount (0.63 mmol) of the crude product of Compound 74, and the mixture was stirred at 65°C for 2 hours. Insolubles were removed by filtration, the solvent of the reaction solution was evaporated under reduced pressure, and the obtained residue was purified by silica gel column chromatography (hexane-ethyl acetate) to give Compound 75 (103 mg, yield 30%) and a mixture of Compound 75 and 4-chloro-2-fluorobenzyl bromide (244 mg, purity 43%, yield 31%).
MS (m/z) = 541.1 [M+H]+

### Step 14

N,N-Dimethylformamide (3.7 mL), copper iodide (185 mg, 0.97 mmol), and methyl difluoro(fluorosulfonyl)acetate (123 µL, 0.97 mmol) were added to a mixture of Compound 75 and 4-chloro-2-fluorobenzyl bromide (244 mg, 43% purity, 0.19 mmol), then the container was degassed under reduced pressure and purged with nitrogen. The reaction solution was stirred at 100°C for 1 hour under a nitrogen atmosphere. The reaction solution was cooled to room temperature, water was added thereto, and the solution was extracted with ethyl acetate. After the organic layer was dried over anhydrous sodium sulfate, the solvent was evaporated under reduced pressure, and the obtained residue was purified by silica gel column chromatography (hexane-ethyl acetate) to give Compound 76 (80 mg, yield 85%).
MS (m/z) = 483.2 [M+H]+

### Step 15

A crude product of Compound 77 was obtained by performing the same reaction as in Step 10 of Example 1.

### Step 16

Acetonitrile (0.64 mL), Compound 12 (49 mg, 0.17 mmol), and potassium carbonate (46 mg, 0.33 mmol) were added to the total amount (0.17 mmol) of the crude product of Compound 77, then the mixture was stirred at 65°C for 2 hours and allowed to stand at room temperature overnight. Water was added to the reaction solution, and the solution was extracted with ethyl acetate. The organic layer was dried over anhydrous magnesium sulfate, and the solvent was evaporated under reduced pressure to give a crude product of Compound 78.

### Step 17

After a 1 M aqueous sodium hydroxide solution (0.5 mL, 0.50 mmol) was added to a methanol (0.5 mL)-THF (0.5 mL) solution of the whole amount (0.17 mmol) of the crude product of Compound 78, then the mixture was stirred at 45°C for 5.5 hours. An aqueous hydrochloric acid solution was added to the reaction solution to adjust the pH to about 4, and the solution was extracted with ethyl acetate. The organic layer was dried over anhydrous sodium sulfate, and the solvent was evaporated under reduced pressure to give a residue of a diastereomeric mixture. The obtained diastereomeric mixture was separated by SFC to give Compound I-200 (21.2 mg, 20% yield) and Compound I-201 (26.6 mg, 26% yield).

### SFC Conditions

Column: ID column (manufactured by Daicel Corporation) 4.6×250 mm
Analysis time: 15 min
Mobile phase: A is carbon dioxide, and B is a 2-propanol solution of 0.1% diethylamine
Fractionation conditions: the solution was sent with a compositional ratio of B/(A + B) = 50% being kept
Flow rate: 2.0 mL/min
Detection wavelength: 220 nm

### Compound I-200

¹H-NMR (CDCl₃) δ: 1.32-1.41 (1H, m), 1.57 (1H, t, J = 12.6 Hz), 1.82 (1H, t, J = 11.7 Hz), 2.27-2.43 (3H, m), 2.65-2.75 (1H, m), 3.07-3.14 (2H, m), 3.47 (1H, d, J = 13.6 Hz), 3.93 (1H, s), 4.09-4.17 (1H, m), 4.26-4.32 (1H, m), 4.54-4.64 (2H, m), 4.85 (1H, dd, J = 15.4, 2.9 Hz), 5.20-5.26 (1H, m), 5.55 (2H, q, J = 13.4 Hz), 7.08-7.16 (2H, m), 7.52 (1H, t, J = 8.1 Hz), 7.72-7.77 (2H, m, J = 8.1 Hz), 8.02 (1H, dd, J = 8.5, 1.4 Hz), 8.20 (1H, s).

MS (m/z) = 631.3 [M+H]+

### Compound I-201

¹H-NMR (CDCl₃) 6: 1.36-1.43 (1H, m), 1.61 (1H, t, J = 12.6 Hz), 1.84 (1H, t, J = 11.5 Hz), 2.29-2.37 (2H, m), 2.43-2.53 (1H, m), 2.74-2.82 (1H, m), 3.07-3.14 (2H, m), 3.42 (1H, d, J = 13.6 Hz), 3.97 (1H, s), 4.08-4.15 (1H, m), 4.43-4.53 (2H, m), 4.66 (1H, dd, J = 14.1, 7.8 Hz), 4.91 (1H, dd, J = 15.2, 7.3 Hz), 5.19 (1H, ddd, J = 14.3, 7.2, 2.5 Hz), 5.56 (2H, dd, J = 29.3, 13.4 Hz), 7.08-7.16 (2H, m), 7.52 (1H, t, J = 8.1 Hz), 7.72-7.74 (2H, m), 8.00 (1H, dd, J = 8.5, 1.3 Hz), 8.20 (1H, s).

MS (m/z) = 631.3 [M+H]+

The following compounds were also synthesized in the same manner by using the general synthesis method described above or the synthesis method described in Examples. I-015 and I-090 to 1-093 in the table are diastereomeric mixtures.

**[Table 1]**

| **No.** | **Structure** | **No.** | **Structure** |
|---|---|---|---|
| **I-001** | 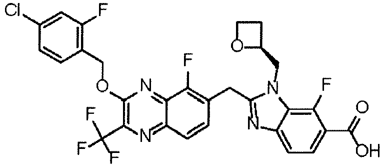 | **I-009** | 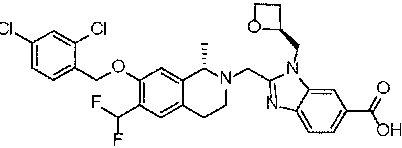 |
| **I-002** | 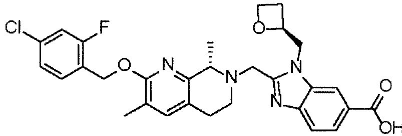 | **I-010** | 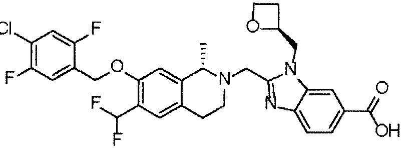 |
| **I-003** | 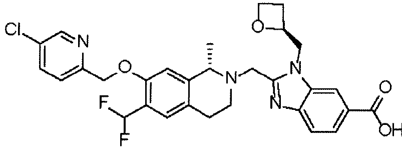 | **I-011** | 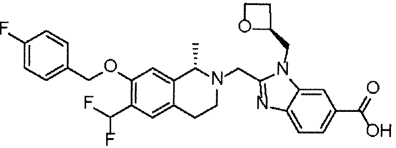 |
| **I-004** | 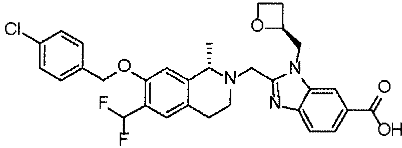 | **I-012** | 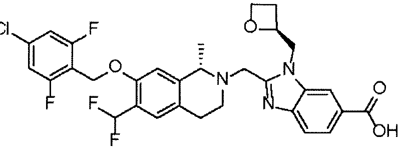 |
| **I-005** | 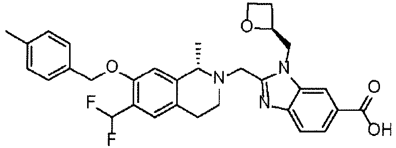 | **I-013** | 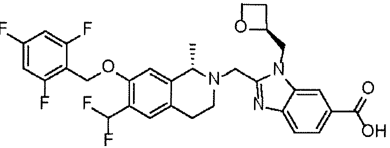 |
| **I-006** | 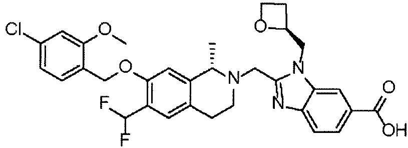 | **I-014** | 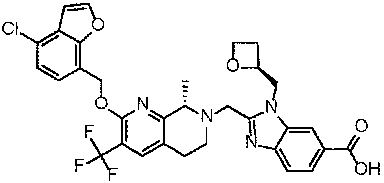 |
| **I-007** | 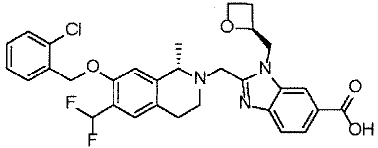 | **I-015** | 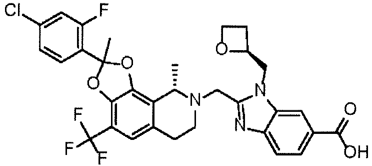 |
| **I-008** | 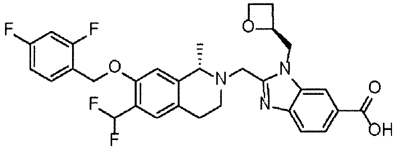 | **I-016** | 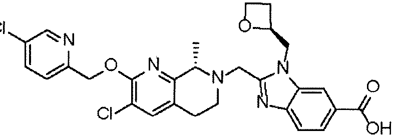 |

**[Table 2]**

| **No.** | **Structure** | **No.** | **Structure** |
|---|---|---|---|
| **I-017** | 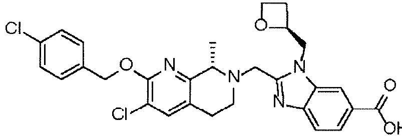 | **I-026** | 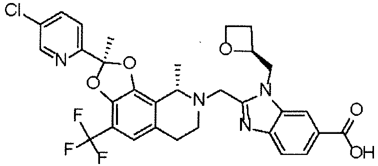 |
| **I-018** | 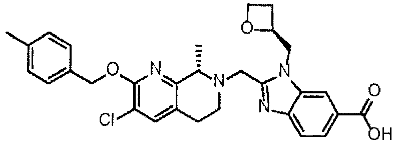 | **I-027** | 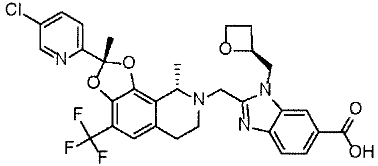 |
| **I-019** | 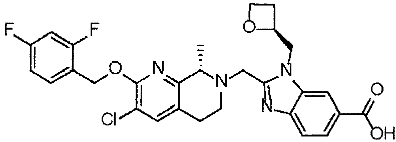 | **I-028** | 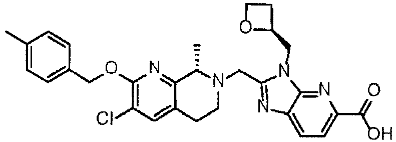 |
| **I-020** | 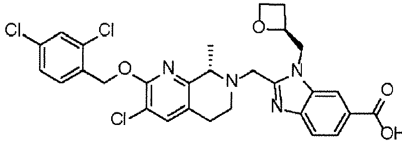 | **I-029** | 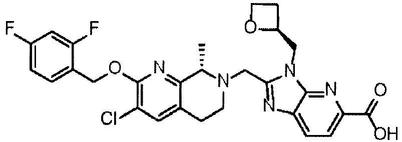 |
| **I-021** | 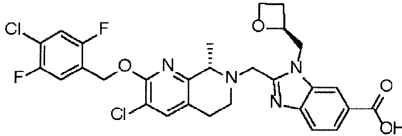 | **I-030** | 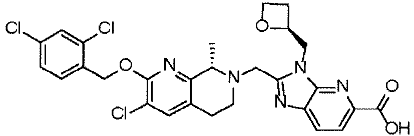 |
| **I-022** | 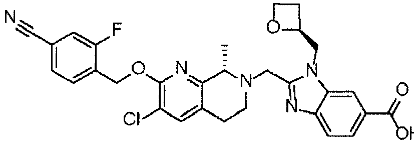 | **I-031** | 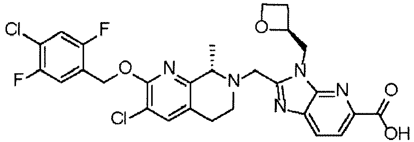 |
| **I-023** | 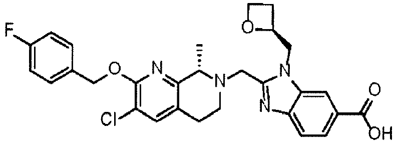 | **I-032** | 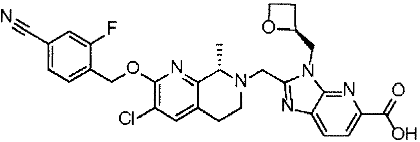 |
| **I-025** | 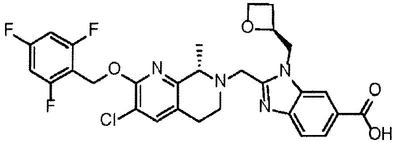 | **I-033** | 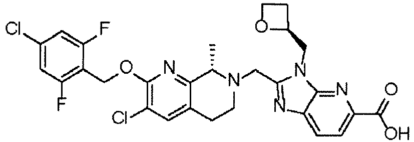 |

**[Table 3]**

| **No.** | **Structure** | **No.** | **Structure** |
|---|---|---|---|
| **I-034** | 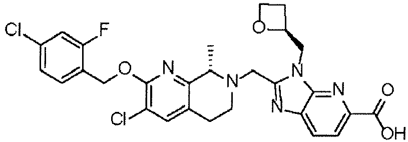 | **I-042** | 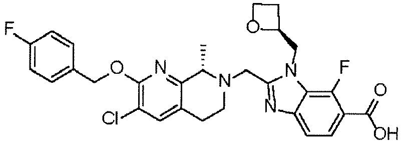 |
| **I-035** | 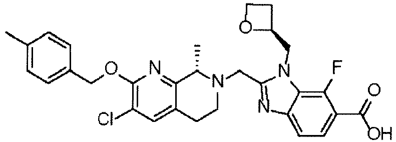 | **I-043** | 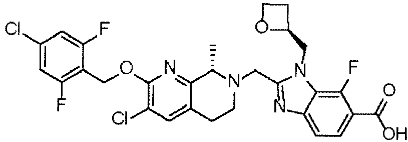 |
| **I-036** | 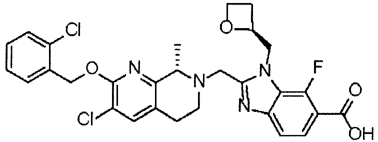 | **I-044** | 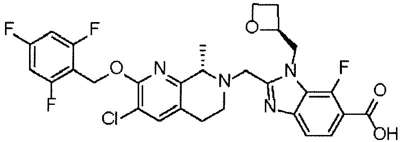 |
| **I-037** | 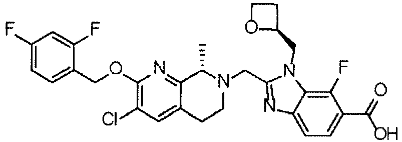 | **I-045** | 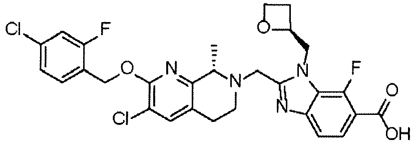 |
| **I-038** | 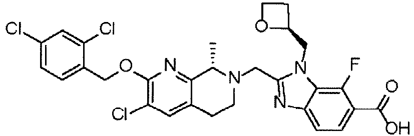 | **I-046** | 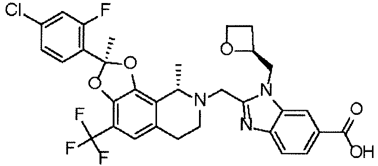 |
| **I-039** | 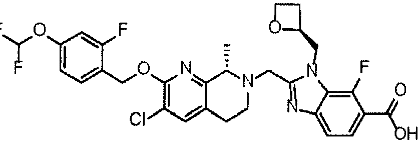 F | **I-048** | 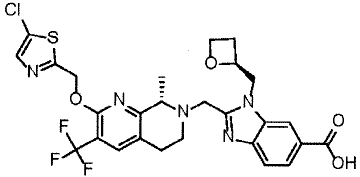 |
| **I-040** | 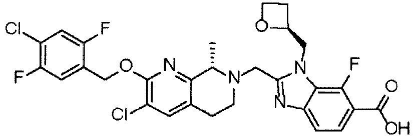 | **I-049** | 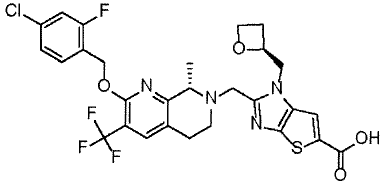 |
| **I-041** | 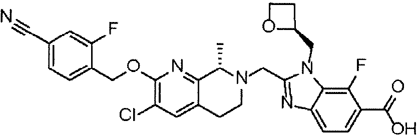 | **I-050** | 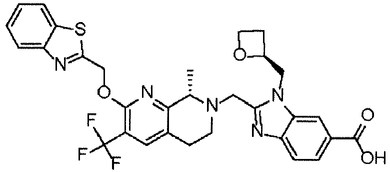 |

**[Table 4]**

| **No.** | **Structure** | **No.** | **Structure** |
|---|---|---|---|
| **I-051** | 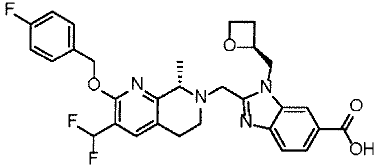 | **I-058** | 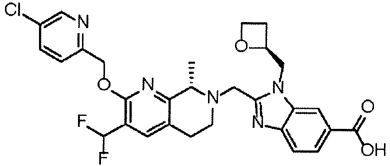 |
| **I-052** | 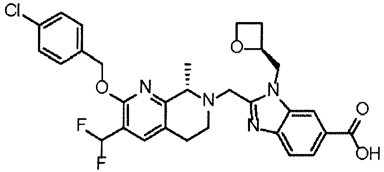 | **I-059** | 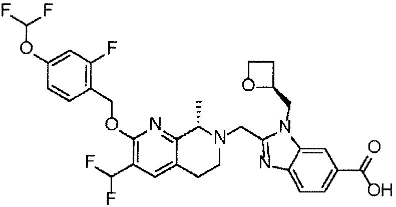 |
| **I-053** | 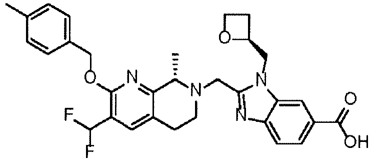 | **I-060** | 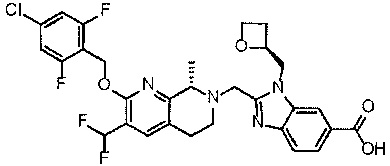 |
| **I-054** | 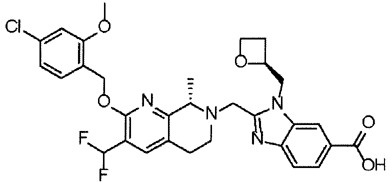 | **I-061** | 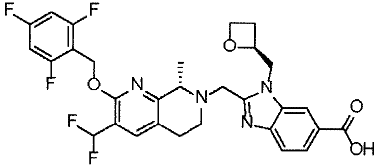 |
| **I-055** | 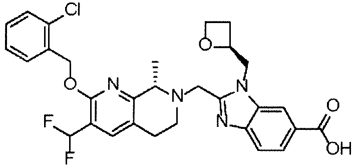 | **I-062** | 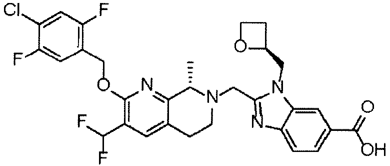 |
| **I-056** | 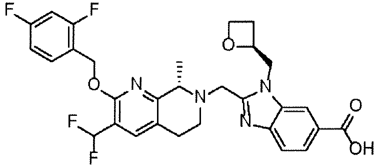 | **I-063** | 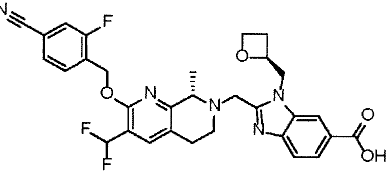 |
| **I-057** | 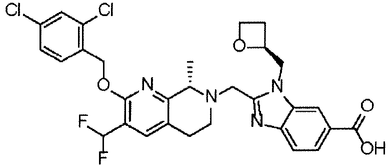 | **I-064** | 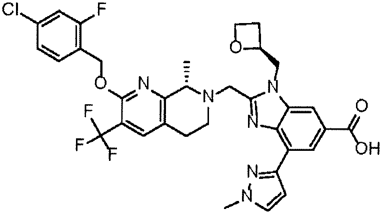 |

**[Table 5]**

| **No.** | **Structure** | **No.** | **Structure** |
|---|---|---|---|
| **I-065** | 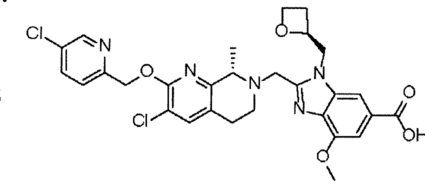 | **I-072** | 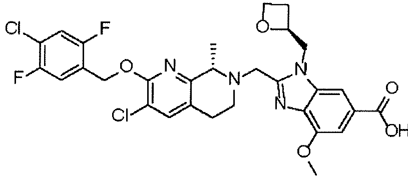 |
| **I-066** | 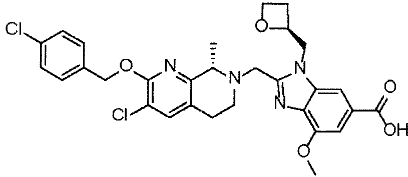 | **I-073** | 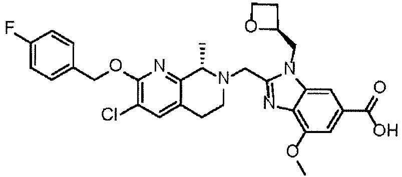 |
| **I-067** | 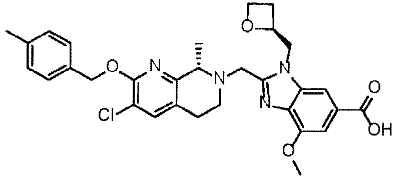 | **I-074** | 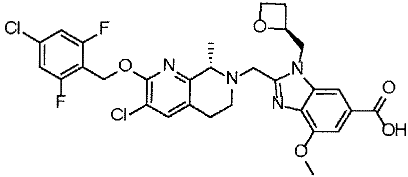 |
| **I-068** | 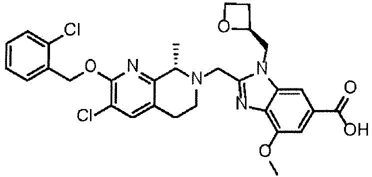 | **I-075** | 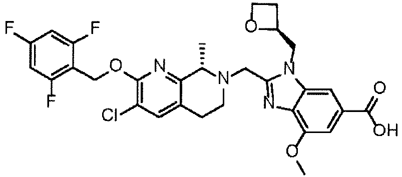 |
| **I-069** | 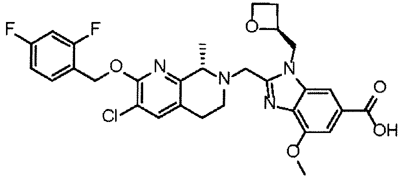 | **I-076** | 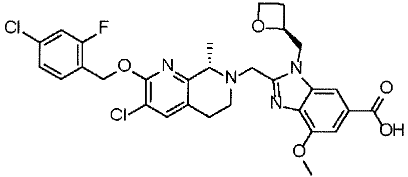 |
| **I-070** | 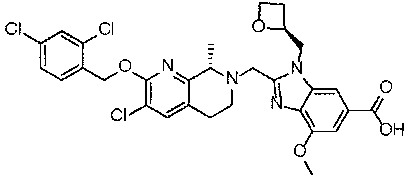 | **I-079** | 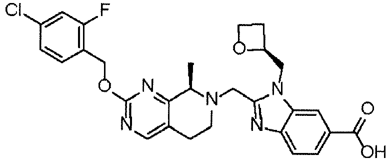 |
| **I-071** | 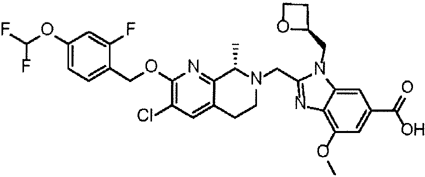 | **I-080** | 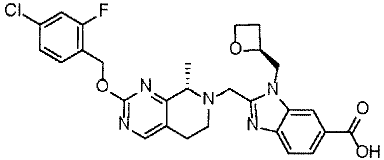 |

**[Table 6]**

| **No.** | **Structure** | **No.** | **Structure** |
|---|---|---|---|
| **I-081** | 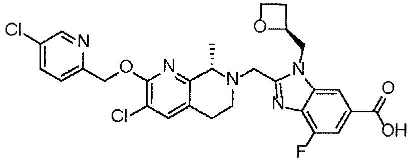 | **I-089** | 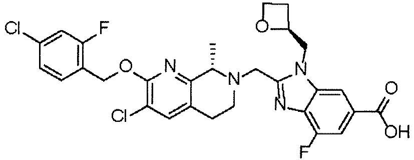 |
| **I-082** | 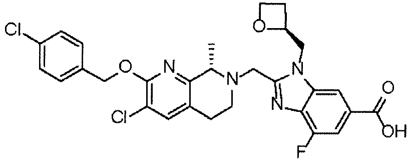 | **I-090** | 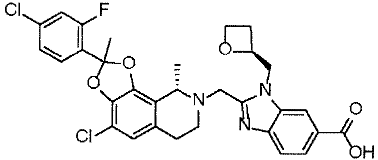 |
| **I-083** | 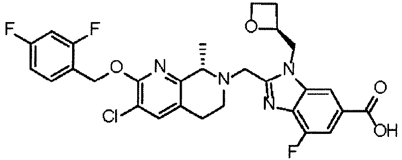 | **I-091** | 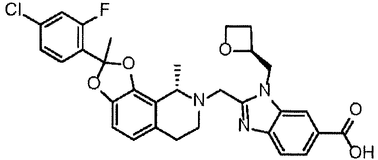 |
| **I-084** | 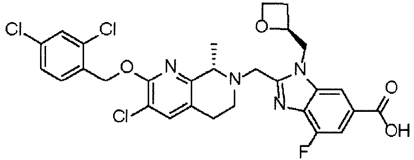 | **I-092** | 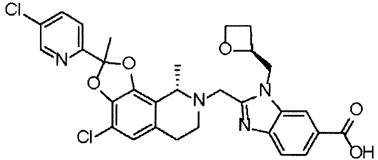 |
| **I-085** | 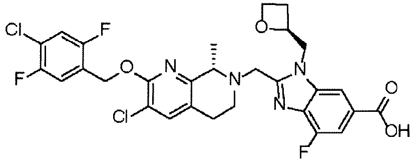 | **I-093** | 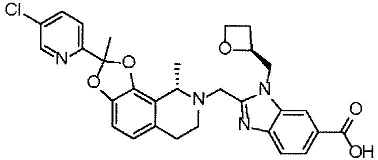 |
| **I-086** | 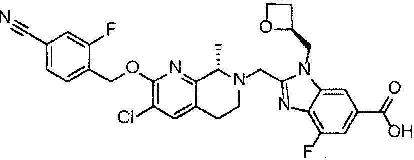 | **I-094** | 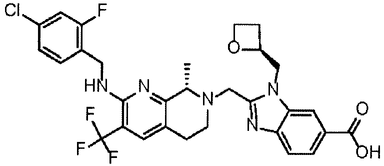 |
| **I-087** | 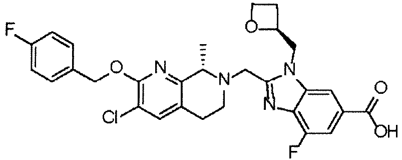 | **I-096** | 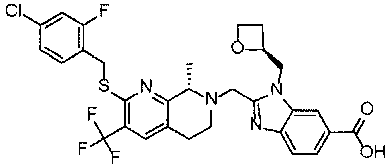 |
| **I-088** | 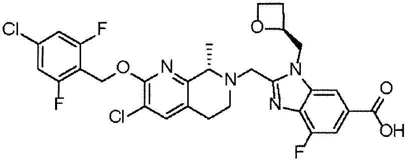 | **I-098** | 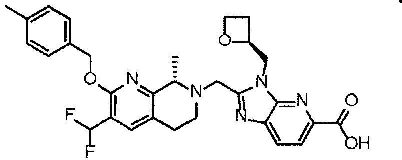 |

**[Table 7]**

| **No.** | **Structure** | **No.** | **Structure** |
|---|---|---|---|
| **I-099** | 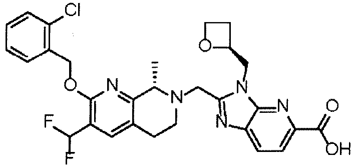 | **I-106** | 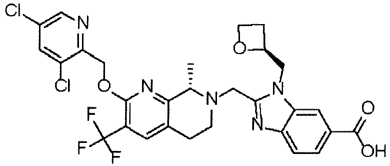 |
| **I-100** | 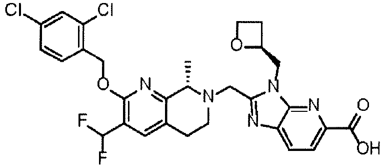 | **I-107** | 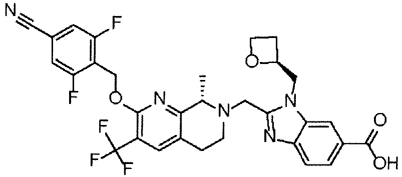 |
| **I-101** | 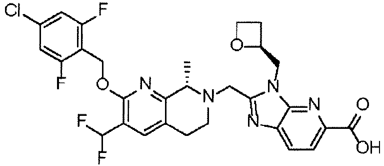 | **I-108** | 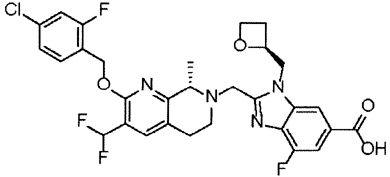 |
| **I-102** | 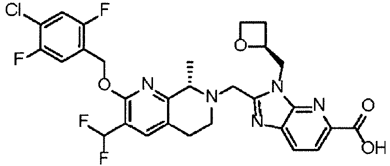 | **I-109** | 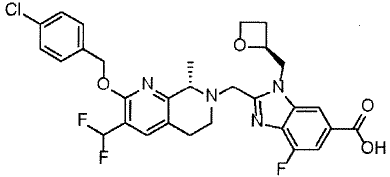 |
| **I-103** | 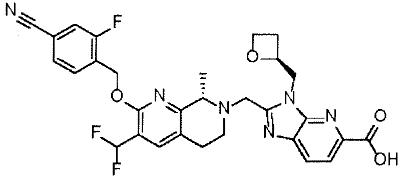 | **I-110** | 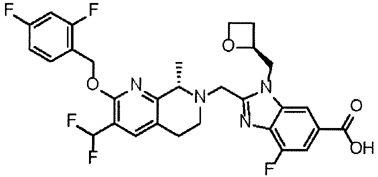 |
| **I-104** | 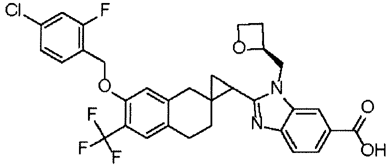 | **I-111** | 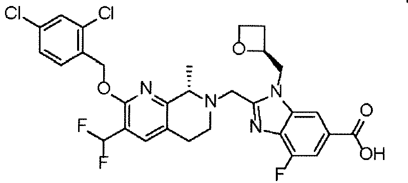 |
| **I-105** | 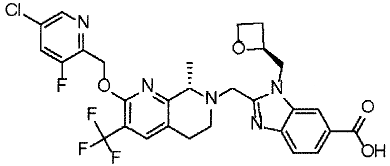 | **I-112** | 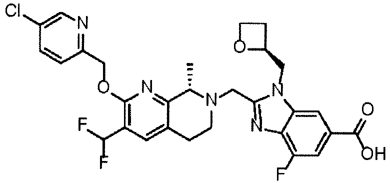 |

**[Table 8]**

| **No.** | **Structure** | **No.** | **Structure** |
|---|---|---|---|
| **I-113** | 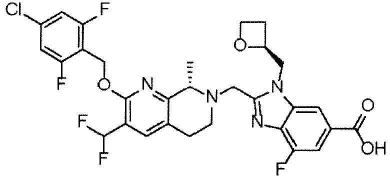 | **I-120** | 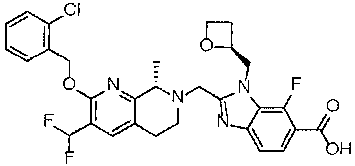 |
| **I-114** | 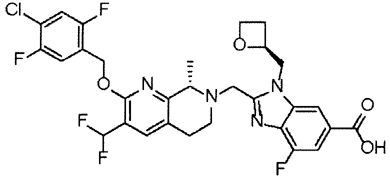 | **I-121** | 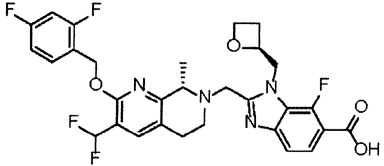 |
| **I-115** | 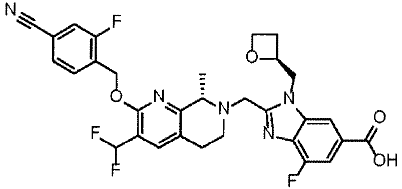 | **I-122** | 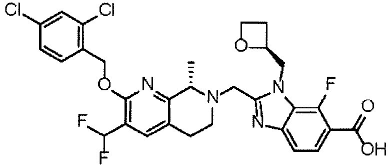 |
| **I-116** | 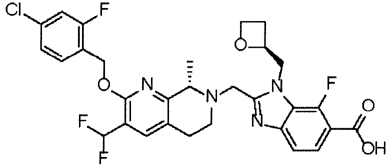 | **I-123** | 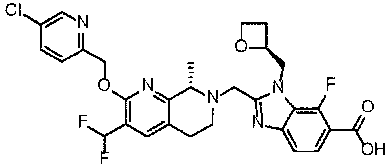 |
| **I-117** | 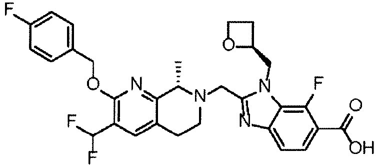 | **I-124** | 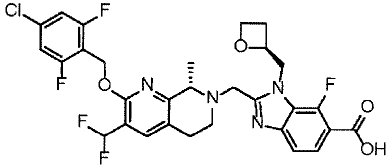 |
| **I-118** | 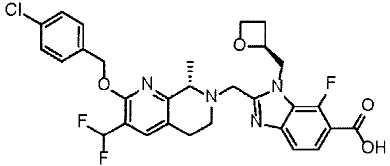 | **I-125** | 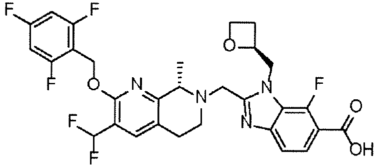 |
| **I-119** | 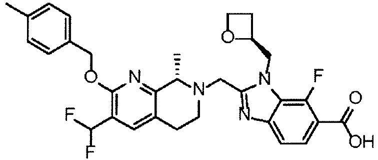 | **I-126** | 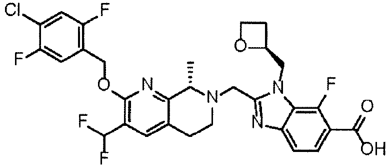 |

**[Table 9]**

| **No.** | **Structure** | **No.** | **Structure** |
|---|---|---|---|
| **I-127** | 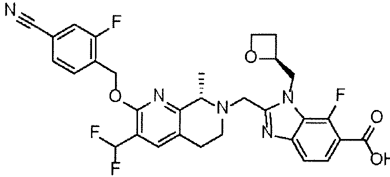 | **I-134** | 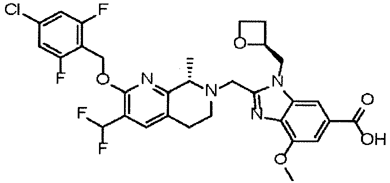 |
| **I-128** | 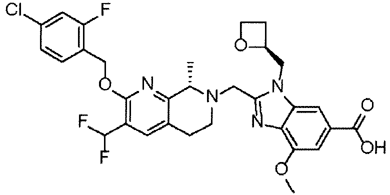 | **I-135** | 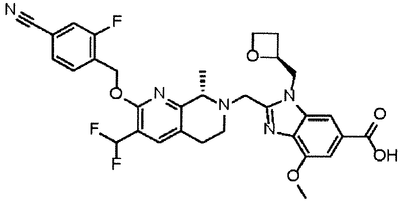 |
| **I-129** | 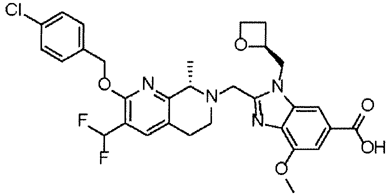 | **I-136** | 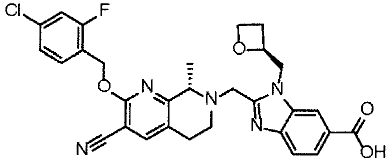 |
| **I-130** | 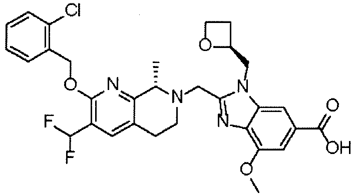 | **I-137** | 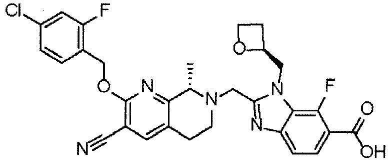 |
| **I-131** | 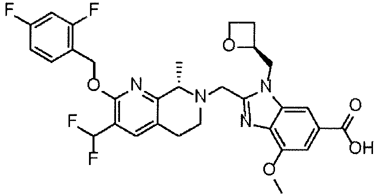 | **I-138** | 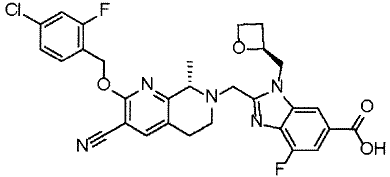 |
| **I-132** | 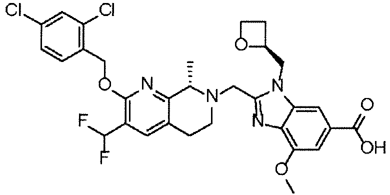 | **I-139** | 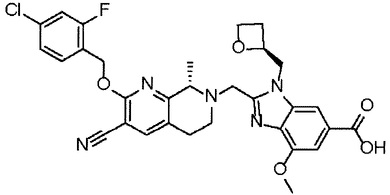 |
| **I-133** | 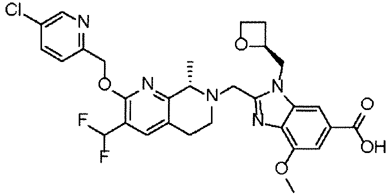 | | |

**[Table 10]**

| **No.** | **Structure** | **No.** | **Structure** |
|---|---|---|---|
| **I-141** | 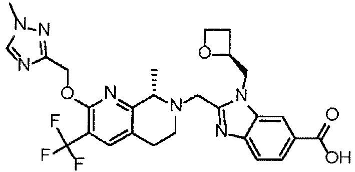 | **I-148** | 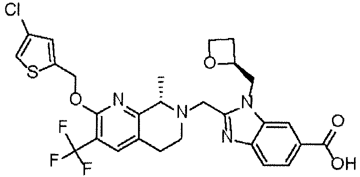 |
| **I-142** | 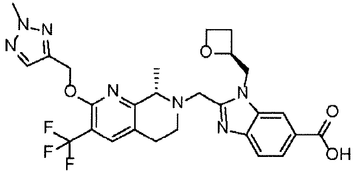 | **I-149** | 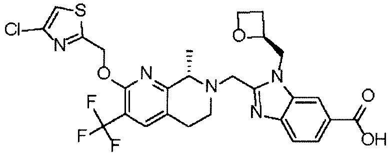 |
| **I-143** | 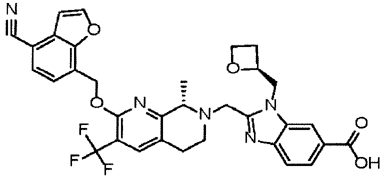 | **I-150** | 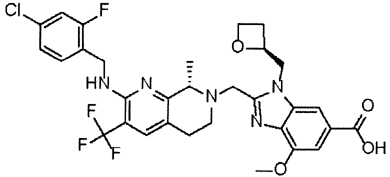 |
| **I-144** | 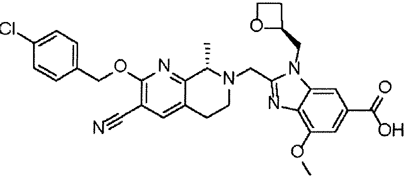 | **I-151** | 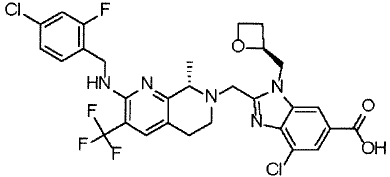 |
| **I-145** | 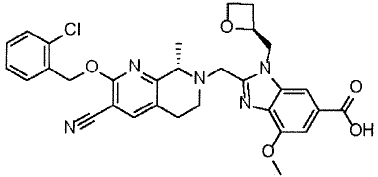 | **I-152** | 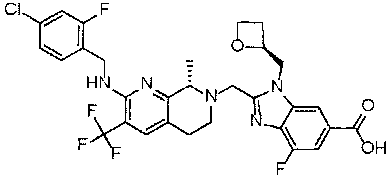 |
| **I-146** | 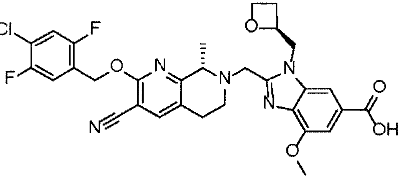 | **I-153** | 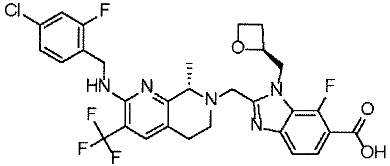 |
| **I-147** | 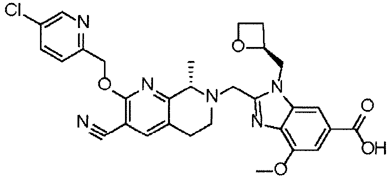 | **I-154** | 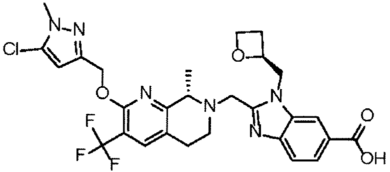 |

**[Table 11]**

| **No.** | **Structure** | **No.** | **Structure** |
|---|---|---|---|
| **I-155** | 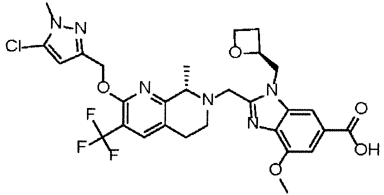 | **I-162** | 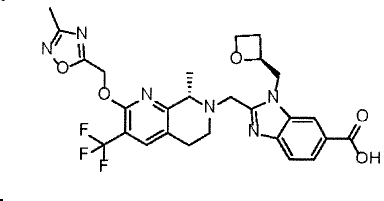 |
| **I-156** | 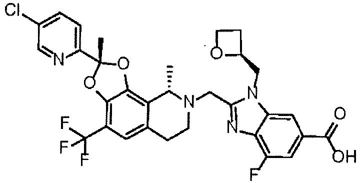 | **I-163** | 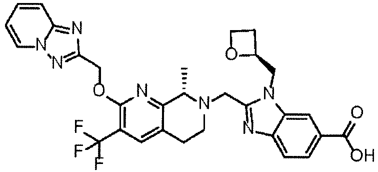 |
| **I-157** | 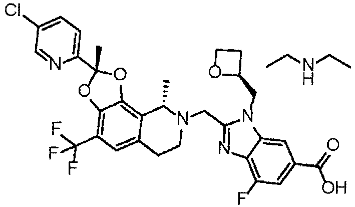 | **I-164** | 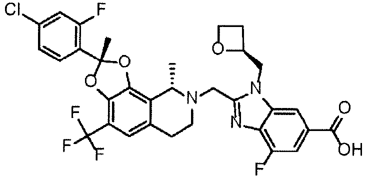 |
| **I-158** | 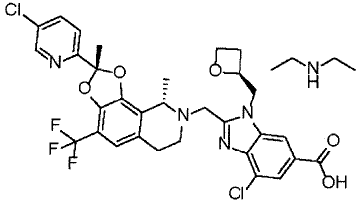 | **I-165** | 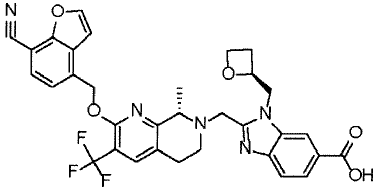 |
| **I-159** | 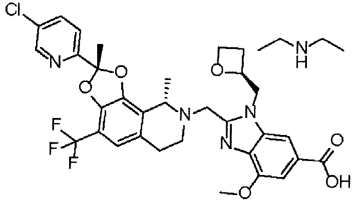 | **I-166** | 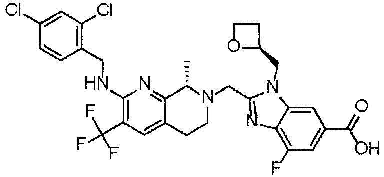 |
| **I-160** | 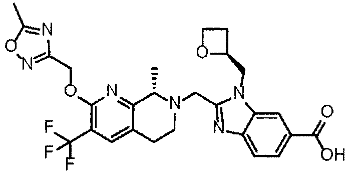 | **I-167** | 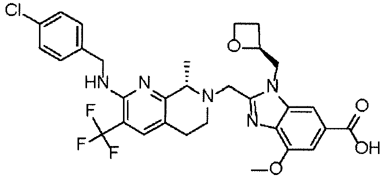 |
| **I-161** | 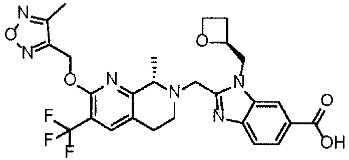 | **I-168** | 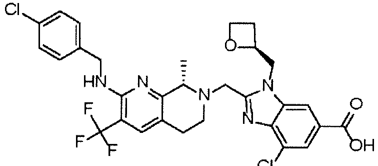 |

**[Table 12]**

| **No.** | **Structure** | **No.** | **Structure** |
|---|---|---|---|
| **I-169** | 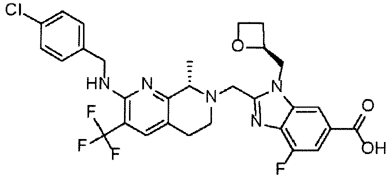 | **I-175** | 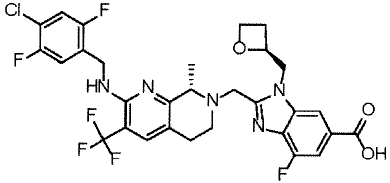 |
| **I-170** | 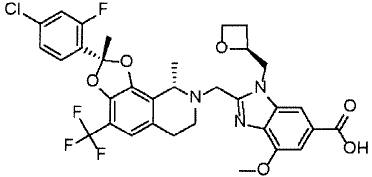 | **I-176** | 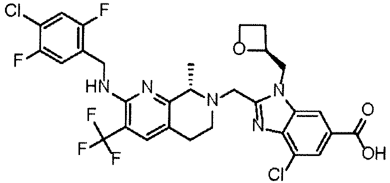 |
| **I-171** | 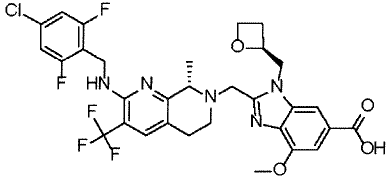 | **I-177** | 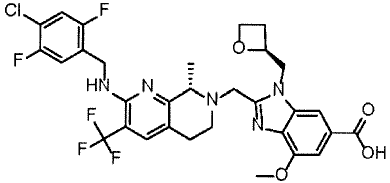 |
| **I-172** | 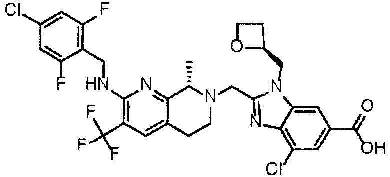 | **I-178** | 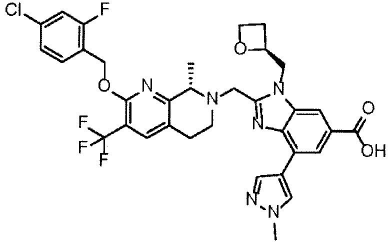 |
| **I-173** | 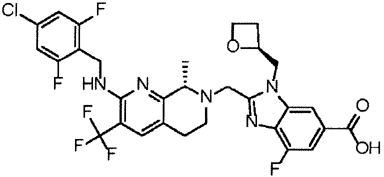 | **I-179** | 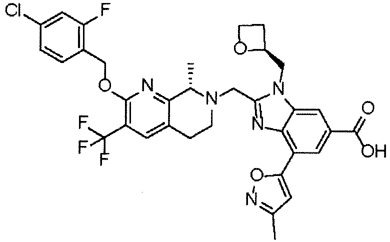 |
| **I-174** | 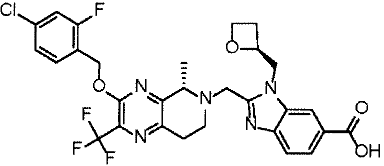 | **I-180** | 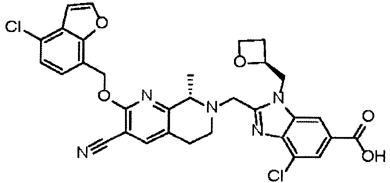 |

**[Table 13]**

| **No.** | **Structure** | **No.** | **Structure** |
|---|---|---|---|
| **I-181** | 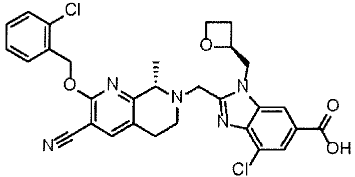 | **I-186** | 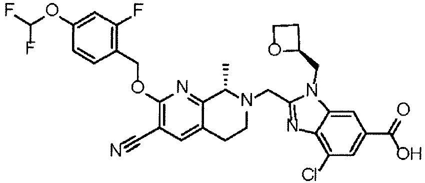 |
| **I-182** | 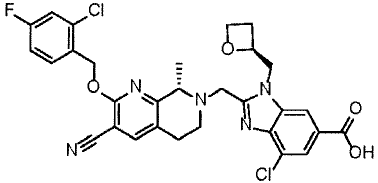 | **I-187** | 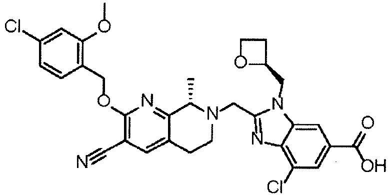 |
| **I-183** | 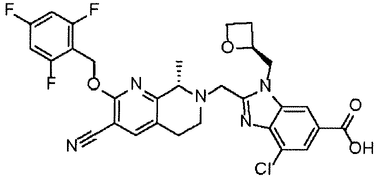 | **I-188** | 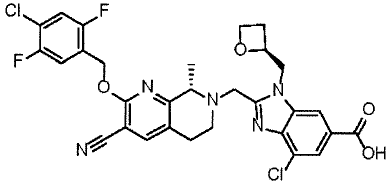 |
| **I-184** | 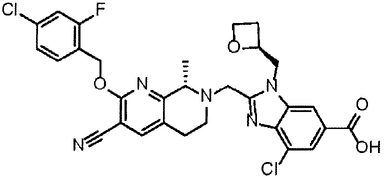 | **I-189** | 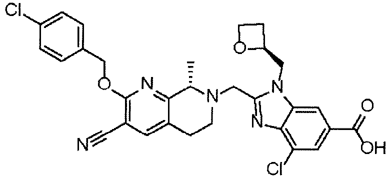 |
| **I-185** | 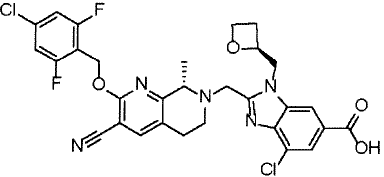 | **I-190** | 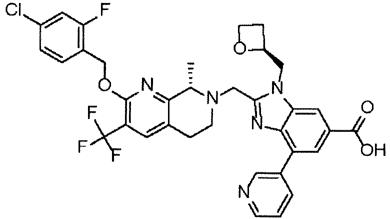 |

**[Table 14]**

| **No.** | **Structure** | **No.** | **Structure** |
|---|---|---|---|
| **I-191** | 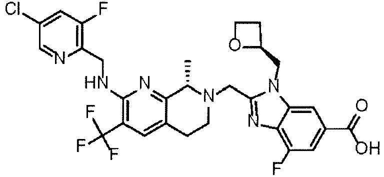 | **I-196** | 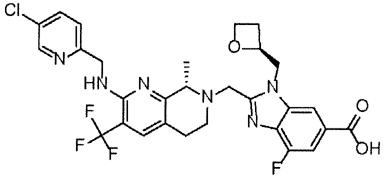 |
| **I-193** | 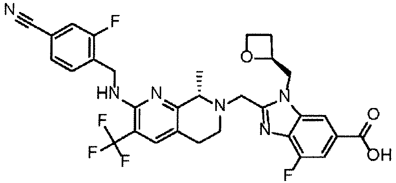 | **I-197** | 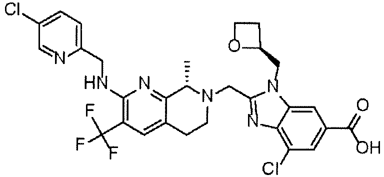 |
| **I-194** | 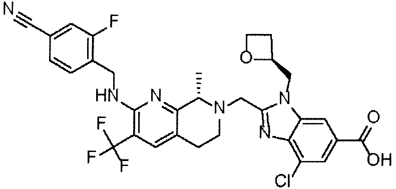 | **I-198** | 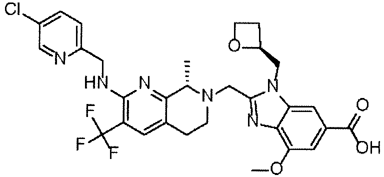 |
| **I-195** | 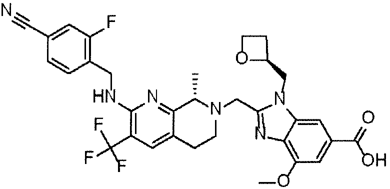 | **I-199** | 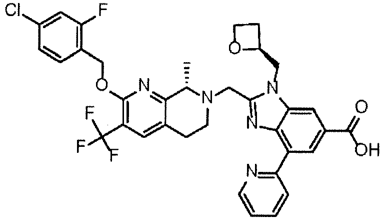 |

**[Table 15]**

| No. | MS | Charge | No. | MS | Charge | No. | MS | Charge | No. | MS | Charge |
|---|---|---|---|---|---|---|---|---|---|---|---|
| I-001 | 637 | M+H | I-049 | 625 | M+H | I-097 | 602 | M+H | I-145 | 588 | M+H |
| I-002 | 565 | M+H | I-050 | 624 | M+H | I-098 | 564 | M+H | I-146 | 624 | M+H |
| I-003 | 583 | M+H | I-051 | 567 | M+H | I-099 | 584 | M+H | I-147 | 589 | M+H |
| I-004 | 582 | M+H | I-052 | 583 | M+H | I-100 | 618 | M+H | I-148 | 607 | M+H |
| I-005 | 562 | M+H | I-053 | 563 | M+H | I-101 | 620 | M+H | I-149 | 610 | M+H |
| I-006 | 612 | M+H | I-054 | 613 | M+H | I-102 | 620 | M+H | I-150 | 649 | M+H |
| I-007 | 582 | M+H | I-055 | 583 | M+H | I-103 | 593 | M+H | I-151 | 652 | M+H |
| I-008 | 584 | M+H | I-056 | 585 | M+H | I-104 | 615 | M+H | I-152 | 636 | M+H |
| I-009 | 616 | M+H | I-057 | 617 | M+H | I-105 | 620 | M+H | I-153 | 636 | M+H |
| I-010 | 618 | M+H | I-058 | 584 | M+H | I-106 | 636 | M+H | I-154 | 605 | M+H |
| I-011 | 566 | M+H | I-059 | 633 | M+H | I-107 | 628 | M+H | I-155 | 635 | M+H |
| I-012 | 618 | M+H | I-060 | 619 | M+H | I-108 | 619 | M+H | I-156 | 648 | M+H |
| I-013 | 602 | M+H | I-061 | 603 | M+H | I-109 | 601 | M+H | I-157 | 647 | M+H |
| I-014 | 641 | M+H | I-062 | 619 | M+H | I-110 | 603 | M+H | I-158 | 663 | M+H |
| I-015 | 646 | M+H | I-063 | 592 | M+H | I-111 | 635 | M+H | I-159 | 659 | M+H |
| I-016 | 568 | M+H | I-064 | 700 | M+H | I-112 | 602 | M+H | I-160 | 573 | M+H |
| I-017 | 567 | M+H | I-065 | 598 | M+H | I-113 | 637 | M+H | I-161 | 573 | M+H |
| I-018 | 547 | M+H | I-066 | 597 | M+H | I-114 | 637 | M+H | I-162 | 573 | M+H |
| I-019 | 569 | M+H | I-067 | 577 | M+H | I-115 | 610 | M+H | I-163 | 608 | M+H |
| I-020 | 601 | M+H | I-068 | 597 | M+H | I-116 | 619 | M+H | I-164 | 664 | M+H |
| I-021 | 603 | M+H | I-069 | 599 | M+H | I-117 | 585 | M+H | I-165 | 632 | M+H |
| I-022 | 576 | M+H | I-070 | 631 | M+H | I-118 | 601 | M+H | I-166 | 652 | M+H |
| I-023 | 551 | M+H | I-071 | 647 | M+H | I-119 | 581 | M+H | I-167 | 630 | M+H |
| I-024 | 603 | M+H | I-072 | 633 | M+H | I-120 | 601 | M+H | I-168 | 634 | M+H |
| I-025 | 587 | M+H | I-073 | 581 | M+H | I-121 | 603 | M+H | I-169 | 618 | M+H |
| I-026 | 629 | M+H | I-074 | 633 | M+H | I-122 | 635 | M+H | I-170 | 676 | M+H |
| I-027 | 629 | M+H | I-075 | 617 | M+H | I-123 | 602 | M+H | I-171 | 666 | M+H |
| I-028 | 548 | M+H | I-076 | 615 | M+H | I-124 | 637 | M+H | I-172 | 670 | M+H |
| I-029 | 570 | M+H | I-077 | 603 | M+H | I-125 | 621 | M+H | I-173 | 654 | M+H |
| I-030 | 602 | M+H | I-078 | 552 | M+H | I-126 | 637 | M+H | I-174 | 620 | M+H |
| I-031 | 604 | M+H | I-079 | 552 | M+H | I-127 | 610 | M+H | I-175 | 654 | M+H |
| I-032 | 577 | M+H | I-080 | 552 | M+H | I-128 | 631 | M+H | I-176 | 670 | M+H |
| I-033 | 604 | M+H | I-081 | 586 | M+H | I-129 | 613 | M+H | I-177 | 666 | M+H |
| I-034 | 586 | M+H | I-082 | 585 | M+H | I-130 | 613 | M+H | I-178 | 699 | M+H |
| I-035 | 565 | M+H | I-083 | 587 | M+H | I-131 | 615 | M+H | I-179 | 700 | M+H |
| I-036 | 585 | M+H | I-084 | 619 | M+H | I-132 | 647 | M+H | I-180 | 632 | M+H |
| I-037 | 587 | M+H | I-085 | 621 | M+H | I-133 | 614 | M+H | I-181 | 592 | M+H |
| I-038 | 619 | M+H | I-086 | 594 | M+H | I-134 | 649 | M+H | I-182 | 610 | M+H |
| I-039 | 635 | M+H | I-087 | 569 | M+H | I-135 | 622 | M+H | I-183 | 612 | M+H |
| I-040 | 621 | M+H | I-088 | 621 | M+H | I-136 | 576 | M+H | I-184 | 610 | M+H |
| I-041 | 594 | M+H | I-089 | 603 | M+H | I-137 | 594 | M+H | I-185 | 628 | M+H |
| I-042 | 569 | M+H | I-090 | 612 | M+H | I-138 | 594 | M+H | I-186 | 642 | M+H |
| I-043 | 621 | M+H | I-091 | 578 | M+H | I-139 | 606 | M+H | I-187 | 622 | M+H |
| I-044 | 605 | M+H | I-092 | 595 | M+H | I-141 | 573 | M+H | I-188 | 628 | M+H |
| I-045 | 603 | M+H | I-093 | 561 | M+H | I-142 | 572 | M+H | I-189 | 592 | M+H |
| I-046 | 646 | M+H | I-094 | 618 | M+H | I-143 | 632 | M+H | I-190 | 696 | M+H |
| I-047 | 646 | M+H | I-095 | 599 | M+H | I-144 | 588 | M+H | I-191 | 637 | M+H |
| I-048 | 608 | M+H | I-096 | 635 | M+H | | | | | | |

**[Table 16]**

| No. | MS | Charge | No. | MS | Charge | No. | MS | Charge | No. | MS | Charge |
|---|---|---|---|---|---|---|---|---|---|---|---|
| I-193 | 627 | M+H | I-196 | 619 | M+H | I-199 | 696 | M+H | I-201 | 631 | M+H |
| I-194 | 643 | M+H | I-197 | 635 | M+H | I-200 | 631 | M+H | | | |
| I-195 | 639 | M+H | I-198 | 631 | M+H | | | | | | |

The following compounds can be also synthesized in the same manner by using the general synthesis method described above or the synthesis method described in Examples.

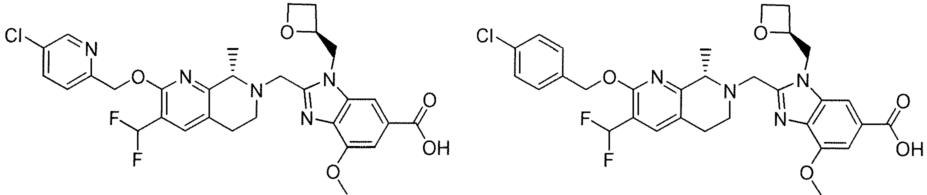

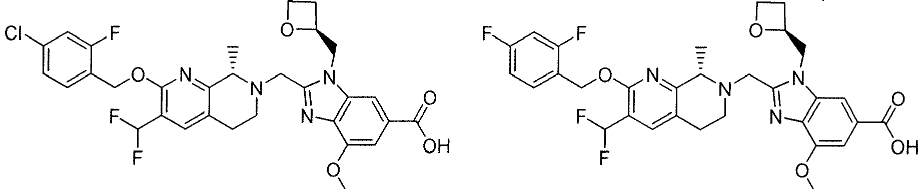

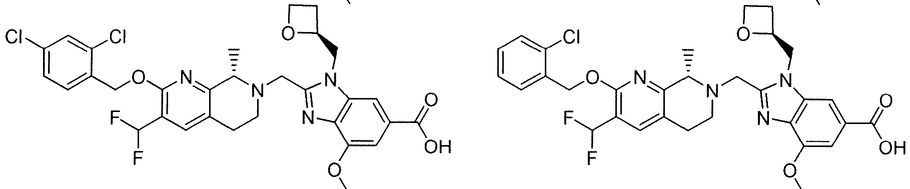

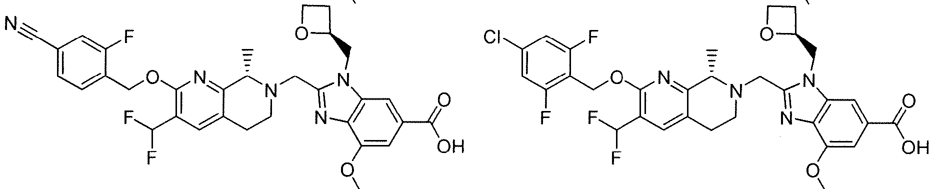

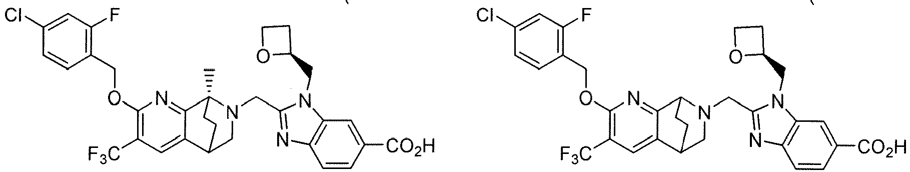

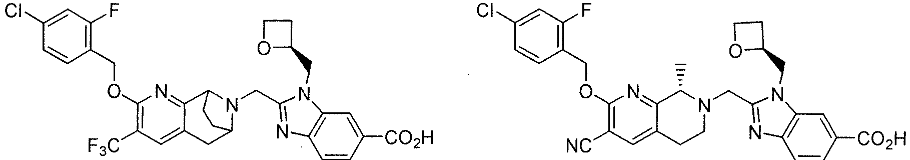

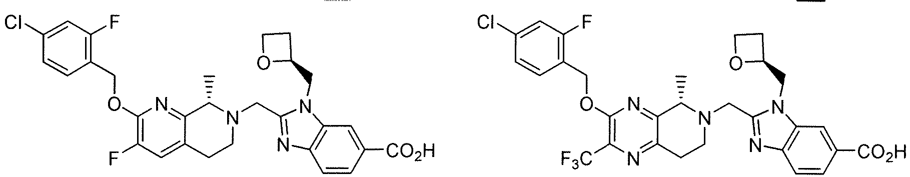

Biological Test Examples for the compounds of the present invention will be described below. The compounds of the present invention can be tested essentially as described in test examples below.

The compound represented by formula (I) according to the present invention has a GLP-1 receptor agonist activity effect.

Specifically, in the evaluation method described below, the EC₅₀ value is preferably 5000 nM or less, more preferably 1000 nM or less, even more preferably 100 nM or less.

### Test Example 1: Measurement of GLP-1 receptor agonist activity

### Cell culture

Human GLP-1 receptor stably expressing cells (hGLP-1R/CHO-K1 cells) are cultured in an α-MEM medium (Sigma) containing 10% FBS (Hyclone), 2% GlutaMAX (Gibco), 1% G418 (Nacalai Tesque), and 1% Penicillin-Streptomycin Mixed Solution (Sigma) at 37°C under 5% CO₂ conditions, and recovered by treating a 10 fold diluted 5.0 g/l-trypsin/5.3 mmol/l-EDTA solution (Nacalai Tesque) and cryopreserved.

### cAMP assay

A DMSO solution containing the compound of the present invention or human GLP-1 (7 -36) (Phoenix Pharmaceuticals) is dispensed into a 384-well microplate (Greiner) at 62.5 nL/well, and in addition, 400 µM Forskolin (Nacalai Tesque) is dispensed therein at 7.5 nL/well. Subsequently, the frozen GLP-1 R/CHO-K1 cells are thawed in a thermostatic bath at 37°C, suspended in HBSS buffer (GIBCO) containing 0.1% BSA (Sigma), 20 mM HEPES, 0.1 mM IBMX (Sigma), and 0.2 mM RO20-1724 (Calbiochem) at 2×10⁴ cells/mL, and the cell suspension is added at 6 µL/well. The solution was incubated at 37°C for 1 hour and intracellular cAMP concentration is measured using cAMP Gs dynamic kit (Cisbio) according to the protocol attached to the product. Specifically, a cAMP-d2/Anti-cAMP-Cryptate (1/1) mixture solution is added in a total amount of 6 µL/well, and is incubated at room temperature for 1 hour, and then, time-resolved fluorescence is measured using PHERAstar (BMG Labtech).

Assuming that the cAMP concentration when human GLP-1 (7-36) is dispensed to a final concentration of 2 nM is 100% and the cAMP concentration when only DMSO is dispensed is 0%, the 50% effective concentration (EC₅₀) and the maximum effect (Emax) of the compound of the present invention are calculated using an increase in the cAMP concentration as an index, using TIBCO Spotfire (TIBCO Software). The dilution concentration or the dilution solvent are changed as necessary.

The compounds of the present invention were tested essentially as described above. EC₅₀ and Emax of each compound of the present invention are shown in the following table.

**[Table 17]**

| No. | EC50_nM | Emax_% | No. | EC50_nM | Emax_% | No. | EC50_nM | Emax_% |
|---|---|---|---|---|---|---|---|---|
| I-001 | 0.538 | 100 | I-049 | 0.307 | 112 | I-097 | 0.343 | 109 |
| I-002 | 0.168 | 109 | I-050 | 0.638 | 97 | I-098 | 1.16 | 118 |
| I-003 | 3.6 | 102 | I-051 | 1.02 | 118 | I-099 | 2.55 | 106 |
| I-004 | 2.88 | 105 | I-052 | 0.252 | 115 | I-100 | 0.58 | 115 |
| I-005 | 3.39 | 111 | I-053 | 0.417 | 123 | I-101 | 0.469 | 108 |
| I-006 | 0.308 | 130 | I-054 | 0.0963 | 121 | I-102 | 0.469 | 117 |
| I-007 | 7.91 | 88 | I-055 | 0.902 | 97 | I-103 | 0.558 | 97 |
| I-008 | 3.89 | 107 | I-056 | 0.354 | 108 | I-104 | 514 | 75.3 |
| I-009 | 0.969 | 122 | I-057 | 0.315 | 86.7 | I-105 | 0.314 | 110 |
| I-010 | 1.99 | 117 | I-058 | 0.924 | 92.7 | I-106 | 0.371 | 112 |
| I-011 | 16.1 | 102 | I-059 | 1.28 | 104 | I-107 | 0.106 | 111 |
| I-012 | 1.25 | 108 | I-060 | 0.147 | 105 | I-108 | 0.038 | 111 |
| I-013 | 4.21 | 114 | I-061 | 0.295 | 101 | I-109 | 0.101 | 112 |
| I-014 | 0.171 | 128 | I-062 | 0.215 | 99.3 | I-110 | 0.0706 | 113 |
| I-015 | 2.33 | 93.3 | I-063 | 0.195 | 92.3 | I-111 | 0.0683 | 110 |
| I-016 | 0.407 | 120 | I-064 | 0.345 | 102 | I-112 | 0.191 | 110 |
| I-017 | 0.17 | 136 | I-065 | 0.0809 | 104 | I-113 | 0.0331 | 116 |
| I-018 | 0.348 | 133 | I-066 | 0.0375 | 106 | I-114 | 0.0604 | 113 |
| I-019 | 0.176 | 124 | I-067 | 0.0605 | 98.3 | I-115 | 0.043 | 113 |
| I-020 | 0.0972 | 135 | I-068 | 0.194 | 112 | I-116 | 0.0463 | 122 |
| I-021 | 0.0833 | 112 | I-069 | 0.0422 | 109 | I-117 | 0.372 | 118 |
| I-022 | 0.125 | 112 | I-070 | 0.0187 | 105 | I-118 | 0.105 | 113 |
| I-023 | 0.443 | 130 | I-071 | 0.0683 | 110 | I-119 | 0.148 | 120 |
| I-024 | 0.117 | 124 | I-072 | 0.0274 | 106 | I-120 | 0.481 | 121 |
| I-025 | 0.307 | 113 | I-073 | 0.0658 | 101 | I-121 | 0.148 | 119 |
| I-026 | 4210 | 53.3 | I-074 | 0.0232 | 104 | I-122 | 0.073 | 112 |
| I-027 | 0.701 | 119 | I-075 | 0.0493 | 118 | I-123 | 0.275 | 127 |
| I-028 | 0.748 | 110 | I-076 | 0.0208 | 122 | I-124 | 0.038 | 115 |
| I-029 | 0.592 | 105 | I-077 | 7.35 | 92.3 | I-125 | 0.0856 | 123 |
| I-030 | 0.37 | 123 | I-078 | 14.8 | 96 | I-126 | 0.0635 | 111 |
| I-031 | 0.314 | 118 | I-079 | 886 | 75.7 | I-127 | 0.104 | 121 |
| I-032 | 0.368 | 126 | I-080 | 9.31 | 88.3 | I-128 | 0.0122 | 128 |
| I-033 | 0.437 | 111 | I-081 | 0.179 | 96.7 | I-129 | 0.0303 | 131 |
| I-034 | 0.249 | 103 | I-082 | 0.0849 | 100 | I-130 | 0.1080 | 141 |
| I-035 | 0.158 | 124 | I-083 | 0.122 | 116 | I-131 | 0.0299 | 123 |
| I-036 | 0.432 | 113 | I-084 | 0.0587 | 94.7 | I-132 | 0.0162 | 127 |
| I-037 | 0.105 | 122 | I-085 | 0.0643 | 118 | I-133 | 0.0591 | 122 |
| I-038 | 0.0595 | 126 | I-086 | 0.0685 | 101 | I-134 | 0.0104 | 128 |
| I-039 | 0.178 | 141 | I-087 | 0.248 | 103 | I-135 | 0.0165 | 134 |
| I-040 | 0.0679 | 129 | I-088 | 0.0865 | 103 | I-136 | 0.3390 | 106 |
| I-041 | 0.0637 | 145 | I-089 | 0.0739 | 106 | I-137 | 0.2240 | 136 |
| I-042 | 0.208 | 127 | I-090 | 2.05 | 98 | I-138 | 0.1190 | 128 |
| I-043 | 0.0647 | 118 | I-091 | 2.64 | 91.7 | I-139 | 0.0326 | 118 |
| I-044 | 0.109 | 126 | I-092 | 1.58 | 108 | I-141 | 21.3 | 110 |
| I-045 | 0.0345 | 131 | I-093 | 6.34 | 96.3 | I-142 | 7.33 | 117 |
| I-046 | 669 | 112 | I-094 | 0.256 | 103 | I-143 | 0.0999 | 129 |
| I-047 | 0.903 | 146 | I-095 | 0.716 | 102 | I-144 | 0.0625 | 124 |
| I-048 | 3.31 | 93.7 | I-096 | 0.247 | 103 | | | |

**[Table 18]**

| No. | EC50_nM | Emax_% | No. | EC50_nM | Emax_% | No. | EC50_nM | Emax_% |
|---|---|---|---|---|---|---|---|---|
| I-145 | 0.1210 | 109 | I-164 | 1.63 | 108 | I-182 | 0.1730 | 119 |
| I-146 | 0.0293 | 115 | I-165 | 0.9650 | 111 | I-183 | 0.1510 | 135 |
| I-147 | 0.1620 | 128 | I-166 | 0.2050 | 109 | I-184 | 0.0730 | 118 |
| I-148 | 0.8900 | 99.3 | I-167 | 0.0520 | 122 | I-185 | 0.0569 | 122 |
| I-149 | 1.09 | 97.7 | I-168 | 0.1930 | 104 | I-186 | 0.4660 | 123 |
| I-150 | 0.0391 | 105 | I-169 | 0.1910 | 109 | I-187 | 0.0400 | 137 |
| I-151 | 0.0738 | 92.7 | I-170 | 0.2430 | 105 | I-188 | 0.1190 | 120 |
| I-152 | 0.0992 | 105 | I-171 | 0.0152 | 124 | I-189 | 0.2220 | 124 |
| I-153 | 0.1900 | 112 | I-172 | 0.0353 | 106 | I-190 | 4.29 | 93.3 |
| I-154 | 7.04 | 101 | I-173 | 0.0889 | 99.7 | I-191 | 0.266 | 114 |
| I-155 | 0.8700 | 96.3 | I-174 | 0.1740 | 96.3 | I-193 | 0.211 | 110 |
| I-156 | 0.6710 | 101 | I-175 | 0.0831 | 107 | I-194 | 0.137 | 103 |
| I-158 | 0.4930 | 111 | I-176 | 0.0693 | 112 | I-195 | 0.0514 | 123 |
| I-159 | 0.1550 | 99.3 | I-177 | 0.0195 | 113 | I-196 | 0.611 | 108 |
| I-160 | 83.5 | 66.3 | I-178 | 12.4 | 99.7 | I-197 | 0.345 | 111 |
| I-161 | 28.2 | 73 | I-179 | 1.46 | 96.3 | | | |
| I-162 | 26 | 88 | I-180 | 0.2800 | 111 | | | |
| I-163 | 32.3 | 83.3 | I-181 | 0.5310 | 120 | | | |

From the above results, since the compound of the present invention exhibited GLP-1 receptor agonist activity, the compound is expected to have an effect as a therapeutic or prophylactic agent for a disease involving the GLP-1 receptor.

### Test Example 2: Metabolism Stability Test

Using commercially available pooled human liver microsomes, a compound of the present invention is reacted for a constant time, and a remaining rate is calculated by comparing a reacted sample and an unreacted sample, thereby, a degree of metabolism in liver is assessed.

A reaction is performed (oxidative reaction) at 37°C for 0 minute or 30 minutes in the presence of 1 mmol/L NADPH in 0.2 mL of a buffer (50 mmol/L Tris-HCl pH 7.4, 150 mmol/L potassium chloride, 10 mmol/L magnesium chloride) containing 0.5 mg protein/mL of human liver microsomes. After the reaction, 70 µL of the reaction solution is added to 140 µL of a methanol/acetonitrile = 1/1 (v/v), mixed and centrifuged at 3000 rpm for 15 minutes. The compound of the present invention in the centrifuged supernatant is quantified by LC/MS/MS or solid-phase extraction (SPE)/MS. The ratio of the amount of the compound after the reaction, with respect to the amount of the compound at 0 minutes of the reaction defined as 100%, is shown as the residual rate. The dilution concentration or the dilution solvent are changed as necessary.

The compounds of the present invention were tested essentially as described above.

### Test Example 3: Solubility test

The solubility of the compound of the present invention is determined under 1% DMSO addition conditions. A 10 mmol/L solution of the compound is prepared with DMSO, and 2 µL of the solution of the compound of the present invention is added, respectively, to 198 µL of the second fluid for the dissolution test in the Japanese Pharmacopeia (JP 17). The mixture is shaken for 3 hours at a room temperature, and the mixture is vacuum-filtered. The filtrate is 100-fold diluted with methanol/acetonitrile/water = 1/1/2 (V/V/V), and the compound concentration in the filtrate is measured with LC/MS/MS by the absolute calibration method.

The compounds of the present invention were tested essentially as described above.

### Test Example 4: CYP Inhibition Test

Using commercially available pooled human liver microsome, and employing, as markers, 7-ethoxyresorufin O-deethylation (CYP1A2), tolbutamide methyl-hydroxylation (CYP2C9), mephenytoin 4'-hydroxylation (CYP2C19), dextromethorphan O-demethylation (CYP2D6), and terfenadine hydroxylation (CYP3A4) as typical substrate metabolism reactions of human main five CYP enzyme forms (CYP1A2, 2C9, 2C19, 2D6, 3A4), an inhibitory degree of each metabolite production amount by the compound of the present invention was assessed.

The reaction conditions are as follows: substrate, 0.5 µmol/L ethoxyresorufin (CYP1A2), 100 µmol/L tolbutamide (CYP2C9), 30 µmol/L or 50 µmol/L S-mephenytoin (CYP2C19), 5 µmol/L dextromethorphan (CYP2D6), 1 µmol/L terfenadine (CYP3A4); reaction time, 15 minutes; reaction temperature, 37°C; enzyme, 0.2 mg protein/mL of pooled human liver microsome; concentrations of the compound of the present invention, 1, 5, 10, 20 µmol/L (four points).

Each five kinds of substrates, human liver microsomes, or compound of the present invention in 50 mmol/L Hepes buffer are added to a 96-well plate at the composition as described above, and NADPH, as a cofactor is added to initiate metabolism reactions. After reaction at 37°C for 15 minutes, the reaction is terminated by the addition of a solution of methanol/acetonitrile = 1/1 (V/V). After the centrifugation at 3000 rpm for 15 minutes, resorufin (CYP1A2 metabolite) in the supernatant is quantified by a fluorescent multilabel counter or LC/MS/MS and hydroxytolbutamide (CYP2C9 metabolite), 4' hydroxymephenytoin (CYP2C19 metabolite), dextromethorphan (CYP2D6 metabolite), and terfenadine alcohol metabolite (CYP3A4 metabolite) are quantified by LC/MS/MS.

The sample in which only DMSO as a solvent dissolving a drug is added to a reaction system is adopted as a control (100%). Remaining activity (%) is calculated and IC₅₀ is calculated by reverse presumption by a logistic model using a concentration and an inhibition rate.

The compounds of the present invention were tested essentially as described above.

### Test Example 5: BA Test

### Materials and methods for experiments to evaluate oral absorption

(1) Experimental animals: Mice or SD rats are used.
(2) Rearing condition: Mice or SD rats are allowed free access to solid feed and sterilized tap water.
(3) Setting of dosage and grouping: Oral administration and Intravenous administration were performed with the predetermined dosages. Grouping is set as below. (Dosage can be changed per compound)
   Oral administration 2 to 60 µmol/kg or 1 to 30 mg/kg (n = 2 to 3)
   Intravenous administration 1 to 20 µmol/kg or 0.5 to 10 mg/kg (n = 2 to 3)
(4) Preparation of administration solutions: Oral administration is performed as solution or suspension. Intravenous administration is performed after solubilization.
(5) Routes of administration: Oral administration is performed mandatory into the stomach by oral sonde. Intravenous administration is performed from caudal vein or femoral vein by a needled syringe.
(6) Evaluation items: Blood was collected over time, and the plasma concentration of the compound of the present invention is measured using LC/MS/MS.
(7) Statistical analysis: An area under plasma concentration-time curve (AUC) is calculated as to change in the concentration of the compound of the present invention in plasma by the moment analysis method, and bioavailability (BA) of the compound of the present invention is calculated from a dose ratio and an AUC ratio between the oral administration group and the intravenous administration group.

The compounds of the present invention can be tested essentially as described above.

### Test Example 6: Clearance Evaluation Test

Materials and methods for experiments
(1) Experimental animals: SD rats are used.
(2) Rearing condition: SD rats are allowed free access to solid feed and sterilized tap water.
(3) Setting of dosage and grouping: Intravenous administration was performed with the predetermined dosage. Grouping is set as below.
   Intravenous administration 1 µmol/kg (n = 2)
(4) Preparation of administration solutions: Administration is performed after solubilization by using dimethyl sulfoxide/propylene glycol = 1/1 as the solvent.
(5) Routes of administration: Intravenous administration is performed from caudal vein by a needled syringe.
(6) Evaluation items: Blood is collected serially and concentration of a compound according to the present invention in plasma is measured by LC/MS/MS.
(7) Statistical analysis: About transition of concentration of a compound according to the present invention in plasma, total clearance (CLtot) of a compound according to the present invention is calculated by the moment analysis method. The dilution concentration or the dilution solvent are changed as necessary.

The compounds of the present invention were tested essentially as described above.

### Test Example 6-2: Clearance Evaluation Test

### Materials and methods for experiments

(1) Experimental animals: Dogs (Marshall Beagles) are used.
(2) Rearing condition: The dogs are allowed to freely take solid food and sterilized tap water.
(3) Setting of dosage and grouping: Intravenous administration was performed with the predetermined dosage. Grouping is set as below.
   Intravenous administration: 0.1 to 1 mg/kg (n = 2)
(4) Preparation of administration solutions: For administration, the mixture is solubilized using any solvent of dimethylacetamide/ethanol/carbonate buffer = 2/3/5, ethanol/carbonate buffer = 1/1, and dimethylacetamide/polyethylene glycol 400/20% hydroxypropyl-β-cyclodextrin = 1/1/2.
(5) Routes of administration: Intravenous administration is performed from caudal vein by a needled syringe.
(6) Evaluation items: Blood is collected serially and concentration of a compound according to the present invention in plasma is measured by LC/MS/MS.
(7) Statistical analysis: About transition of concentration of a compound according to the present invention in plasma, total clearance (CLtot) of a compound according to the present invention is calculated by the moment analysis method. The dilution concentration or the dilution solvent are changed as necessary.

The compounds of the present invention were tested essentially as described above.

### Test Example 7: CYP3A4 (MDZ) MBI Test

CYP3A4 (MDZ) MBI test is a test of investigating mechanism based inhibition (MBI) potential on CYP3A4 by the enhancement of inhibitory degree of a metabolic reaction caused by the compound of the present invention. The inhibition of CYP3A4 is evaluated in pooled human liver microsomes by using the 1-hydroxylation reaction of midazolam (MDZ) as an index.

The reaction conditions are as follows: substrate, 10 µmol/L MDZ; pre-reaction time, 0 or 30 minutes; substrate reaction time, 2 minutes; reaction temperature, 37°C; protein content of pooled human liver microsomes, at pre-reaction time 0.5 mg/mL, at reaction time 0.05 mg/mL (at 10-fold dilution); concentrations of the compound of the present invention, 0.83, 5, 10, 20 µmol/L (four points).

Pooled human liver microsomes and a solution of the compound of the present invention in K-Pi buffer (pH 7.4) as a pre-reaction solution are added to a 96-well plate at the composition of the pre-reaction. A part of pre-reaction solution is transferred to another 96-well plate, and 1/10 diluted by K-Pi buffer containing a substrate. NADPH as a co-factor is added to initiate a reaction as a marker reaction (without preincubation). After a predetermined time of a reaction, methanol/acetonitrile=1/1 (V/V) solution is added to stop the reaction. In addition, NADPH is added to a remaining pre-reaction solution to initiate a pre-reaction (with preincubation). After a predetermined time of a pre-reaction, a part is transferred to another plate, and 1/10 diluted by K-Pi buffer containing a substrate to initiate a reaction as a marker reaction. After reaction for a given time, the reaction is terminated by the addition of a solution of methanol/acetonitrile = 1/1 (V/V). Each plate where the index reaction has been performed is centrifuged at 3000 rpm for 15 minutes. Then, midazolam 1-hydroxide in the centrifugation supernatants is quantified by LC/MS/MS.

The sample in which only DMSO as a solvent dissolving the compound of the present invention is added to a reaction system is adopted as a control (100 %). Remaining activity (%) is calculated at each concentration of the compound of the present invention compared to control, and IC value is calculated by reverse-presumption by a logistic model using a concentration and an inhibition rate. Shifted IC value is calculated as "IC of preincubation at 0 min/ IC of preincubation at 30min". When a shifted IC is 1.5 or more, this is defined as positive. When a shifted IC is 1.0 or less, this is defined as negative.

The compounds of the present invention were tested essentially as described above.

### Test Example 8: Powder Solubility Test

Appropriate quantity of the compound of the present invention is put in suitable containers. 200 µL of JP-1 fluid (water is added to 2.0 g of sodium chloride and 7.0 mL of hydrochloric acid to reach 1000 mL), 200 µL of JP-2 fluid (1.70 g of sodium dihydrogen phosphate and 1.775 g of anhydrous disodium hydrogen phosphate are dissolved in 1000 mL of water to obtain a buffer solution of pH 6.8 to 6.9), or 20 mmol/L sodium taurocholate (TCA)/JP-2 fluid (JP-2 fluid is added to 1.08 g of TCA to reach 100 mL) is independently added to each container. When the total amount is dissolved after adding the test reagent, the compound of the present invention is added appropriately. The containers are hermetically sealed, shaken at 37°C for 1 hour, and then filtered. Each filtrate is diluted 2-fold by addition of 100 µL of methanol to 100 µL of the filtrate. The dilution rate is changed as necessary. After checking that there is no bubble and precipitate, the container is sealed and shaken. The compound of the present invention is measured using HPLC by absolute calibration curve method.

The compounds of the present invention can be tested essentially as described above.

### Test Example 9: Fluctuation Ames Test

The compound of the present invention is evaluated for its mutagenicity.

Cryopreserved Salmonella typhimurium (TA98 strain and TA100 strain), 20 µL, was inoculated to 10 mL of a liquid nutrient medium (2.5% Oxoid nutrient broth No. 2) and shake-precultured at 37°C for 10 hours. The bacterial solution of the TA98 strain, 8.0 to 11.0 mL, is centrifuged (2000 × g, 10 min) to remove the culture solution. The bacteria was suspended in 8.0 to 11.0 mL of a Micro F buffer (K₂HPO₄: 3.5 g/L, KH₂PO₄: 1 g/L, (NH₄)₂SO₄: 1 g/L, trisodium citrate dihydrate: 0.25 g/L, MgSO₄•7H₂O: 0.1 g/L), the suspension was added to 120 mL of an Exposure medium (Micro F buffer containing Biotin: 8 µg/mL, histidine: 0.2 µg/mL, glucose: 8 mg/mL). The TA100 strain was added to 120 mL of the Exposure medium relative to 3.1 mL of the bacterial solution to prepare a test bacterial solution. 12 µL each of a DMSO solution of the compound of the present invention (several serial dilutions from maximum dose 50 mg/mL at 2- to 3-fold common ratio), DMSO as a negative control, and 50 µg/mL of a 4-nitroquinoline-1-oxide DMSO solution for the TA98 strain and 0.25 µg/mL of a 2-(2-furyl)-3-(5-nitro-2-furyl)acrylamide DMSO solution for the TA100 strain under non-metabolic activation conditions or 40 µg/mL of a 2-aminoanthracene DMSO solution for the TA98 strain and 20 µg/mL of a 2-aminoanthracene DMSO solution for the TA100 strain under metabolic activation conditions as a positive control, and 588 µL of the test bacterial solution (a mixed solution of 498 µL of the test bacterial solution and 90 µL of S9 mix under metabolic activation conditions) are mixed, and the mixture is shake-cultured at 37°C for 90 minutes. A microbial cell solution exposed to the compound of the present invention and an Indicator medium (Micro F buffer containing 8 µg/mL biotin, 0.2 µg/mL histidine, 8 mg/mL glucose, and 37.5 µg/mL Bromo Cresol Purple) are mixed at a ratio of 23: 115, and 50 µL of the microbial cell solution containing Indicator, 2760 µL in total, is dispensed to microplate 48 wells/dose, and this is incubated at 37°C for 3 days. The color of the solution in a well containing a bacterium that has acquired growth ability due to a mutation in amino acid (histidine) synthase gene is changed from purple to yellow depending on pH change. Therefore, yellow-colored wells with bacterial growth among the 48 wells per dose are counted and evaluated by comparison with the negative control group. Negativity to mutagenicity is indicated by (-), and positivity thereto is indicated by (+).

The compounds of the present invention were tested essentially as described above.

### Test Example 10: hERG Test

For the purpose of evaluating the compound of the present invention for the risk of electrocardiogram QT interval prolongation, the effect of the compound of the present invention on delayed rectifier K⁺ current (I_{Kr}) which plays an important role in a ventricular repolarization process is studied using CHO cells caused to express a human ether-a-go-go related gene (hERG) channel.

The cells are kept at a membrane potential of -80 mV by the whole cell patch clamp method using a fully automated patch clamp system (QPatch; Sophion Bioscience A/S), given a leak potential of -50 mV, and then given depolarization stimulation of +20 mV for 2 seconds and further repolarization stimulation of -50 mV for 2 seconds. I_{Kr} induced by this procedure is recorded. An extracellular fluid (NaCl: 145 mmol/L, KCl: 4 mmol/L, CaCl₂: 2 mmol/L, MgCl₂: 1 mmol/L, glucose: 10 mmol/L, HEPES (4-(2-hydroxyethyl)-1-piperazineethanesulfonic acid): 10 mmol/L, pH = 7.4) adjusted to 0.1% with dimethyl sulfoxide is used as a vehicle to apply the vehicle or the extracellular fluid containing the compound of the present invention dissolved at a concentration of interest to the cells under conditions of room temperature for 7 minutes or longer. From the obtained I_{Kr}, the absolute value of the maximum tail current based on the current value at the membrane potential where the cells have been kept is measured using analytical software (QPatch Assay software; Sophion Bioscience A/S). The maximum tail current after the application of the compound of the present invention with respect to the maximum tail current after the application of the vehicle is further calculated as the rate of inhibition to evaluate the influence of the compound of the present invention on I_{Kr}. The dilution concentration or the dilution solvent are changed as necessary.

The compounds of the present invention can be tested essentially as described above.

### Formulation Example

The compound of the present invention can be administered as a pharmaceutical composition by any conventional route, in particular enterally, for example, orally, for example, in the form of tablets or capsules, or parenterally, for example, in the form of injectable solutions or suspensions, topically, for example, in the form of lotions, gels, ointments or creams, or in a nasal or suppository form. Pharmaceutical compositions comprising a compound of the present invention in free form or in a pharmaceutically acceptable salt form in association with at least one pharmaceutically acceptable carrier or diluent can be manufactured in a conventional manner by mixing, granulating or coating methods. For example, the oral composition can be a tablet, a granular preparation, or a capsule, each containing an excipient, a disintegrating agent, a binder, a lubricating agent, and the like, as well as an active ingredient and the like. Furthermore, the composition for injection can be prepared as a solution or a suspension, may be sterilized, and may contain a preservative, a stabilizer, a buffering agent, and the like.

### [INDUSTRIAL APPLICABILITY]

The compounds of the present invention have GLP-1 receptor agonist activity and are considered to be useful as a therapeutic and/or preventive agent for diseases or conditions associated with the GLP-1 receptor.

## Claims

1. A compound represented by formula (I): 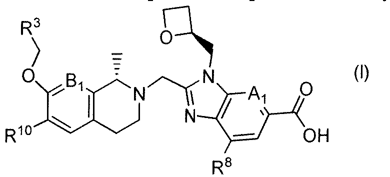 wherein
A₁ is C(R¹) or N,
R¹ is a hydrogen atom or halogen,
R⁸ is a hydrogen atom, halogen, alkyloxy, or 5- or 6-membered aromatic heterocyclyl optionally substituted with halogen or alkyl,
B₁ is CH or N,
R¹⁰ is a hydrogen atom, cyano, a fluorine atom, a chlorine atom, methyl, difluoromethyl, or trifluoromethyl,
R³ is phenyl optionally substituted with substituent group F, 5- or 6-membered aromatic heterocyclyl optionally substituted with substituent group F, bicyclic 9- or 10-membered aromatic heterocyclyl optionally substituted with substituent group F, or 5- to 12-membered non-aromatic heterocyclyl optionally substituted with substituent group F,
the substituent group F: halogen, cyano, alkyl, haloalkyl, alkyloxy, and haloalkyloxy,
wherein
(i) when R¹⁰ is a chlorine atom, Bi is N,
(ii) when R¹⁰ is trifluoromethyl, R³ is 5-membered aromatic heterocyclyl optionally substituted with substituent group F, bicyclic 9- or 10-membered aromatic heterocyclyl optionally substituted with substituent group F, or 5- to 12-membered non-aromatic heterocyclyl optionally substituted with substituent group F, or R⁸ is 5- or 6-membered aromatic heterocyclyl optionally substituted with halogen or alkyl, and
(iii) the compound is other than those below: 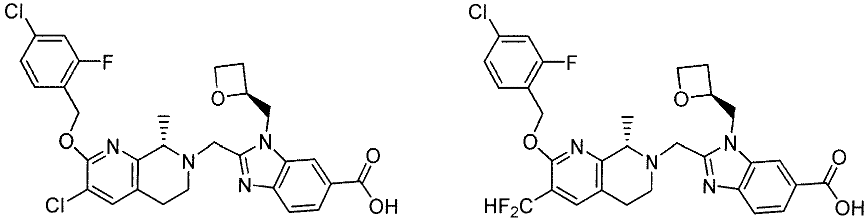 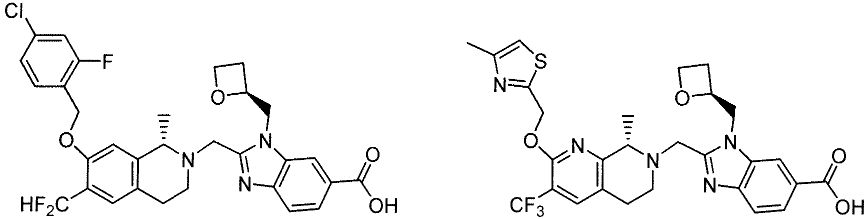 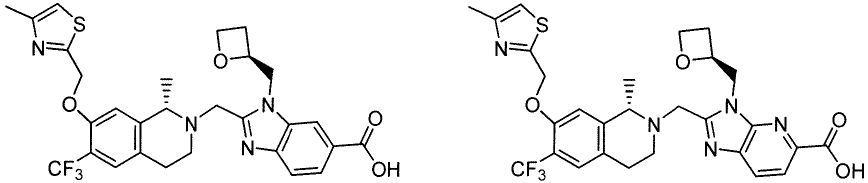 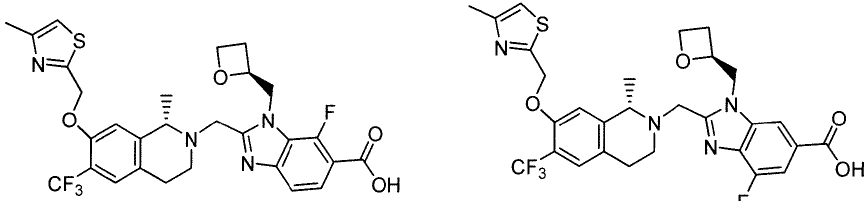 and 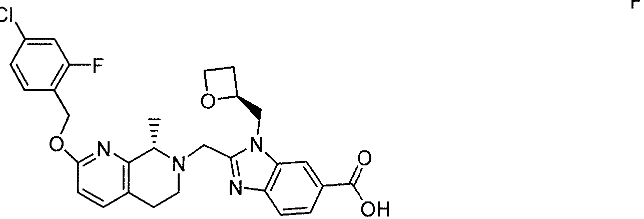
or a pharmaceutically acceptable salt thereof.

2. The compound according to claim 1 or a pharmaceutically acceptable salt thereof, wherein R¹⁰ is a hydrogen atom, cyano, a fluorine atom, a chlorine atom, methyl, or difluoromethyl.

3. The compound according to claim 1 or 2 or a pharmaceutically acceptable salt thereof, wherein R³ is a group below: 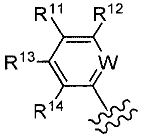 wherein
W is N, or CR¹⁵,
R¹¹ is a hydrogen atom, halogen, cyano, alkyl, haloalkyl, alkyloxy, or haloalkyloxy,
R¹² and R¹³ are each independently a hydrogen atom or halogen,
R¹⁴ and R¹⁵ are each independently a hydrogen atom, halogen, cyano, alkyl, haloalkyl, alkyloxy, or haloalkyloxy,
R¹¹ and R¹² may be taken together to form 5-membered aromatic heterocycle optionally substituted with substituent group F, or 5- to 7-membered non-aromatic heterocycle optionally substituted with substituent group F,
R¹¹ and R¹³ may be taken together to form 5-membered aromatic heterocycle optionally substituted with substituent group F, or 5- to 7-membered non-aromatic heterocycleoptionally substituted with substituent group F, and
R¹³ and R¹⁴ may be taken together to form 5-membered aromatic heterocycle optionally substituted with substituent group F, or 5- to 7-membered non-aromatic heterocycle optionally substituted with substituent group F.

4. The compound according to any one of claims 1 to 3 or a pharmaceutically acceptable salt thereof, wherein R³ is any one of groups below: 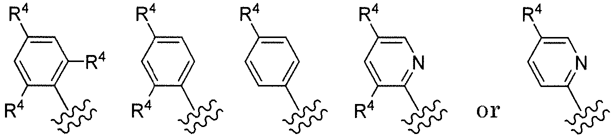 wherein R⁴s are each independently halogen, cyano, alkyl, haloalkyl, alkyloxy, or haloalkyloxy.

5. The compound according to claim 4 or a pharmaceutically acceptable salt thereof, wherein R⁴s are each independently a fluorine atom, a chlorine atom, cyano, methyl, methyloxy, or difluoromethyloxy.

6. The compound according to any one of claims 1 to 5 or a pharmaceutically acceptable salt thereof, wherein R¹⁰ is a chlorine atom or difluoromethyl.

7. The compound according to any one of claims 1 and 3 to 5 or a pharmaceutically acceptable salt thereof, wherein
R¹⁰ is trifluoromethyl, and
R³ is 5-membered aromatic heterocyclyl optionally substituted with substituent group F, bicyclic 9- or 10-membered aromatic heterocyclyl optionally substituted with substituent group F, or bicyclic 8- to 11-membered non-aromatic heterocyclyl optionally substituted with substituent group F.

8. The compound according to any one of claims 1 and 3 to 5 or a pharmaceutically acceptable salt thereof, wherein
R¹⁰ is trifluoromethyl, and
R⁸ is 5- or 6-membered aromatic heterocyclyl optionally substituted with halogen or alkyl.

9. A compound represented by formula (II): 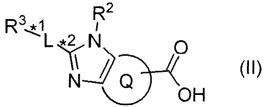 wherein
Q is a substituted or unsubstituted benzene ring or a substituted or unsubstituted 5- or 6-membered aromatic heterocycle,
R² is substituted or unsubstituted alkyl, or substituted or unsubstituted non-aromatic heterocyclyl,
-L- is any one of groups below: 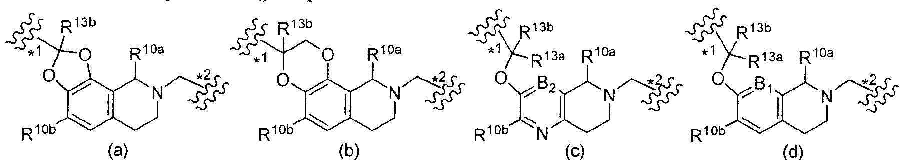 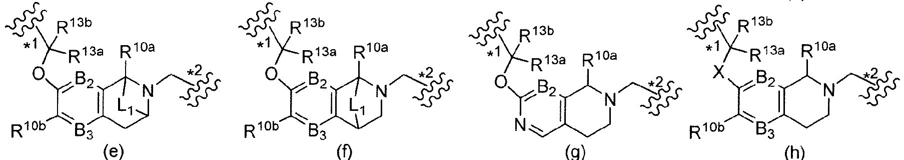 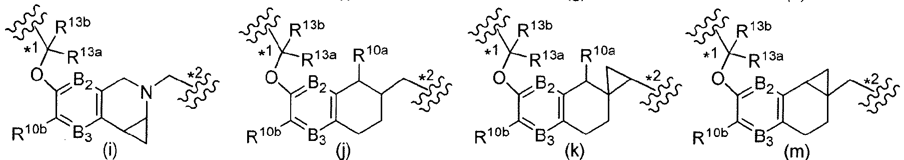 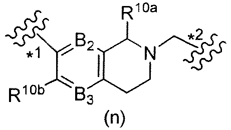
wherein
B₁ is CH or N,
B₂ is C(R⁹) or N,
R⁹ is a hydrogen atom or R⁹ and R^{13a} are taken together to form -O- or -CH₂-O-, B₃ is CH or N,
R^{10a} is a hydrogen atom, or substituted or unsubstituted alkyl,
R^{10b} is a hydrogen atom, cyano, halogen, or substituted or unsubstituted alkyl,
-L₁- is alkylene,
X is S, NH or CH₂,
R^{13a} is a hydrogen atom or R^{13a} and R⁹ are taken together to form -O- or -CH₂-O-, and
R^{13b} is a hydrogen atom, or substituted or unsubstituted alkyl, and
R³ is phenyl optionally substituted with substituent group F, 5- or 6-membered aromatic heterocyclyl optionally substituted with substituent group F, bicyclic 9- or 10-membered aromatic heterocyclyl optionally substituted with substituent group F, or 5- to 12-membered non-aromatic heterocyclyl optionally substituted with substituent group F,
the substituent group F: halogen, cyano, alkyl, haloalkyl, alkyloxy, and haloalkyloxy,
wherein
(i) when -L- is a group represented by (d), Q is a benzene ring substituted with substituted or unsubstituted aromatic heterocyclyl, 6-membered aromatic heterocycle substituted with substituted or unsubstituted aromatic heterocyclyl, or substituted or unsubstituted 5-membered aromatic heterocycle, and
(ii) the compound is other than those below: 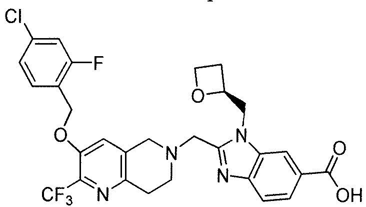
or a pharmaceutically acceptable salt thereof.

10. The compound according to claim 9 or a pharmaceutically acceptable salt thereof, wherein R³ is a group below: 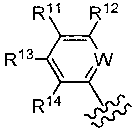
wherein
W is N, or CR¹⁵,
R¹¹ is a hydrogen atom, halogen, cyano, alkyl, haloalkyl, alkyloxy, or haloalkyloxy,
R¹² and R¹³ are each independently a hydrogen atom or halogen,
R¹⁴ and ^{R15} are each independently a hydrogen atom, halogen, cyano, alkyl, haloalkyl, alkyloxy, or haloalkyloxy,
R¹¹ and R¹² may be taken together to form 5-membered aromatic heterocycle optionally substituted with substituent group F, or 5- to 7-membered non-aromatic heterocycle optionally substituted with substituent group F,
R¹¹ and R¹³ may be taken together to form 5-membered aromatic heterocycle optionally substituted with substituent group F, or 5- to 7-membered non-aromatic heterocycle optionally substituted with substituent group F, and
R¹³ and R¹⁴ may be taken together to form 5-membered aromatic heterocycle optionally substituted with substituent group F, or 5- to 7-membered non-aromatic heterocycle optionally substituted with substituent group F.

11. The compound according to claim 9 or 10 or a pharmaceutically acceptable salt thereof, wherein R³ is any one of groups below: 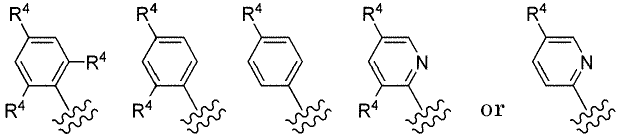 wherein R⁴s are each independently halogen, cyano, alkyl, haloalkyl, alkyloxy, or haloalkyloxy.

12. The compound according to claim 11 or a pharmaceutically acceptable salt thereof, wherein R⁴s are each independently a fluorine atom, a chlorine atom, cyano, methyl, methyloxy, or difluoromethyloxy.

13. The compound according to any one of claims 9 to 12 or a pharmaceutically acceptable salt thereof, wherein Q is any one of groups below: 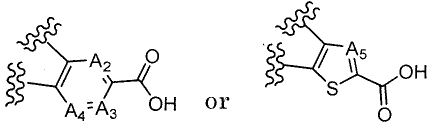 wherein
A₂ is C(R⁵) or N,
As is C(R⁶) or N,
A₄ is C(R⁷) or N,
R⁵, R⁶, and R⁷ are each independently a hydrogen atom, halogen, cyano, substituted or unsubstituted alkyl, substituted or unsubstituted alkyloxy, substituted or unsubstituted aromatic heterocyclyl, or substituted or unsubstituted non-aromatic carbocyclyl,
A₅ is C(R⁹) or N, and
R⁹ is each independently a hydrogen atom, halogen, cyano, substituted or unsubstituted alkyl, or substituted or unsubstituted alkyloxy.

14. The compound according to claim 13 or a pharmaceutically acceptable salt thereof, wherein
(i) A₂ is C(R⁵), A₃ is C(R⁶), and A₄ is C(R⁷),
(ii) A₂ is N, A₃ is C(R⁶), and A₄ is C(R⁷),
(iii) A₂ is C(R⁵), A₃ is C(R⁶), and A₄ is N, or
(iv) A₂ is N, A₃ is C(R⁶), and A₄ is N.

15. The compound according to claim 14 or a pharmaceutically acceptable salt thereof, wherein
(i) A₂ is C(R⁵), A₃ is C(R⁶), and A₄ is C(R⁷), or
(ii) A₂ is N, A₃ is C(R⁶), and A₄ is C(R⁷).

16. The compound according to any one of claims 13 to 15 or a pharmaceutically acceptable salt thereof, wherein R⁵, R⁶, and R⁷ are each independently a hydrogen atom, halogen, alkyl, or alkyloxy.

17. The compound according to any one of claims 9 to 16 or a pharmaceutically acceptable salt thereof, wherein -L- is any one of groups below:

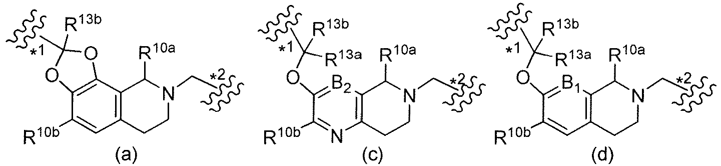

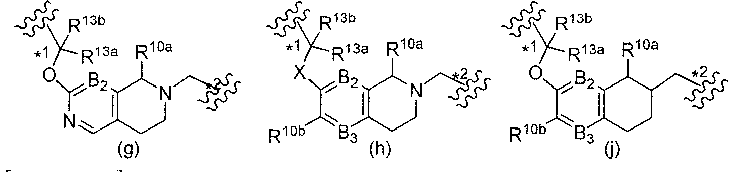

18. The compound according to any one of claims 9 to 17 or a pharmaceutically acceptable salt thereof, wherein R² is oxetanylmethyl.

19. A pharmaceutical composition comprising the compound according to any one of claims 1 to 18 or a pharmaceutically acceptable salt of the compound.

20. The pharmaceutical composition according to claim 19, which is a GLP-1 receptor agonist.
